(19) Europäisches Patentamt
European Patent Office
Office européen des brevets

(11) Veröffentlichungsnummer : **0 072 348**
**B1**

(12) # EUROPÄISCHE PATENTSCHRIFT

(45) Veröffentlichungstag der Patentschrift : **05.03.86**

(21) Anmeldenummer : **82810324.2**

(22) Anmeldetag : **02.08.82**

(51) Int. Cl.[4] : **C 07 C101/16**, C 07 C101/18, C 07 C101/30, C 07 C103/183, C 07 C103/48, C 07 C103/49, C 07 C103/50, C 07 C109/097, C 07 C 93/14, C 07 C121/42, C 07 C127/22

(54) **Neue Phenoxyphenyl-aminosäure-Derivate, ihre Herstellung, sie enthaltende Mittel sowie ihre Verwendung.**

(30) Priorität : **07.08.81 CH 5099/81**
**07.06.82 CH 3504/82**

(43) Veröffentlichungstag der Anmeldung : **16.02.83 Patentblatt 83/07**

(45) Bekanntmachung des Hinweises auf die Patenterteilung : **05.03.86 Patentblatt 86/10**

(84) Benannte Vertragsstaaten :
**AT BE CH DE FR IT LI NL SE**

(56) Entgegenhaltungen :
**EP-A- 0 003 295**
**EP-A- 0 014 880**
**EP-A- 0 021 692**
**EP-A- 0 027 555**
**EP-A- 0 030 676**
**FR-A- 2 189 368**
**LU-A- 67 204**
**CHEMICAL ABSTRACTS, Band 97, Nr. 19, 8. November 1982, Seite 767, Nr. 163488g, Columbus, Ohio, USA**

**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Dürr, Dieter, Dr.**
**Brändelistalweg 16**
**CH-4103 Bottmingen (CH)**

**EP 0 072 348 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Phenoxyphenyl-aminosäure-Derivate, Verfahren zu ihrer Herstellung, Mittel, welche die neuen Verbindungen als Wirkstoffe enthalten, und die Verwendung der neuen Verbindungen oder sie enthaltender Mittel zur Bekämpfung von unerwünschtem Pflanzenwuchs oder zur Desiccation oder Defoliation von Baumwoll- und Kartoffelpflanzen.

Diphenyläther mit herbizider Wirkung sind aus der europäischen Patentanmeldung 27555, der amerikanischen Patentschrift 4.277.624 und der deutschen Offenlegungsschrift 2.311.638 bekannt. Ferner sind Phenoxyphenylthio-alkancarbonsäurederivate mit herbizider Wirkung in der europäischen Patentanmeldung 351 beschrieben worden, und aus den europäischen Patentanmeldüngen 14880, 30676 und 21692 sind herbizide 2-Nitro-5-(2-chlor-4-trifluoromethylphenoxy)-phenyl-Verbindungen bekannt, welche in Nachbarstellung zur Nitrogruppe in unterschiedlicher Weise Substituiert sind.

Die neuen Verbindungen gemäss vorliegender Erfindung entsprechen der Formel I

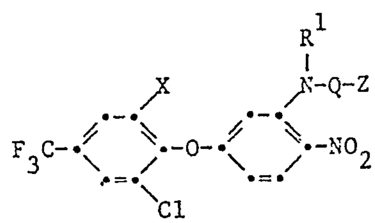

(I)

worin

X Wasserstoff, Chlor, Fluor oder Brom,

$R^1$ Wasserstoff, Nitroso oder $C_1$-$C_4$-Alkyl, $C_2$-$C_4$-Alkenyl, $C_1$-$C_4$-Alkylcarbonyl oder $C_2$-$C_4$-Alkenylcarbonyl, welche unsubstituiert oder durch ein oder mehrere Halogenatome substituiert sind,

Q einen geradkettigen oder verzweigten Alkylen- oder Alkenylenrest mit bis zu 5 Kohlenstoffatomen, in welchem neben der Hauptkette 0 bis 3 Seitenketten vorliegen, wobei die Hauptkette gesättigt oder ungesättigt und unsubstituiert oder ein- oder zweifach durch Substituenten der Gruppe $-OR^2$, $-SR^3$ oder $-COOR^4$ substituiert ist, wobei keines der Kohlenstoffatome disubstituiert ist, und die Seitenketten ungesättigt und unsubstituiert oder eine oder zwei der Seitenketten durch $-OR^5$, $-SR^6$ oder $-COOR^7$ substituiert sind, wobei gleichzeitig mit der Hauptkette nur eine der Seitenketten substituiert sein kann, und $R^2$, $R^3$, $R^4$, $R^5$, $R^6$ und $R^7$ für Wasserstoff oder $C_1$-$C_4$-Alkyl stehen, und

$Z-COO^{\ominus}Me^{\oplus}$, $-COOR^8$, $-CONR^9R^{10}$, $-COR^{11}$ oder $-CN$, wobei $Me^{\oplus}$ für ein Kation, $R^8$ für Wasserstoff, $C_1$-$C_8$-Alkyl, welches unsubstituiert oder durch Halogen, Cyan oder $C_1$-$C_4$-Alkoxy substituiert ist, $C_2$-$C_4$-Alkinyl, $C_1$-$C_4$-Alkoxy oder $C_3$-$C_6$-Cycloalkylaminocarbonyl und $R^{11}$ für Wasserstoff oder für Wasserstoff, Amino, Hydroxy, $C_1$-$C_4$-Alkyl, welches unsubstituiert oder durch $C_1$-$C_4$-Alkoxy substituiert ist, $C_2$-$C_4$-Alkinyl, $C_1$-$C_4$-Alkoxy oder $C_3$-$C_6$-Cycloalkylaminocarbonyl und $R^{11}$ für Waserstoff oder $C_1$-$C_4$-Alkyl, welches gegebenenfalls durch $C_1$-$C_4$-Alkoxy substituiert ist, stehen, oder

das Strukturelement $-N(R^1)-Q-Z$ einen Pyrrolidinoring, welcher in 2-Postion durch Z und G' substituiert ist, worin G' für Wasserstoff oder $C_1$-$C_4$-Alkyl steht, bedeutet,

mit der Massgabe, dass, wenn $-N(R^1)-$ und $-Z$ unmittelbar nur über ein einziges Kohlenstoffatom miteinander verbunden sind und $R^1$ Wasserstoff bedeutet, Q für $-CH(OR^2)$, $-CH(COOR^4)-$, $-CH(C_1-C_4-$Alkyl, welches durch $-OR^5$, $-SR^6$ oder $-COOR^7$ substituiert ist$)-$, $-C(C_1-C_4-$Alkyl$)(OR^2)-$, $-C(C_1$-$C_4-$Alkyl$)(COOR^4)-$, $-C(CH_3)(C_1-C_3-$Alkyl, welches durch $-OR^5$, $-SR_6$ oder $-COOR^7$ substituiert ist$)-$, $-C(C_2H_5)(C_1-C_2-$Alkyl, welches durch $-OR^5$, $-SR^6$ oder $-COOR^7$ substituiert ist$)-$, $-C(C_3H_7)(CH_2OR^5-$, $-C(C_3H_7)(CH_2SR^6)-$, $-C(C_3H_7)(CH_2COOR^7)-$, $-C(CH_3)(C_3H_7)-$ oder $-C(C_2H_5)_2-$ steht.

Unter $C_1$-$C_4$-Alkyl als Substituent oder Teil eines Substituenten sind Methyl, Aethyl, n-Propyl, Isopropyl, n-Butyl, sec.-Butyl, tert.-Butyl und Isobutyl, unter $C_1$-$C_4$-Alkoxy, Methoxy, Aethoxy, n-Propoxy, Isopropoxy, n-Butoxy, sec.-Butoxy, tert.-Butoxy und Isobutoxy zu verstehen. $C_1$-$C_8$-Alkyl umfasst die vorstehend genannten Alkylgruppen sowie n-Pentyl, n-Hexyl, n-Heptyl, n-Oktyl und ihre Isomeren.

Alkenyl und Alkinyl als Substituenten oder Teil eines Substituenten umfassen die unter die jeweils angegebene Anzahl von Kohlenstoffatomen fallenden geradkettigen und verzweigten Reste.

$C_3$-$C_6$-Cycloalkyl steht für Cyclopropyl, Cyclobutyl, Cyclopentyl und Cyclohexyl.

Als Kation $Me^{\oplus}$ kommen vorzugsweise das Ammoniumkation, ein Metallkation, eine organische Stickstoffbase, ein Sulfoniumkation oder ein Sulfoxoniumkation in Betracht.

Als zur Salzbildung geeignete Metalle kommen Erdalkalimetalle, wie beispielsweise Magnesium oder Calcium, vor allem aber die Alkalimetalle in Betracht, wie beispielsweise Lithium, Kalium und insbesondere Natrium. Ferner sind als Salzbildner auch Uebergangsmetalle wie beispielsweise Eisen, Nickel, Kobalt, Kupfer, Zink, Chrom oder Mangan geeignet.

Beispiele für zur Salzbildung geeignete organische Stickstoffbasen sind primäre, sekundäre und tertiäre, aliphatische und aromatische, gegebenenfalls am Kohlenwasserstoffrest hydroxylierte Amine,

wie Methylamin, Aethylamin, n-Propylamin, Isopropylamin, die vier isomeren Butylamine, Dimethylamin, Diäthylamin, Di-n-propylamin, Diisopropylamin, Di-n-butylamin, Pyrrolidin, Piperidin, Morpholin, Trimethylamin, Triäthylamin, Tri-n-propylamin, Chinuclidin, Pyridin, Chinolin, Isochinolin, sowie Aethanolamin, Propanolamin, Diäthanolamin oder Triäthanolamin in Betracht, sowie die quaternären Ammoniumhydroxyde von Tetraalkylammonium, worin die Alkylreste unabhängig voneinander geradkettige oder verzweigte $C_1$-$C_4$-Alkylgruppen sind, wie Tetramethylammonium, Tetraäthylammonium oder Trimethyläthylammonium, sowie weiterhin Trimethylbenzylammonium, Triäthylbenzylammonium und Trimethyl-2-hydroxyäthylammonium.

Unter Sulfonium- und Sulfoxonium-Kationen sind $-^{\oplus}SH_3$ und $-^{\oplus}S(O)H_3$ zu verstehen, in denen ein oder mehrere Wasserstoffatome durch organische Reste, beispielsweise die vorher für die organischen Stickstoffbasen angeführten Kohlenwasserstoffreste, ersetzt sein können.

Im Rahmen der Definition von Q gilt als Hauptkette diejenige Kohlenstoffkette, welche $-N(R^1)-$ und $-Z$ unmittelbar, d. h. auf direktem oder kürzestem Weg miteinander verbindet. Die angegebene Gesamtzahl von Kohlenstoffatomen gilt für die Hauptkette und von dieser ausgehende Seitenketten, jedoch ohne das Kohlenstoffatom der Carboxylsäure oder ihrer Derivate.

Bevorzugt sind Verbindungen der Formel I, die einer der nachfolgend aufgeführten Verbindungsgruppen angehören :

a) Verbindungen der Formel I, in denen X Wasserstoff, Chlor, Fluor oder Brom, $R^1$ Wasserstoff, Nitroso, $C_1$-$C_4$-Alkyl oder $C_1$-$C_4$-Alkylcarbonyl, welches unsubstituiert oder durch ein oder mehrere Halogenatome substituiert ist, Q einen geradkettigen oder verzweigten Alkylen- oder Alkenylenrest mit bis zu 5 Kohlenstoffatomen, in welchem neben der Hauptkette 0 bis 3 Seitenketten vorliegen, wobei die Hauptkette gesättigt oder ungesättigt und unsubstituiert oder ein- oder zweifach durch Substituenten der Gruppe $-OR^2$, $-SR^3$ oder $-COOR^4$ substituiert ist, wobei keines der Kohlenstoffatome disubstituiert ist, und die Seitenketten ungesättigt und unsubstituiert oder eine oder zwei der Seitenketten durch $-OR^5$, $-SR^6$ oder $-COOR^7$ substituiert sind, wobei gleichzeitig mit der Hauptkette nur eine der Seitenketten substituiert sein kann, und $R^2$, $R^3$, $R^4$, $R^5$, $R^6$ und $R^7$ für Wasserstoff oder $C_1$-$C_4$-Alkyl stehen und Z $-COO^{\ominus}Me^{\oplus}$, $-COOR^8$, $-CONR^9R^{10}$, oder $-CN$, wobei $Me^{\oplus}$ für ein Kation, $R^8$ für Wasserstoff, $C_1$-$C_4$-Alkyl, welches unsubstituiert oder durch Halogen oder $C_1$-$C_4$-Alkoxy substituiert ist, oder $C_2$-$C_4$-Alkenyl, $R^9$ für Wasserstoff, $C_1$-$C_4$-Alkyl oder $C_3$-$C_6$-Cycloalkyl und $R^{10}$ für Wasserstoff, Amino, Hydroxy, $C_1$-$C_4$-Alkyl, welches unsubstituiert oder durch $C_1$-$C_4$-Alkoxy substituiert ist, $C_2$-$C_4$-Alkinyl, $C_1$-$C_4$-Alkoxy oder $C_3$-$C_6$-Cycloalkylaminocarbonyl stehen, oder das Strukturelement $-N(R^1)-Q-Z$ einen Pyrrolidinoring, welcher in 2-Position durch Z und G' substituiert ist, worin G' für Wasserstoff oder $C_1$-$C_4$-Alkyl steht, bedeutet, mit der Massgabe, dass, wenn $-N(R^1)-$ und $-Z$ unmittelbar nur über ein einziges Kohlenstoffatom miteinander verbunden sind und $R^1$ Wasserstoff bedeutet, Q für $-CH(COOR^4)-$, $-CH(C_1$-$C_4$-Alkyl, welches durch $-OR^5$, $-SR^6$ oder $-COOR^7$ substituiert ist)-, $-C(C_1$-$C_4$-Alkyl) $(COOR^4)-$, $-C(CH_3)(C_1$-$C_3$-Alkyl, welches durch $-OR^5$, $-SR^6$ oder $-COOR^7$ substituiert ist)-, $-C(C_2H_5)(C_1$-$C_2$-Alkyl, welches durch $-OR^5$, $-SR^6$ oder $-COOR^7$ substituiert ist)-, $-C(C_3H_7)(CH_2OR^5)-$, $-C(C_3H_7)(CH_2SR^6)-$, $-C(C_3H_7)(CH_2COOR^7)-$, $-C(CH_3)(C_3H_7)-$ oder $-C(C_2H_5)_2$ steht ;

b) Verbindungen der Formel I, in denen X Wasserstoff, Chlor, Fluor oder Brom, $R^1$ Wasserstoff oder $C_1$-$C_4$-Alkyl, Q einen geradkettigen oder verzweigten Alkylenrest mit 1 bis 5 Kohlenstoffatomen, in welchem neben der Hauptkette, welche die Strukturelemente $-N(R^1)-$ und $-Z$ unmittelbar miteinander verbindet, 0 bis 2 Seitenketten vorliegen, wobei die Hauptkette unsubstituiert oder ein- oder zweifach durch $-OR^2$, $-SR^3$ oder $-COOR^4$ substituiert ist, wobei keines der Kohlenstoffatome disubstituiert ist und jedes Kohlenstoffatom mindestens ein Wasserstoffatom trägt, und die Seitenketten unsubstituiert oder durch $-OR^5$, $-SR^6$ oder $-COOR^7$ substituiert sind, wobei gleichzeitg mit der Hauptkette nur eine der Seitenketten substituiert sein kann und $R^2$, $R^3$, $R^4$, $R^5$, $R^6$ und $R^7$ für Wasserstoff oder $C_1$-$C_4$-Alkyl stehen, und Z$-COO^{\ominus}Me^{\oplus}$, $-COOR^8$, $-CONR^9R^{10}$ oder $-CN$, wobei $Me^{\oplus}$ für ein Metallkation, $R^8$ für Wasserstoff oder $C_1$-$C_4$-Alkyl, $R^9$ und $R^{10}$ für Wasserstoff oder $C_1$-$C_4$-Alkyl und $R^{11}$ für durch $C_1$-$C_4$-Alkoxy substituiertes $C_1$-$C_4$-Alkyl stehen, oder das Strukturelement $-N(R^1)-Q-Z$ einen Pyrrolidinoring, in welchem Z in 2-Position steht, bedeutet, mit der Massgabe, dass, wenn $-N(R^1)-$ und $-Z$ unmittelbar nur über ein einziges Kohlenstoffatom miteinander verbunden sind und $R^1$ Wasserstoff bedeutet, Q für $-CH(OR^2)-$, $CH(COOR^4)-$ oder $-CH(C_1$-$C_4$-Alkyl, welches durch $-OR^5$, $-SR^6$ oder $-COOR^7$ substituiert ist)- steht ;

c) Verbindungen der Formel I, in denen X Wasserstoff, Chlor, Fluor oder Brom, $R^1$ Wasserstoff oder $C_1$-$C_4$-Alkyl, Q einen Alkylenrest mit bis zu 5 Kohlenstoffatomen, welcher

a) nur aus der Hauptkette, welche die Strukturelemente $-NR^1-$ und $-Z$ unmittelbar miteinander verbindet und aus 1 bis 3 Kohlenstoffatomen besteht und unsubstituiert oder durch $-OR^2$, $-SR^3$ oder $-COOR^4$ monosubstituiert ist, oder

b) aus der Hauptkette und einer Seitenkette besteht, wobei die Hauptkette aus 1 bis 3 Kohlenstoffatomen besteht und unsubstituiert ist, und die Seitenkette unsubstituiert oder durch $-OR^5$, $-SR^6$ oder $-COOR^7$ substituiert ist, und $R^2$, $R^3$, $R^4$, $R^5$, $R^6$ und $R^7$ für Wasserstoff oder $C_1$-$C_4$-Alkyl stehen, und

Z—$COO^{\ominus}Me^{\oplus}$, —$COOR^8$, —$COR^{11}$ oder —CN, wobei $Me^{\oplus}$ für ein Metallkation, $R^8$ für Wasserstoff oder $C_1$-$C_4$-Alkyl und $R^{11}$ für durch $C_1$-$C_4$-Alkoxy substituiertes $C_1$-$C_4$-Alkyl steht, oder das Strukturelement —$N(R^1)$—Q—Z einen Pyrrolidinoring bedeutet, in welchem Z in 2-Position steht, bedeutet, mit der Massgabe, dass, wenn —$N(R^1)$— und —Z unmittelbar nur über ein einziges Kohlenstoffatom miteinander verbunden sind und $R^1$ Wasserstoff bedeutet, Q für —$CH(OR^2)$—, —$CH(COOR^4)$— oder —CH($C_1$-$C_4$—Alkyl, welches durch —$OR^5$, —$SR^6$ oder —$COOR^7$ substituiert ist)— steht ;

d) Verbindungen der Formel I, in denen X Wasserstoff oder Chlor, $R^1$ Wasserstoff, Nitroso, Methyl, Methylcarbonyl, Aethylcarbonyl, Vinylcarbonyl, Chlormethylcarbonyl, Trichlormethylcarbonyl oder Trifluormethylcarbonyl, Q einen geradkettigen oder verzweigten Alkylen- oder Alkenylenrest mit 1 bis 5 Kohlenstoffatomen, in welchem neben der Hauptkette, welche die Strukturelemente —$N(R^1)$— und —Z unmittelbar miteinander verbindet, 0 bis 2 Seitenketten vorliegen, wobei die Hauptkette gesättigt oder ungesättigt und unsubstituiert oder durch —$OR^2$, —$SR^3$ oder —$COOR^4$ monosubstituiert ist, und die Seitenketten gesättigt und unsubstituiert oder eine der Seitenketten durch —$OR^5$, —$SR^6$ oder —$COOR^7$ substituiert ist, wobei nicht gleichzeitig die Hauptkette und die Seitenketten substituiert sind, und $R^2$, $R^3$, $R^4$, $R^5$, $R^6$ und $R^7$ für Wasserstoff oder $C_1$-$C_4$-Alkyl stehen, und Z—$COO^{\ominus}Me^{\oplus}$, —$COOR^8$, —$CONR^9R^{10}$ oder —CN, wobei $Me^{\oplus}$ für ein Kation, $R^8$ für Wasserstoff, $C_1$-$C_8$-Alkyl, welches unsubstituiert oder durch Halogen oder $C_1$-$C_4$-Alkoxy substituiert ist, $C_2$-$C_8$-Alkenyl oder $C_3$-$C_8$-Alkinyl, $R^9$ für Wasserstoff, $C_1$-$C_4$-Alkyl oder $C_3$-$C_6$-Cycloalkyl und $R^{10}$ für Wasserstoff, Amino, Hydroxy, $C_1$-$C_4$-Alkyl, welches unsubstituiert oder durch $C_1$-$C_4$-Alkoxy substituiert ist, $C_2$-$C_4$-Alkinyl, $C_1$-$C_4$-Alkoxy oder $C_3$-$C_6$-Cycloalkylaminocarbonyl stehen, oder das Strukturelement —$N(R^1)$—Q—Z einen Pyrrolidinoring, welcher in 2-Position durch Z und G' substituiert ist, worin G' für Wasserstoff oder $C_1$-$C_4$-Alkyl steht, bedeutet, mit der Massgabe, dass, wenn —$N(R^1)$— und —Z unmittelbar nur über ein einziges Kohlenstoffatom miteinander verbunden sind und $R^1$ Wasserstoff bedeutet, Q für —$CH(COOR^4)$—, —CH($C_1$—$C_4$—Alkyl, welches durch —$OR^5$, —$SR^6$ oder —$COOR^7$ substituiert ist)-, —C($C_1$-$C_4$—Alkyl)($COOR^4$)—, —C($CH_3$)($C_1$—$C_3$—Alkyl, welches durch —$OR^5$, —$SR^6$ oder —$COOR^7$ substituiert ist)—, —C($C_2H_5$)($C_1$-$C_2$—Alkyl, welches durch —$OR^5$, —$SR^6$ oder —$COOR^7$ substituiert ist)—, —$C(C_3H_7)(CH_2OR^5)$—, —$C(C_3H_7)(CH_2SR^6)$—, —$C(C_3H_7)(CH_2COOR^7)$—, —$C(CH_3)(C_3H_7)$— oder —$C(C_2H_5)_2$— steht.

e) Verbindungen der Formel I, in denen X Wasserstoff oder Chlor, $R^1$ Wasserstoff oder Methyl, Q einen Alkylenrest mit bis zu 5 Kohlenstoffatomen, welcher

a) nur aus der Hauptkette, welche die Strukturelemente —$N(R^1)$— und —Z unmittelbar miteinander verbindet und aus 1 bis 3 Kohlenstoffatomen besteht und unsubstituiert oder durch —$OR^2$, —$SR^3$ oder —$COOR^4$ monosubstituiert ist, oder

b) aus der Hauptkette und einer Seitenkette besteht, wobei die Hauptkette 1 bis 3 Kohlenstoffatome enthält und unsubstituiert ist, und die Seitenkette unsubstituiert oder durch —$OR^5$, —$SR^6$ oder —$COOR^7$ substituiert ist, und $R^2$, $R^3$, $R^4$, $R^5$, $R^6$ und $R^7$ für Wasserstoff oder $C_1$-$C_4$-Alkyl stehen, und Z—$COO^{\ominus}Me^{\oplus}$, —$COOR^8$, —$COR^{11}$ oder —CN, wobei $Me^{\oplus}$ für ein Metallkation, $R^8$ für Wasserstoff oder $C_1$-$C_4$-Alkyl und $R^{11}$ für dur $C_1$-$C_4$-Alkoxy substituiertes $C_1$-$C_4$-Alkyl steht, oder das Strukturelement —$N(R^1)$—Q—Z einen Pyrrolidinoring, in welchem Z in 2-Position steht, bedeutet, mit der Massgabe, dass, wenn —$N(R^1)$— und —Z unmittelbar nur über ein einziges Kohlenstoffatom miteinander verbunden sind und $R^1$ Wasserstoff bedeutet, Q für —$CH(OR^2)$—, —$CH(COOR^4)$— oder —CH($C_1$-$C_4$-Alkyl, welches durch —$OR^5$, —$SR^6$ oder —$COOR^7$ substituiert ist)— steht ;

f) Verbindungen der Formel I, in denen X Wasserstoff oder Chlor, $R^1$ Wasserstoff, Nitroso oder $C_1$-$C_4$-Alkyl, $C_2$-$C_4$-Alkenyl, $C_1$-$C_4$-Alkylcarbonyl oder $C_2$-$C_4$-Alkenylcarbonyl, welche unsubstituiert oder durch 1 bis 3 Chlor- oder Fluoratome substituiert sind, Q 1,5-Pentamethylen oder einen Alkylen- oder Alkenylenrest mit insgesamt bis zu

a) nur aus der Hauptkette, welche die Strukturelemente —$N(R^1)$— und —Z unmittelbar miteinander verbindet und gesättigt oder ungesättigt und unsubstituiert oder durch Carboxyl oder $C_1$-$C_4$-Alkoxycarbonyl monosubstituiert ist, besteht, oder

b) aus einer Hauptkette, welche aus 2 Kohlenstoffatomen besteht und gesättigt oder ungesättigt ist, und einer Seitenkette mit einem Kohlenstoffatom besteht, wobei i) die Hauptkette unsubstituiert oder durch Carboxyl oder $C_1$-$C_4$-Alkoxycarbonyl monosubstituiert ist und die Seitenkette aus einer unsubstituiert en Methylgruppe besteht, oder ii) die Hauptkette unsubstituiert ist und die Seitenkette aus einer Methylgruppe, welche durch Hydroxy oder $C_1$-$C_4$-Alkylthio substituiert ist, besteht, oder

c) —CH(COOH)—, —CH($COOC_1$—$C_4$—Alkyl)—, —C($C_1$—$C_2$—Alkyl)(COOH)—, —C($C_1$-$C_2$-Alkyl)($CCOC_1$—$C_4$—Alkyl)—, —$CH(CH_2OH)$—, —CH($CH_2$—S—$C_1$—$C_4$—Alkyl)—, —$CH(C_2H_5OH)$—, —CH($C_2H_5$—S—$C_1$—$C_4$—Alkyl)—, —$C(CH_3)(CH_2OH)$—, —C($CH_3$)($CH_2$—S—$C_1$—$C_4$—Alkyl)—, —$C(CH_3)_2$ oder —CH($C_1$—$C_2$—Alkyl) und Z—$COO^{\ominus}Me^{\oplus}$, —$COOR^8$, —$CONR^9R^{10}$ oder —CN, wobei $Me^{\oplus}$ für ein Alkalimetallkation, $R^8$ für Wasserstoff, $C_1$-$C_8$-Alkyl, welches unsubstituiert oder durch Chlor oder $C_1$-$C_4$-Alkoxy monosubstituiert ist, $C_2$-$C_8$-Alkenyl oder $C_3$-$C_8$-Alkinyl, $R^9$ für Wasserstoff, $C_1$-$C_4$-Alkoxy oder $C_3$-$C_6$-Cycloalkyl und $R^{10}$ für Wasserstoff, Amino, Hydroxy, $C_1$-$C_4$-Alkyl, welches unsubstituiert oder durch $C_1$-$C_4$-Alkoxy monosubstituiert ist, $C_2$-$C_4$-Alkenyl, $C_1$-$C_4$-Alkoxy oder $C_3$-$C_6$-Cycloalkylaminocarbonyl stehen, oder das Strukturelement —$N(R^1)$—Q—Z einen Pyrrolidinoring, welcher in 2-Position

durch Z und G' substituiert ist, worin G' für Wasserstoff oder $C_1$-$C_4$-Alkyl steht, bedeuten, mit der Massgabe, dass, wenn —N($R^1$) und —Z unmittelbar nur über ein einziges Kohlenstoffatom miteinander verbunden sind und $R^1$ Wasserstoff bedeutet, Q für —CH(COOH)—, —CH(COOC$_1$—C$_4$—Alkyl)—, —C(C$_1$—C$_2$—Alkyl)(COOH)—, —C(C$_1$—C$_2$—Alkyl)(COOC$_1$—C$_4$—Alkyl)—, —CH(CH$_2$OH)—, —CH(CH$_2$—S—C$_1$—C$_4$—Alkyl)—, —CH(C$_2$H$_5$OH)—, —CH(C$_2$H$_5$—S—C$_1$—C$_4$—Alkyl)—, —C(CH$_3$)(CH$_2$OH)—, —C(CH$_3$)(CH$_2$—S—C$_1$—C$_4$—Alkyl)— oder —C(CH$_3$)$_2$ steht ;

g) Verbindungen der Formel I, in denen X Wasserstoff, $R^1$ Wasserstoff, Q eine 1,2-Aethylenbrücke und Z—COO$^\ominus$Me$^\oplus$, —COOR$^8$, —COSR$^8$, —CONR$^9$R$^{10}$, —COR$^{11}$ oder —CN, wobei Me$^\oplus$ für ein Kation, $R^8$ für Wasserstoff, $C_1$-$C_8$-Alkyl, welches unsubstituiert oder durch Halogen, Cyan oder $C_1$-$C_4$-Alkoxy substituiert ist, $C_2$-$C_8$-Alkenyl oder $C_3$-$C_8$-Alkinyl, $R^9$ für Wasserstoff, $C_1$-$C_4$-Alkyl oder $C_3$-$C_6$-Cycloalkyl, $R^{10}$ für Wasserstoff, Amino, Hydroxy, $C_1$-$C_4$-Alkyl, welches unsubstituiert oder durch $C_1$-$C_4$-Alkoxy substituiert ist, $C_2$-$C_4$-Alkinyl, $C_1$-$C_4$-Alkoxy oder $C_3$-$C_6$-Cycloalkylaminocarbonyl und $R^{11}$ für Wasserstoff oder $C_1$-$C_4$-Alkyl, welches gegebenenfalls durch $C_1$-$C_4$-Alkoxy substituiert ist, stehen, bedeuten ;

h) Verbindungen der Formel I, in denen X Wasserstoff, Chlor, Fluor oder Brom, $R_1$ Wasserstoff, Nitroso oder $C_1$-$C_4$-Alkyl, $C_2$-$C_4$-Alkenyl, $C_1$-$C_4$-Alkylcarbonyl oder $C_2$-$C_4$-Alkenylcarbonyl, welche unsubstituiert oder durch ein oder mehrere Halogenatome substituiert sind, Q 1,5-Pentamethylen oder einen Alkylen- oder Alkenylenrest mit insgesamt bis zu 3 Kohlenstoffatomen, welcher

a) nur aus der Hauptkette, welche die Strukturelemente —N($R^1$)— und —Z unmittelbar miteinander verbindet und gesättigt oder ungesättigt und unsubstituiert oder durch Carboxyl, Methoxycarbonyl oder Aethoxycarbonyl monosubstituiert ist, besteht, oder

b) aus einer Hauptkette, welche aus 2 Kohlenstoffatomen besteht und gesättigt oder ungesättigt ist, und einer Seitenkette mit einem Kohlenstoffatom besteht, wobei i) die Hauptkette unsubstituiert oder durch Carboxyl, Methoxycarbonyl oder Aethoxycarbonyl monosubstituiert ist und die Seitenkette aus einer unsubstituierten Methylgruppe besteht, oder ii) die Hauptkette unsubstituiert ist und die Seitenkette Hydroxymethyl ist, besteht, oder

c) aus einem Mitglied der Gruppe —CH(COOH)—, —CH(COOCH$_3$)—, —CH(COOC$_2$H$_5$)—, —CH(CH$_2$OH)—, —CH(CHOH—CH$_3$)—, —CH(CH$_2$CH$_2$OH)—, —CH(CH$_2$CH$_2$—S—CH$_3$)—, —C(CH$_3$)—COOH)—, —C(CH$_3$)(COOCH$_3$)—, —C(CH$_3$)(COOC$_2$H$_5$)—, —C(CH$_3$)(CH$_2$OH)—, —CH(C$_1$—C$_2$—Alkyl)— oder —C(CH$_3$)$_2$ besteht, und Z—COO$^\ominus$Me$^\oplus$, —COOR$^8$, —CONR$^9$R$^{10}$ oder —CN, wobei Me$^\oplus$ für ein Natriumkation, $R^8$ für Wasserstoff, $C_1$-$C_4$-Alkyl, 2-Chloräthyl, 2-Methoxyäthyl, Allyl oder 2-Propinyl, $R^9$ für Wasserstoff, Methyl oder Cyclohexyl und $R^{10}$ für Wasserstoff, Amino, Hydroxy, $C_1$-$C_3$-Alkyl, 2-Methoxyäthyl, 3-Butin-2-yl, Methoxy oder Cyclohexylaminocarbonyl stehen, oder das Strukturelement —N($R^1$)—Q—Z einen Pyrrolidinoring, welcher in 2-Position durch Z und G' substituiert ist, worin G' für Wasserstoff oder Methyl steht, bedeutet, mit der Massgabe, dass, wenn —N($R^1$)— und —Z unmitelbar nur über ein einziges Kohlenstoffatom miteinander verbunden sind und $R^1$ Wasserstoff bedeutet, Q für —C(CH$_3$)$_2$—, —CH(COOH)—, —CH(COOCH$_3$)—, —CH(COOC$_2$H$_5$)—, —CH(CH$_2$OH)—, —CH(CHOH—CH$_3$)—, —CH(CH$_2$CH$_2$OH)—, —CH(CH$_2$CH$_2$—S—CH$_3$)—, —C(CH$_3$)(COOH)—, —C(CH$_3$)(COOCH$_3$)—, —C(CH$_3$)(COOC$_2$H$_5$)— oder —C(CH$_3$)(CH$_2$OH)— steht ;

i) Verbindungen der Formel I, in denen X Wasserstoff oder chlor, $R^1$ Wasserstoff, Nitroso, $C_1$-$C_4$-Alkyl, $C_1$-$C_4$-Alkylcarbonyl, welches unsubstituiert oder durch 1 bis 3 Chlor- oder Fluoratome substituiert ist oder $C_2$-$C_4$-Alkenylcarbonyl, Q 1,5-Pentamethylen oder einen Alkylen- oder Alkenylenrest mit insgesamt bis zu 3 Kohlenstoffatomen, welcher

a) nur aus der Hauptkette, welche die Strukturelemente —N($R^1$)— und —Z unmittelbar miteinander verbindet und gesättigt oder ungesättigt und unsubstituiert oder durch Carboxyl oder $C_1$-$C_4$-Alkoxycarbonyl monosubstituiert ist, besteht, oder

b) aus einer Hauptkette, welche aus 2 Kohlenstoffatomen besteht und gesättigt oder ungesättigt ist, und einer Seitenkette mit einem Kohlenstoffatom besteht, wobei i) die Hauptkette unsubstituiert oder durch Carboxy oder $C_1$-$C_4$-Alkoxycarbonyl monosubstituiert ist und die Seitenkette aus einer unsubstituierten Methylgruppe besteht, oder ii) die Hauptkette unsubstituiert ist und die Seitenkette aus einer Methylgruppe, welche durch Hydroxy oder $C_1$-$C_4$-Alkylthio substituiert ist, besteht, oder

c) —CH(COOH)—, —CH(COOC$_1$—C$_4$—Alkyl)—, —C(C$_1$—C$_2$—Alkyl)(COOH)—, —C(C$_1$—C$_2$—Alkyl)(COOC$_1$—C$_4$—Alkyl)—, —CH(CH$_2$OH)—, —CH(CH$_2$—S—C$_1$—C$_4$—Alkyl)—, —CH(C$_2$H$_5$OH)—, —CH(C$_2$H$_5$—S—C$_1$—C$_4$—Alkyl)—, —C(CH$_3$)(CH$_2$OH)—, —C(CH$_3$)(CH$_2$—S—C$_1$—C$_4$—Alkyl)—, —C(CH$_3$)$_2$ oder —CH(C$_1$—C$_2$—Alkyl) und Z—COO$^\ominus$Me$^\oplus$, —COOR$^8$, —CONR$^9$R$^{10}$ oder —CN, wobei Me$^\oplus$ für ein Alkalimetallkation, $R^8$ für Wasserstoff, $C_1$-$C_4$-Alkyl, welches unsubstituiert oder durch Chlor oder $C_1$-$C_4$-Alkoxy monosubstituiert ist, oder $C_2$-$C_4$-Alkenyl, $R^9$ für Wasserstoff, $C_1$-$C_4$-Alkyl oder $C_3$-$C_6$-Cycloalkyl und $R^{10}$ für Wasserstoff, Amino, Hydroxy, $C_1$-$C_4$-Alkyl, welches unsubstituiert oder durch $C_1$-$C_4$-Alkoxy monosubstituiert ist, $C_2$-$C_4$-Alkinyl, $C_1$-$C_4$-Alkoxy oder $C_3$-$C_6$-Cycloalkylaminocarbonyl stehen, oder das Strukturelement —N($R^1$)—Q—Z einen Pyrrolidinoring, welcher in 2-Position durch Z und G' substituiert ist, worin G' für Wasserstoff oder $C_1$-$C_4$-Alkyl steht, bedeutet, mit der Massgabe, dass, wenn —N($R^1$)— und —Z unmittelbar nur über ein

einziges Kohlenstoffatom miteinander verbunden sind und $R^1$ Wasserstoff bedeutet, Q für —CH(COOH)—, —CH(COOC$_1$—C$_4$—Alkyl)—, —C(C$_1$—C$_2$—Alkyl)(COOH)—, —C(C$_1$—C$_2$—Alkyl)(COOC$_1$—C$_4$—Alkyl)—, —CH(CH$_2$OH)—, —CH(CH$_2$—S—C$_1$—C$_4$—Alkyl)—, —CH(C$_2$H$_5$OH)—, —CH(C$_2$H$_5$—S—C$_1$—C$_4$—Alkyl)—, —C(CH$_3$)(CH$_2$OH)—, —C(CH$_3$)(CH$_2$—S—C$_1$—C$_4$—Alkyl)— oder —C(CH$_3$)$_2$ steht ;

k) Verbindungen der Formel I, in denen X Wasserstoff oder Chlor, $R^1$ Wasserstoff, Nitroso, Methyl, Methylcarbonyl, Aethylcarbonyl, Vinylcarbonyl, Chlormethylcarbonyl, Trichlormethylcarbonyl oder Trifluormethylcarbonyl, Q 1,5-Pentamethylen oder einen Alkylen- oder Alkenylenrest mit insgesamt bis zu 3 Kohlenstoffatomen, welcher

a) nur aus der Hauptkette, welche die Strukturelemente —N($R^1$)— und —Z unmittelbar miteinander verbindet und gesättigt oder ungesättigt und unsubstituiert oder durch Carboxyl, Methoxycarbonyl oder Aethoxycarbonyl monosubstituiert ist, besteht, oder

b) aus einer Hauptkette, welche aus 2 Kohlenstoffatomen besteht und gesättigt oder ungesättigt ist, und einer Seitenkette mit einem Kohlenstoffatom besteht, wobei i) die Hauptkette unsubstituiert oder durch Carboxyl, Methoxycarbonyl oder Aethoxycarbonyl monosubstituiert ist und die Seitenkette aus einer unsubstituierten Methylgruppe besteht, oder ii) die Hauptkette unsubstituiert ist und die Seitenkette Hydroxymethyl ist, oder

c) aus einem Mitglied der Gruppe —CH(COOH)—, —CH(COOCH$_3$)—, —CH(COOC$_2$H$_5$)—, —CH(CH$_2$OH)—, —CH(CHOH—CH$_3$)—, —CH(CH$_2$CH$_2$OH)—, —CH(CH$_2$CH$_2$—S—CH$_3$)—, —C(CH$_3$)(COOH)—, —C(CH$_3$)(COOCH$_3$)—, —C(CH$_3$)(COOC$_2$H$_5$)—, —C(CH$_3$)(CH$_2$OH)— oder —C(CH$_3$)$_2$— besteht, und Z—COO$^\ominus$Me$^\oplus$, —COOR$^8$, —CONR$^9$R$^{10}$ oder —CN, wobei Me$^\oplus$ für ein Natriumkation, $R^8$ für Wasserstoff, C$_1$-C$_4$-Alkyl, 2-Chloräthyl, 2-Methoxyäthyl, Allyl oder 2-Propinyl, $R^9$ für Wasserstoff, Methyl oder Cyclohexyl und $R^{10}$ für Wasserstoff, Amino, Hydroxy, C$_1$-C$_3$-Alkyl, 2-Methoxyäthyl, 3-Butin-2-yl, Methoxy oder Cyclohexylaminocarbonyl stehen, oder das Strukturelement —N($R^1$)—Q—Z einen Pyrrolidinoring, welcher in 2-Position durch Z und G' substituiert ist, worin G' für Wasserstoff oder Methyl steht, bedeutet, mit der Massgabe, dass, wenn —N($R^1$)— und —Z unmittelbar nur über ein einziges Kohlenstoffatom miteinander verbunden sind und $R^1$ Wasserstoff bedeutet, Q für —C(CH$_3$)$_2$—, —CH(COOH)—, —CH(COOCH$_3$)—, —CH(COOC$_2$H$_5$)—, —CH(CH$_2$OH)—, —CH(CHOH—CH$_3$)—, —CH(CH$_2$CH$_2$OH)—, —CH(CH$_2$CH$_2$—S—CH$_3$)—, —C(CH$_3$)(COOH)—, —C(CH$_3$)(COOCH$_3$)—, —C(CH$_3$)(COOC$_2$H$_5$)— oder —C(CH$_3$)(CH$_2$OH)— steht ;

l) Verbindungen der Formel I, in denen X Wasserstoff oder Chlor, $R^1$ Wasserstoff, Nitroso, Methyl, Methylcarbonyl, Aethylcarbonyl, Chlormethylcarbonyl, Trichlormethylcarbonyl oder Trifluormethylcarbonyl, Q 1,5-Pentamethylen oder einen Alkylen- oder Alkenylenrest mit bis zu 3 Kohlenstoffatomen, welcher

a) nur aus der Hauptkette, welche die Strukturelemente —N($R^1$)— und —Z unmittelbar miteinander verbindet und gesättigt oder ungesättigt und unsubstituiert oder durch Carboxyl oder Aethoxycarbonyl monosubstituiert ist, besteht, oder

b) aus einer Hauptkette, welche aus 2 Kohlenstoffatomen besteht und gesättigt oder ungesättigt ist, und einer Seitenkette mit einem Kohlenstoffatom besteht, wobei i) die Hauptkette unsubstituiert oder durch Carboxyl oder Aethoxycarbonyl monosubstituiert ist und die Seitenkette aus einer unsubstituierten Methylgruppe besteht, oder ii) die Hauptkette unsubstituiert und die Seitenkette Hydroxymethyl ist, oder

c) aus einem Mitglied der Gruppe —CH(COOH)—, —CH(COOC$_2$H$_5$)—, —CH(CH$_2$OH)—, —CH(CHOH—CH$_3$)—, —CH(CH$_2$CH$_2$—S—CH$_3$)—, —C(CH$_3$)(COOH)—, —CH(CH$_3$)(COOC$_2$H$_5$)—, —C(CH$_3$)(CH$_2$OH)— oder —C(CH$_3$)$_2$ besteht, und Z—COOR$^8$, —CONR$^9$R$^{10}$ oder —CN, wobei $R^8$ für Wasserstoff, C$_1$-C$_4$-Alkyl, 2-Chloräthyl, 2-Methoxyäthyl oder Allyl, $R^9$ für Wasserstoff, Methyl oder Cyclohexyl und $R^{10}$ für Wasserstoff, Amino, Hydroxy, C$_1$-C$_3$-Alkyl, 2-Methoxyäthyl, 3-Butin-2-yl, Methoxy oder Cyclohexylaminocarbonyl stehen, oder das Strukturelement —N($R^1$)—Q—Z einen Pyrrolidinoring, welcher in 2-Position durch Z und G' substituiert ist, worin G' für Wasserstoff oder Methyl steht, bedeutet, mit der Massgabe, dass, wenn —N($R^1$)— und —Z unmittelbar nur über ein einziges Kohlenstoffatom miteinander verbunden sind und $R^1$ Wasserstoff bedeutet, Q für —CH(COOH)—, —CH(COOC$_2$H$_5$)—, —CH(CH$_2$OH)—, —CH(CHOH—CH$_3$)—, —CH(CH$_2$CH$_2$—S—CH$_3$)—, —C(CH$_3$)(COOH)—, —C(CH$_3$)(COOC$_2$H$_5$)—, —C(CH$_3$)(CH$_2$OH)— oder —C(CH$_3$)$_2$— steht ;

m) Verbindungen der Formel I, in denen X Wasserstoff, $R^1$ Wasserstoff oder Methyl, Q ein Mitglied der Gruppe —CH$_2$—, —(CH$_2$)$_2$—, —(CH$_2$)$_3$—, —CH(CH$_3$)—CH$_2$—, —CH$_2$—CH(CH$_3$)—, —CH(CH$_2$OH)—CH$_2$—, —CH$_2$—CH(CH$_2$OH)—, —CH(CH$_3$)—, —CH(CH$_2$OH)—, —CH(CHOH—CH$_3$)— oder —CH(CH$_2$CH$_2$—S—CH$_3$)— und Z —COOH, —COOC$_1$—C$_4$—Alkyl, —COO—CH$_2$—CH$_2$—O—CH$_3$ oder —CN, oder das Strukturelement —N($R^1$)—Q—Z einen Pyrrolidinoring, in welchem Z in 2-Position steht, bedeutet, mit der Massgabe, dass, wenn —N($R^1$)— und —Z unmittelbar nur über ein einziges Kohlenstoffatom miteinander verbunden sind und $R^1$ Wasserstoff bedeutet, Q für —CH(CH$_2$OH)—, —CH(CHOH—CH$_3$)— oder —C(CH$_2$CH$_2$—S—CH$_3$)— steht.

n) Verbindungen der Formel I, in denen X Wasserstoff, $R^1$ Wasserstoff, Q eine Aethylen- oder

Trimethylenbrücke und Z —$COOR^8$ oder —$CONR^9R^{10}$, wobei $R^8$ für Wasserstoff, $C_1$-$C_3$-Alkyl oder Allyl und $R^9$ und $R^{10}$ für Wasserstoff stehen, bedeuten.

Speziell bevorzugte Verbindungen sind :
N-[2-nitro-5-(2-chlor-4-trifluormethyl-phenoxy)-phenyl]-3-amino-propionsäure-methylester,
N-[2-nitro-5-(2-chlor-4-trifluormethyl-phenoxy)-phenyl]-4-amino-n-buttersäure-methylester,
N-[2-nitro-5-(2-chlor-4-trifluormethyl-phenoxy)-phenyl]-3-amino-propionsäure-isopropylester,
N-[2-nitro-5-(2-chlor-4-trifluormethyl-phenoxy)-phenyl]-3-amino-propionsäure-äthylester,
N-[2-nitro-5-(2-chlor-4-trifluormethyl-phenoxy)-phenyl]-3-amino-propionsäure,
N-[2-nitro-5-(2-chlor-4-trifluormethyl-phenoxy)-phenyl]-4-amino-n-buttersäure,
3-[N-{2-nitro-5-(2-chlor-4-trifluormethyl-phenoxy)-phenyl}-amino]-propionsäure-amid und
N-[2-nitro-5-(2-chlor-4-trifluormethyl-phenoxy)-phenyl]-3-amino-proprionsäure-allylester.
Die Herstellung von Verbindungen der Formel I erfolgt, indem man
a) eine Verbindung der Formel II

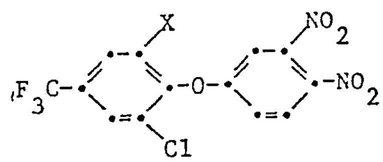

(II)

worin X die für Formel I angegebene Bedeutung hat, mit einer Verbindung der Formel III

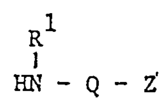

(III)

worin $R^1$, Q und Z die für Formel I angegebenen Bedeutungen haben, in Gegenwart einer Base umsetzt, oder

b) eine Verbindung der Formel IV

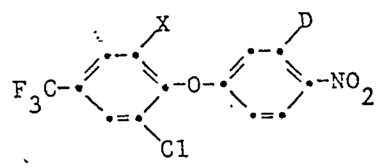

(IV)

worin X die für Formel I angegebene Bedeutung hat und D einen abspaltbaren Rest bedeutet, mit einer Verbindung der Formel III

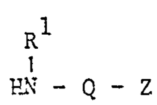

(III)

worin $R^1$, Q und Z die für Formel I angegebenen Bedeutungen haben, umsetzt, oder

c) zur Herstellung von Verbindungen der Formel I, in denen $R^1$ für $C_1$-$C_4$-Alkylcarbonyl steht, eine Verbindung der Formel V

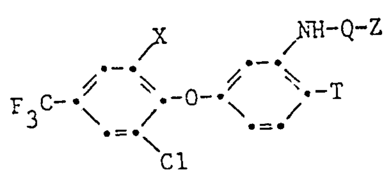

(V)

worin X, Q und Z die für Formel I angegebenen Bedeutungen haben und T Wasserstoff oder Nitro bedeutet, mit einem Säureanhydrid oder Säurechlorid umsetzt und, wenn T Wasserstoff bedeutet, das erhaltene Reaktionsprodukt nitriert, oder

d) eine Verbindung der Formel VII

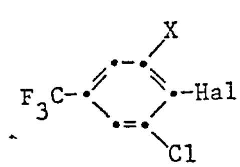

(VII)

worin X die für Formel I angegebene Bedeutung hat und Hal ein Halogenatom bedeutet, mit einer Verbindung der Formel VIII

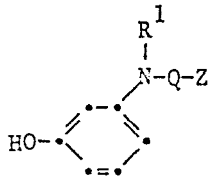

(VIII)

worin $R^1$, Q und Z die für Formel I angegebenen Bedeutungen haben, in Gegenwart einer Base bei erhöhter Temperatur umsetzt und das erhaltene Reaktionsprodukt der Formel IX

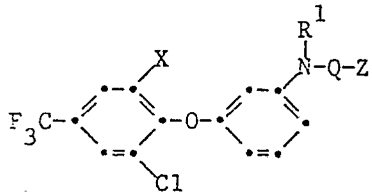

(IX)

worin X, $R^1$, Q und Z die für Formel I angegebenen Bedeutungen haben, nitriert, oder

e) zur Herstellung von Verbindungen der Formel I, in denen $R^1$ für $C_1$-$C_4$-Alkyl steht, eine Verbindung der Formel V, worin X, Q, Z und T die angegebenen Bedeutungen haben, mit einem entsprechenden Alkylierungsmittel umsetzt, und

f) gegebenenfalls die erhaltenen Verbindungen der Formel I, in denen Z für eine Carboxylgruppe steht, in an sich bekannter Weise in die entsprechenden Salze, Ester, Amide, Ketone oder Nitrile überführt.

Die Ausgangsverbindungen der Formeln II, III, IV, V, VI, VII, VIII und IX sind bekannt oder lassen sich analog bekannten Verfahren herstellen.

Die Umsetzungen gemäss den Verfahrensvarianten a, b, c, d und e können ohne oder in Gegenwart von gegenüber den Reaktionsteilnehmern inerten Lösungs- oder Verdünnungsmitteln durchgeführt werden. Bevorzugt sind organische Lösungsmittel wie beispielsweise niedermolekulare Alkohole, Ketone, wie beispielsweise Methyläthylketon, oder Dimethylformamid, Dimethylsulfoxid oder chlorierte Kohlenwasserstoffe, Aether oder aromatische Kohlenwasserstoffe.

Die Reaktionstemperaturen liegen zwischen 0 und 200 °C, vorzugsweise im Bereich von 20 bis 100 °C.

Als Basen kommen beispielsweise KOH, $NaOCH_3$, $K_2CO_3$, Kalium-tert.-butylat oder organische Basen wie Triäthylamin, in Betracht.

Als abspaltbare Reste D in Verfahrensvariante b) kommen beispielsweise Halogen, eine Methoxy-, 2-Chlor-4-trifluormethyl-phenoxy- oder 2,6-Dichlor-4-trifluormethyl-phenoxygruppe in Betracht.

Als Alkylierungsmittel kommen die entsprechenden Alkylhalogenide oder Alkylsulfate in Betracht.

Die Nitrierung des Reaktionsproduktes der Formel IX erfolgt auf an sich bekannte Weise durch Behandeln mit einem der üblichen Nitriersäuregemische. Als solche können beispielsweise konzentrierte Schwefelsäure/Salpetersäuregemische oder konzentrierte Schwefelsäure und Alkalinitratsalze verwendet werden.

Zur Applikation als Herbizide, Desiccants oder Defoliants werden die Verbindungen der Formel I in unveränderter Form oder vorzugsweise zusammen mit den in der Formulierungstechnik üblichen Hilfsmitteln eingesetzt und werden daher z. B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, auch Verkapselungen in z. B. polymeren Stoffen in bekannter Weise verarbeitet. Die Anwendungsverfahren wie Versprühen, Vernebeln, Verstäuben, Verstreuen, Bestreichen oder Giessen werden gleich wie die Art der Mittel den angestreben Zielen und den gegebenen Verhältnissen entsprechend gewählt.

Die Formulierungen, d. h., die den Wirkstoff der Formel I und gegebenenfalls einen festen oder flüssigen Zusatzstoff enthaltenden Mittel, Zubereitungen oder Zusammensetzungen werden in bekannter Weise hergestellt, z. B. durch inniges Vermischen und/oder Vermahlen der Wirkstoffe mit Steckmitteln, wie z. B. mit Lösungsmitteln, festen Trägerstoffen, und gegebenenfalls oberflächenaktiven Verbindungen (Tensiden).

Als Lösungsmittel können in Frage kommen : Aromatische Kohlenwasserstoffe, bevorzugt die Fraktionen $C_8$ bis $C_{12}$, wie z. B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl ; oder Wasser.

Als feste Trägerstoffe, z. B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z. B. Bimsstein, Ziegelbruch, Sepiolit oder Bentonit, als nicht sorptive Trägermaterialien, z. B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z. B. die Na- oder K-Salze der Oel- oder Stearinsäure, oder von natürlichen Fettsäuregemischen genannt, die z. B. aus Kokosnuss- oder Talgöl gewonnen werden können. Ferner sind auch die Fettsäure-methyl-laurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z. B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z. B. die Na-, Ca- oder triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z. B. Salze des Phosphorsäureesters eines p-Nonylphenol-(4-14)-Aethylenoxyd-Adduktes in Frage.

Als nichtionische Tenside kommen in erster Linie Polyglykoläther-derivate von aliphatisfchen oder cycloaliphatischen Alkoholen, gesättigten oder ungesättigten Fettsäuren und Alkylphenolen in Frage, die 3 bis 30 Glykolätergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxydaddukte und Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölglykoläther, Polypropylen-Polyäthylenoxydaddukte, Tributylphenoxypolyäthoxyäthanol, Polyäthylenglykol und Octylphenoxypolyäthoxyäthanol erwähnt.

Ferner kommen auch Fettsäureester von Polyoxyäthylensorbitan wie das Polyoxyäthylensorbitantrioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide; Methylsulfate oder Aethylsulfate vor, z. B. das Stearyltrimethylammoniumchlorid oder das Benzyldi(2-chloräthyl)äthylammoniumbromid.

Die in der Formulierungstechnik gebräuchlichen Tenside sind u. a. in folgenden Publikation beschrieben :

« McCutcheon's Detergents and Emulsifiers Annual » MC Publishing Corp., Ridgewood New Jersey, 1980 Sisley and Wood, « Encyclopedia of Surface Active Agents », Chemical Publishing Co., Inc. New York, 1980.

Die agrochemischen Zubereitungen enthalten in der Regel 0,1 bis 99 %, insbesondere 0,1 bis 95 %, Wirkstoff der Formel I, 99,9 bis 1 %, insbesondere 99,8 bis 5 % eines festen oder flüssigen Zusatzstoffes und 0 bis 25 %, insbesondere 0,1 bis 25 %, eines Tensides.

Während als Handelsware eher konzentrierte Mittel bevorzugt werden, verwendet der Endverbraucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die neuen Verbindungen der Formel I weisen eine herbizide, insbesondere eine ausgeprägte selektivherbizide Wirksamkeit gegen Unkräuter in verschiedenen, vorzugsweise monokotylen Kulturpflanzenbeständen auf. Als monokotyle Kulturpflanzen kommen vor allem Weizen, Gerste, Roggen,

Hafer, Hirse, Mais und Reis in Betracht, wobei die Wirkung in Kulturen von Weizen, Gerste und Reis besonders hervorzuheben ist.

Die Wirkstoffe können zur pre- oder postemergenten Bekämpfung von Unkräutern angewendet werden.

Die Verbindungen der Formel I oder sie enthaltende Mittel eignen sich besonders zur postemergenten Bekämpfung von Unkräutern in Sojakulturen. Ein ganz besonders bevorzugtes Einsatzgebiet ist jedoch die selektive Bekämpfung von Unkräutern, vor allem dikotylen Unkräutern, in Getreidekulturen, insbesondere bei pre-emergenter, aber auch bei post-emergenter Applikation. Hierbei erweisen sich insbesondere diejenigen Verbindungen als vorteilhaft, in denen X und $R^1$ Wasserstoff, Q eine Aethylen- oder Trimethylenbrücke und Z—$COOR^8$ oder —$CONR^9R^{10}$ bedeuten, wobei $R^8$ für Wasserstoff oder $C_1$-$C_3$-Alkyl und $R^9$ und $R^{10}$ für Wasserstoff stehen, vor allem die Verbindungen N-[2-Nitro-5-(2-chlor-4-trifluormethyl-phenoxy)-phenyl]-3-amino-propionsäure-methylester, N-[2-Nitro-5-(2-chlor-4-trifluormethyl-phenoxy)-phenyl]-4-amino-n-buttersäure-methylester, N-[2-Nitro-5-(2-chlor-4-trifluormethylphenoxy)-phenyl]-3-amino-propionsäure-isopropylester, N-[2-Nitro-5-(2-chlor-4-trifluormethyl-phenoxy)-phenyl]-3-amino-propionsäureäthylester, N-[2-Nitro-5-(2-chlor-4-trifluormethyl-phenoxy)-phenyl]-3-aminopropionsäure, N-[2-Nitro-5-(2-chlor-4-trifluormethyl-phenoxy)-phenyl]-4-amino-n-buttersäure und 3-[N-(2-Nitro-5-(2-chlor-4-trifluormethyl-phenoxy)-phenyl)-amino]-propionsäure-amid.

Die Verbindungen der Formel I und sie enthaltende Mittel eignen sich ferner ausgezeichnet zur Desiccation und Defoliation von Baumwoll- und Kartoffelpflanzen. Die Behandlung der Pflanzen erfolgt vor der Ernte, wobei der genaue Zeitpunkt von verschiedenen Faktoren, wie beispielsweise Alter und Zustand der Pflanzen und klimatischen Bedingungen, abhängt. Zweckmässigerweise erfolgt die Behandlung der Pflanzen etwa zwei Wochen vor der Ernte. Besonders geeignet für dieses Einsatzgebiet sind Verbindungen der Formel I, in denen X und $R^1$ Wasserstoff, Q eine Aethylen- oder Trimethylenbrücke bilden und Z Methoxycarbonyl oder Allyloxycarbonyl bedeuten, insbesondere die Verbindungen N-[2-nitro-5-(2-chlor-4-trifluormethyl-phenoxy)-phenyl]-4-amino-n-buttersäure-methylester, N-[2-nitro-5-(2-chlor-4-trifluormethyl-phenoxy)-phenyl]-3-amino-propionsäure-methylester und N-[2-nitro-5-(2-chlor-4-trifluormethyl-phenoxy)-phenyl]-3-amino-propionsäure-allylester.

## Beispiel 1

N-[2-nitro-5-(2-chlor-4-trifluormethyl-phenoxy)-phenyl]-4-amino-n-buttersäure (Verbindung Nr. 1)

9 g 3,4-Dinitro-2'-chlor-4'-trifluormethyl-diphenyläther werden in 20 ml Dimethylsulfoxid, 3 ml Wasser, 5,1 g 4-Amino-n-buttersäure und 5 ml 30 %-iger Natronlauge verrührt. Zur Vervollständigung der exothermen Reaktion wird das Reaktionsgemisch kurz auf 110 °C erhitzt. Anschliessend wird das Reaktionsgemisch in ca. 500 ml Wasser gegossen und einmal mit ca. 100 ml Toluol extrahiert. Die so gereinigte wässerige Phase wird mit Eisessig angesäuert und mehrmals mit Essigsäureäthylester extrahiert. Die vereinigten Essigesterlösungen werden auf ein Volumen von ca. 30 ml eingeengt und mit ca. 100 ml Hexan versetzt. Man erhält 9 g N-[2-nitro-5-(2-chlor-4-trifluormethylphenoxy)-phenyl]-4-amino-n-buttersäure in kristalliner Form. Smp. 126-127 °C.

## Beispiel 2

N-[2-nitro-5-(2-chlor-4-trifluormethyl-phenoxy)-phenyl]-4-amino-n-buttersäure-methylester (Verbindung Nr. 2)

8g N-[2-nitro-5-(2-chlor-4-trifluormethyl-phenoxy)-phenyl]-4-amino-n-buttersäure werden in 50 ml Methanol gelöst, langsam mit 10 ml konz. Schwefelsäure versetzt und einige Stunden stehengelassen. Anschliessend wird das Reaktionsgemisch auf Eis gegossen mit Natronlauge alkalisch gestellt und mit Toluol extrahiert. Der Toluolextrakt wird mit Wasser neutral gewaschen, am Vakuum weitgehend eingeengt und mit Hexan versetzt. Man erhält 6 g N-[2-nitro-5-(2-chlor-4-trifluormethylphenyl)-phenoxy]-4-amino-n-buttersäure-methylester in kristalliner Form. Smp. 60-62 °C.

## Beispiel 3

3-[N-{2-nitro-5-(2-chlor-4-trifluormethyl-phenoxy)-phenyl}-amino]-propionsäure-amid (Verbindung Nr. 33)

88 g N-[2-nitro-5-(2-chlor-4-trifluormethyl-phenoxy)-phenyl]-3-aminopropionsäure (Verbindung Nr. 3) werden in 500 ml Methylenchlorid mit 34,3 ml Triäthylamin bei − 25 °C vorgelegt. Man lässt unter Rühren während 30 Minuten 22,8 ml Chlorameisensäuremethylester zufliessen und rührt bei − 10 bis − 20 °C weiter. Nach zwei Stunden werden 40 ml konz. Ammoniaklösung zugegeben und das Reaktionsgemisch auf Raumtemperatur erwärmt. Anschliessend wird das Gemisch mit Wasser gewaschen und die organische Phase eingedampft. Der Rückstand wird aus Essigester/Hexan umkristallisiert. Smp. 137-138 °C.

Beispiel 4

N-Acetyl-N-[2-nitro-5-(2-chlor-4-trifluormethyl-phenoxy)-phenyl]-4-amino-n-buttersäure-methylester (Verbindung Nr. 54)

10 g N-[2-nitro-5-(2-chlor-4-trifluormethyl-phenoxy)-phenyl]-4-amino-n-buttersäure-methylester (Verbindung Nr. 2) werden in 30 ml Acetylchlorid am Rückfluss gekocht bis die Salzsäureentwicklung beendet ist. Das überschüssige Acetylchlorid wird am Vakuum entfernt. Man erhält die Titelverbindung in quantitativer Ausbeute. $n_D^{21}$ 1.543 7.

Auf analoge Weise können auch die folgenden, in der Tabelle 1 zusammen mit den Verbindungen aus den Beispielen 1 bis 4 aufgeführten Verbindungen der Formel I hergestellt werden :

Tabelle 1

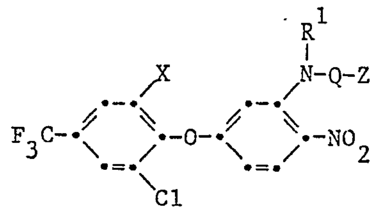

| Verbindung Nr. | X | $R^1$ | Q | Z | physikal. Daten |
|---|---|---|---|---|---|
| 1 | H | H | $-CH_2-CH_2-CH_2-$ | -COOH | Smp. 126-127°C |
| 2 | H | H | $-CH_2-CH_2-CH_2-$ | $-COOCH_3$ | Smp. 60-62°C |
| 3 | H | H | $-CH_2-CH_2-$ | -COOH | Smp. 105°C |
| 4 | H | H | $-CH(CH_2-OH)-$ | -COOH | Smp. 179-180°C |
| 5 | H | H | $-CH(CH_3)-CH_2-$ | -COOH | Smp. 143°C |
| 6 | H | H | $-CH_2-CH(CH_3)-$ | -COOH | Smp. 118-121°C |
| 7 | H | H | $-CH(CH_2-CH_2-S-CH_3)-$ | -COOH | Smp. 118°C |
| 8 | H | H | $-CH_2-CH_2-$ | $-COOCH_3$ | Sdp. 240°C/ 0.15 Torr |
| 9 | H | $-CH_3$ | $-CH_2-$ | -COOH | $n_D^{25}$ 1.5771 |
| 10 | H | $-CH_3$ | $-CH_2-$ | $-COOC_2H_5$ | $n_D^{33}$ 1.5751 |
| 11 | H | H | $-CH_2-CH_2-$ | $-COOisoC_3H_7$ | Smp. 93-95°C |
| 12 | H | H | $-CH_2-CH_2-CH_2-$ | $-COOisoC_3H_7$ | Sdp. 245°C/ 0.09 Torr |
| 13 | H | H | $-CH_2-CH_2-$ | $-COOC_2H_5$ | Smp. 93-94°C |
| 14 | H | H | $-CH(CH_3)-CH_2-$ | $-COOsek.C_4H_9$ | $n_D^{31}$ 1.5633 |

Tabelle 1 (Fortsetzung)

| Verbindung Nr. | X | $R^1$ | Q | Z | physikal. Daten |
|---|---|---|---|---|---|
| 15 | H | H | $CH_3$<br>$HC-OH$<br>$-CH-$ | $-COOCH_3$ | $n_D^{28}$ 1.5848 |
| 16 | H | H | $-CH_2-CH_2-$ | $-COOCH_2-CH_2-O-CH_3$ | $n_D^{24}$ 1.5635 |
| 17 | H | H | $-CH_2-CH_2-CH_2-$ | $-COOnC_4H_9$ | $n_D^{23}$ 1.5695 |
| 18 | H | H | $-CH_2-CH_2-$ | $-CN$ | $n_D^{23}$ 1.5905 |
| 19 | H | $-CH_2-CH_2-CH_2-CH<$ | | $-COOH$ | Smp. 80°C |
| 20 | H | H | $CH_3$<br>$HC-OH$<br>$-CH-$ | $-COOH$ | Smp. 89-90°C |
| 21 | H | H | $CH_2-CH_2-OH$<br>$-CH-$ | $-COOH$ | |
| 22 | H | H | $COOCH_3$<br>$-CH-$ | $-COOCH_3$ | |
| 23 | H | H | $-CH_2-CH_2-$ | $-COO^{\ominus}Na^{\oplus}$ | |
| 24 | Cl | H | $-CH_2-CH_2-$ | $-COOH$ | Smp. 151-153°C |
| 25 | H | H | $CH_3$<br>$-CH_2-CH-$ | $-COOC_2H_5$ | $n_D^{36}$ 1.5698 |
| 26 | H | $-CH_3$ | $-CH_2-$ | $-COOCH_3$ | $n_D^{28}$ 1.5725 |
| 27 | H | H | $-CH_2-CH_2-CH_2-$ | $-CONH-CH_3$ | Smp. 117-118°C |
| 28 | H | H | $-CH_2-CH_2-$ | $-CONH-C_2H_5$ | Smp. 168-170°C |
| 29 | H | H | $-CH_2-CH_2-CH_2-$ | $-CONH-C_3H_{7n}$ | Smp. 94-96°C |
| 30 | Cl | $-CH_3$ | $-CH_2-$ | $-COOH$ | Harz |

Tabelle 1 (Fortsetzung)

| Verbindung Nr. | X | $R^1$ | Q | Z | physikal. Daten |
|---|---|---|---|---|---|
| 31 | H | $-CH_3$ | $-CH_2-$ | $-CONH-CH_3$ | Smp. 103-105°C |
| 32 | H | H | $-CH_2-CH_2-$ | $-CON(CH_3)_2$ | Smp. 101-102°C |
| 33 | H | H | $-CH_2-CH_2-$ | $-CONH_2$ | Smp. 137-138°C |
| 34 | H | H | $-CH_2-CH_2-CH_2-$ | $-CONH-C_3H_7iso$ | Smp. 116-117°C |
| 35 | H | H | $-CH_2-CH_2-$ | $-CN$ | Smp. 126-127°C |
| 36 | H | H | 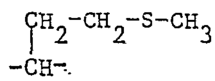 | $-COOCH_3$ | Oel |
| 37 | H | H | 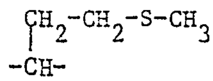 | $-CONH_2$ | Smp. 118-120°C |
| 38 | H | -NO | $-CH_2-CH_2-$ | $-COOH$ | Smp. 140°C |
| 39 | H | H | 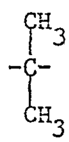 | $-COOH$ | Smp. 193°C |
| 40 | H | H | $-CH_2-CH_2-$ | $-COOCH_2CH_2Cl$ | Smp. 73-75°C |
| 41 | H | $-CH_3$ | $-CH_2-$ | $-COOCH_2CH_2Cl$ | $n_D^{25.5}$ 1.5701 |
| 42 | H | H | $-CH_2-CH_2-$ | $-CONH-NH_2$ | Smp. 139-141°C |
| 43 | H | -NO | $-CH_2-CH_2-$ | $-COOCH_3$ | $n_D^{25}$ 1.5665 |
| 44 | H | -NO | $-CH_2-CH_2-$ | $-CONH-CH_3$ | $n_D^{27}$ 1.5525 |
| 45 | H | H | $-CH_2-CH_2-$ | $-CON(CH_3)-OCH_3$ | Smp. 88-90°C |
| 46 | H | H | $-CH_2-CH_2-$ | $-CON(CH_3)-CH_2CH_2-O-CH_3$ | Smp. 104-106°C |
| 47 | H | H | $-CH_2-CH_2-$ | $-CON(CH_3)-CH(CH_3)-C{\equiv}CH$ | Harz |
| 48 | H | H | $-CH_2-CH_2-$ | $-COOCH_2-CH=CH_2$ | Smp. 58-60°C |

Tabelle 1 (Fortsetzung)

| Verbindung Nr. | X | $R_1$ | Q | Z | physikal. Daten |
|---|---|---|---|---|---|
| 49 | H | $-CO-CH_2Cl$ | $-CH_2-CH_2-$ | $-COOH$ | flüssig/fest |
| 50 | H | $-CO-CH_3$ | $-CH_2-CH_2-$ | $-COOH$ | $n_D^{24}$ 1.5465 |
| 51 | H | $-CO-CH_2Cl$ | $-CH_2-CH_2-CH_2-$ | $-COOCH_3$ | $n_D^{24}$ 1.5483 |
| 52 | H | H | $-CH=C(\underset{\underset{O}{\|}}{C}-OC_2H_5)-$ | $-CN$ | Smp. 184-185°C |
| 53 | H | $-CO-CF_3$ | $-CH_2-CH_2-CH_2-$ | $-COOCH_3$ | $n_D^{22}$ 1.5215 |
| 54 | H | $-CO-CH_3$ | $-CH_2-CH_2-CH_2-$ | $-COOCH_3$ | $n_D^{21}$ 1.5437 |
| 55 | H | H | $-CH_2-CH_2-CH_2-$ | $-CONH-OH$ | Smp. 139-141°C |
| 56 | H | $-CO-CCl_3$ | $-CH_2-CH_2-CH_2-$ | $-COOCH_3$ | Harz |
| 57 | H | H | $-CH_2-CH_2-CH_2-$ | $-CONH-NH_2$ | Smp. 96-99°C |
| 58 | H | H | $-CH_2-CH_2-CH_2-$ | 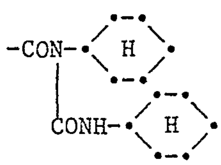 | Smp. 148-149°C |
| 59 | H | $-CO-C_2H_5$ | $-CH_2-CH_2-$ | $-COOCH_3$ | Oel |
| 60 | H | H | $-\overset{\overset{COOH}{\|}}{C}H-CH_2-$ | $-CONH_2$ | Smp. 152-154°C |
| 61 | H | H | $-(CH_2)_5-$ | $-COOH$ | Smp. 68-72°C |
| 62 | H | H | $-CH_2-CH_2-$ | $-COOCH_2-C\equiv CH$ | |
| 63 | H | $-CO-CH=CH_2$ | $-CH_2-CH_2-$ | $-COOC_2H_5$ | |

Formulierungsbeispiele für flüssige Wirkstoffe der Formel I (% = Gewichtsprozent)

| 5. Emulsions-Konzentrate | a) | b) | c) |
|---|---|---|---|
| Wirkstoff aus Tabelle 1 | 25% | 40% | 50% |
| Ca-Dodecylbenzolsulfonat | 5% | 8% | 6% |
| Ricinusöl-polyäthylenglykoläther (36 Mol AeO) | 5% | - | - |
| Tributylphenyl-polyäthylenglykoläther (30 Mol AeO) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| 6. Lösungen | a) | b) | c) | d) |
|---|---|---|---|---|
| Wirkstoff aus Tabelle 1 | 80% | 10% | 5% | 95% |
| Aethylenglykol-monomethyl-äther | 20% | - | - | - |
| Polyäthylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenze 160-190°C) | - | - | 94% | - |

Die Lösungen sind zur Anwendung in Form kleinster Tropfen geeignet.

| 7. Granulate | a) | b) |
|---|---|---|
| Wirkstoff aus Tabelle 1 | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| 8. Stäubemittel | a) | b) |
|---|---|---|
| Wirkstoff aus Tabelle 1 | 2% | 5% |
| Hochdisperse Kieselsäure | 1% | 5% |
| Talkum | 97% | - |
| Kaolin | - | 90% |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

| 9. Spritzpulver | a) | b) | c) |
|---|---|---|---|
| Wirkstoff aus Tabelle 1 | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyäthylenglykoläther (7-8 Mol AeO) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

10. Emulsions-Konzentrat

| | |
|---|---|
| Wirkstoff aus Tabelle 1 | 10% |
| Octylphenolpolyäthylenglykoläther (4-5 Mol AeO) | 3% |
| Ca-Dodecylbenzolsulfonat | 3% |
| Ricinusölpolyglykoläther (35 Mol AeO) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| 11. Stäubemittel | a) | b) |
|---|---|---|
| Wirkstoff aus Tabelle 1 | 5% | 8% |
| Talkum | 95% | - |
| Kaolin | - | 92% |

Man erhält anwendungsfertige Stäubemittel, indem der Wirkstoff mit den Trägerstoffen vermischt und auf einer geeigneten Mühle vermahlen wird.

12. Extruder Granulat

| | |
|---|---|
| Wirkstoff aus Tabelle 1 | 10% |
| Na-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrom getrocknet.

13. Umhüllungs-Granulat

| | |
|---|---|
| Wirkstoff aus Tabelle 1 | 3% |
| Polyäthylenglykol (M G 200) | 3% |
| Kaolin | 94% |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

14. Suspensions-Konzentrat

| | |
|---|---|
| Wirkstoff aus Tabelle 1 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37%-ige wässerige Formaldehyd-Lösung | 0,2 % |
| Silikonöl in Form einer 75%-igen wässerigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 15

Pre-emergente Herbizid-Wirkung (Keimhemmung)

Im Gewächshaus wird unmittelbar nach Einsaat der Samen von Versuchspflanzen in Saatschalen die

Erdoberfläche mit einer wässrigen Dispersion von Wirkstoffen der Formel I, erhalten aus 25%-igem Spritzpulver, behandelt. Es werden Konzentrationen von 4 kg Wirksubstanz pro Hektar angwendet. Die Saatschalen werden im Gewächshaus bei 22-25 °C und 50-70 °C relativer Luftfeuchtigkeit gehalten. Der Versuch wird nach 3 Wochen ausgewertet und die Resultate werden nach folgender Notenskala bonitiert :

1 = Pflanzen nicht gekeimt oder total abgestorben
2-3 = sehr starke Wirkung
4-6 = mittlere Wirkung
7-8 = geringe Wirkung
9 = keine Wirkung (wie unbehandelte Kontrolle)

Die Verbindungen der Formel I zeigen in diesem Test eine gute Wirkung. So wurden gegen die Unkräuter Sinapis, Setaria und Stellaria folgende Ergebnisse für die nachstehend aufgeführten Verbindungen erhalten :

Tabelle 2

| Verbindung Nr. | Sinapis | Setaria | Stellaria |
|---|---|---|---|
| 1 | 1 | 3 | 1 |
| 2 | 1 | 2 | 1 |
| 3 | 1 | 2 | 1 |
| 4 | 3 | 3 | 2 |
| 5 | 1 | 1 | 1 |
| 6 | 1 | 4 | 1 |
| 8 | 1 | 1 | 1 |
| 9 | 1 | 1 | 2 |
| 10 | 1 | 1 | 2 |
| 11 | 1 | 1 | 1 |
| 12 | 1 | 1 | 1 |
| 13 | 1 | 1 | 1 |
| 14 | 1 | 1 | 1 |
| 15 | 2 | 3 | 5 |
| 16 | 1 | 2 | 1 |
| 17 | 1 | 1 | 1 |
| 18 | 3 | 1 | 1 |
| 24 | 1 | 1 | 1 |
| 25 | 1 | 1 | 1 |
| 26 | 1 | 1 | 1 |
| 27 | 1 | 1 | 1 |
| 28 | 3 | 3 | 1 |
| 29 | 1 | 1 | 1 |
| 31 | 4 | 2 | 2 |
| 32 | 1 | 2 | 1 |
| 33 | 1 | 1 | 1 |
| 34 | 1 | 1 | 1 |
| 38 | 1 | 1 | 1 |
| 39 | 5 | 3 | 1 |
| 40 | 1 | 2 | 1 |

Tabelle 2 (Fortsetzung)

| Verbindung Nr. | Sinapis | Setaria | Stellaria |
|---|---|---|---|
| 41 | 2 | 1 | 2 |
| 42 | 4 | 5 | 1 |
| 43 | 1 | 1 | 1 |
| 44 | 2 | 2 | 2 |
| 45 | 1 | 1 | 1 |
| 46 | 2 | 2 | 2 |
| 48 | 1 | 4 | 1 |
| 49 | 3 | 4 | 3 |
| 50 | 1 | 3 | 2 |
| 54 | 1 | 4 | 1 |

Beispiel 16

Pre-emergente Herbizid-Wirkung (Keimhemmung)

Unter den im Beispiel 15 angegebenen Bedingungen wird ein Test mit einer grossen Anzahl von Kulturpflanzen und Unkräutern durchgeführt, wobei Konzentration von 4 kg und 2 kg Wirkstoff pro Hektar angewendet werden. Die Resultate werden nach der im Beispiel 15 angegebenen Notenskala bonitiert.

Die Verbindungen der Formel I zeigen in diesem Test eine gute selektivherbizide Wirkung. So wurden folgende Ergebnisse für die nachfolgend aufgeführten Verbindungen erhalten :

Tabelle 3

| Verbindung Nr. 3 | | |
|---|---|---|
| Testpflanze | Aufwandmenge | |
| | 4 kg/ha | 2 kg/ha |
| Gerste | 9 | 9 |
| Weizen | 9 | 9 |
| Mais | 9 | 9 |
| Sorghum hybr. | 8 | 9 |
| Reis trocken | 6 | 7 |
| Digitaria sang. | 4 | 7 |
| Echinochloa c.g. | 4 | 4 |
| Soja | 6 | 8 |
| Baumwolle | 5 | 7 |
| Abutilon | 1 | 1 |
| Sida spinosa | 1 | 2 |
| Amaranthus ret. | 1 | 1 |
| Chenopodium res. | 1 | 1 |
| Solanum nigrum | 1 | 1 |
| Ipomoea | 2 | 3 |
| Sinapis | 1 | 1 |
| Stellaria | 1 | 1 |
| Crysanthem. leuc. | 1 | 1 |
| Galium aparine | 1 | 2 |
| Viola tricolor | 1 | 1 |
| Veronica Sp. | 1 | 1 |

Tabelle 4

| Verbindung Nr. | 1 | 2 | 4 | 8 | 11 | 12 | 13 | 14 | 16 | 19 | 25 | 27 | 33 | 38 | 40 |
|---|---|---|---|---|---|---|---|---|---|---|---|---|---|---|---|
| kg AS/ha / Testpflanze | 4 2 | 4 2 | 4 2 | 4 2 | 4 2 | 4 2 | 4 2 | 4 2 | 4 2 | 4 2 | 4 2 | 4 2 | 4 2 | 4 2 | 4 2 |
| Weizen | 6 9 | 7 7 | 9 9 | 8 9 | 8 9 | 9 9 | 8 9 | 8 9 | 7 9 | 9 9 | 7 8 | 6 8 | 3 4 | 9 9 | 9 9 |
| Mais | - - | 8 9 | - - | 9 9 | 9 9 | 9 9 | 8 9 | 8 9 | 9 9 | 9 9 | 8 9 | 3 8 | 3 5 | 9 9 | 9 9 |
| Reis trocken | 7 8 | 6 7 | 6 8 | 7 7 | 8 9 | 9 9 | 3 6 | 8 9 | 7 9 | 8 9 | 4 5 | 7 9 | 6 7 | 6 6 | 7 9 |
| Echinochloa c.g. | 8 9 | 3 4 | 7 8 | 1 2 | 2 4 | 6 9 | 2 3 | 2 4 | 7 9 | 7 8 | 1 2 | 1 2 | 1 2 | 6 7 | 8 9 |
| Soja | 6 8 | 6 7 | 8 9 | 7 8 | 9 9 | 8 9 | 9 9 | 5 7 | 9 9 | 9 9 | 7 8 | 7 8 | 6 6 | 7 9 | 6 7 |
| Baumwolle | - - | 2 8 | - - | 2 2 | 4 6 | 3 4 | 2 3 | 2 2 | 9 9 | 4 6 | 1 2 | 2 4 | 3 4 | 8 9 | 4 8 |
| Abutilon | 1 1 | 1 2 | 1 1 | 1 1 | 1 2 | 1 4 | 1 1 | 1 1 | 1 2 | 1 3 | 1 1 | 1 1 | 1 1 | 1 2 | 1 1 |
| Chenopodium res. | 1 1 | 1 1 | 1 1 | 1 1 | 1 2 | 1 2 | 1 1 | 1 1 | 1 1 | 1 1 | 1 1 | 1 1 | 1 1 | 1 1 | 1 1 |
| Ipomoea | 4 7 | 1 2 | 1 4 | 1 1 | 1 2 | 1 4 | 1 1 | 2 2 | 4 7 | 3 4 | 1 2 | 2 7 | 2 2 | 3 3 | 2 6 |
| Sinapis | 1 1 | 1 1 | 1 2 | 1 1 | 1 1 | 1 1 | 1 1 | 1 1 | 1 1 | 1 1 | 1 1 | 1 1 | 1 2 | 1 1 | 1 1 |
| Galium aparine | 1 1 | 1 2 | 1 3 | 1 2 | 2 3 | 2 3 | 2 3 | 2 3 | 2 4 | 2 3 | 2 3 | 2 3 | 3 4 | 1 1 | 2 3 |
| Viola tricolor | 1 1 | 1 1 | 1 1 | 1 1 | 1 1 | 1 1 | 1 1 | 1 1 | 1 1 | 1 1 | 1 1 | 1 1 | 1 1 | 1 1 | 1 1 |

Beispiel 17

Post-emergente Herbizid-Wirkung (Kontaktherbizid)

Die zu testenden Unkräuter werden nach dem Auflaufen (im 4- bis 6-Blattstadium) mit einer wässrigen Dispersion von Wirkstoffen der Formel I, erhalten aus einem 25%-igen Spritzpulver, in einer Dosierung von 4 kg Wirkstoff pro Hektar auf die Pflanzen gespritzt und diese bei 24-26 °C und 45-60 °C relativer Luftfeuchtigkeit gehalten. Nach 15 Tagen wird der Versuch ausgewertet und das Ergebnis nach derselben Notenskala wie im pre-emergenten Versuch (Beispiel 15) bonitiert.

Die Verbindungen der Formel I zeigen in diesem Test eine gute Wirkung, wie die nachstehenden Resultate zeigen :

Tabelle 5

| Verbindung Nr. | Setaria | Solanum | Sinapis | Stellaria | Phaseolus |
|---|---|---|---|---|---|
| 1 | 4 | 1 | 1 | 2 | 1 |
| 2 | 2 | 1 | 1 | 1 | 1 |
| 3 | 3 | 1 | 1 | 2 | 1 |
| 4 | 5 | 1 | 3 | 6 | 3 |
| 5 | 4 | 1 | 1 | 1 | 1 |
| 6 | 5 | 1 | 1 | 1 | 2 |
| 7 | 5 | 1 | 1 | 3 | 4 |
| 8 | 1 | 1 | 1 | 1 | 1 |
| 9 | 2 | 1 | 2 | 1 | 2 |
| 10 | 2 | 1 | 1 | 1 | 1 |
| 11 | 6 | 1 | 1 | 1 | 3 |
| 12 | 4 | 1 | 1 | 1 | 2 |
| 13 | 5 | 1 | 1 | 2 | 3 |
| 14 | 2 | 1 | 1 | 2 | 3 |
| 15 | 3 | 1 | 1 | 2 | 2 |
| 16 | 3 | 1 | 1 | 1 | 1 |
| 17 | 3 | 1 | 1 | 1 | 1 |

(Siehe Fortsetzung Tabelle 5 Seite 21 f.)

Tabelle 5 (Fortsetzung)

| Verbindung Nr. | Setaria | Solanum | Sinapis | Stellaria | Phaseolus |
|---|---|---|---|---|---|
| 18 | 4 | 1 | 2 | 2 | 2 |
| 19 | 5 | 1 | 1 | 3 | 4 |
| 24 | 3 | 1 | 1 | 1 | 2 |
| 25 | 1 | 1 | 1 | 1 | 1 |
| 26 | 1 | 1 | 1 | 1 | 1 |
| 27 | 4 | 1 | 1 | 1 | 3 |
| 28 | 4 | 1 | 1 | 1 | 3 |
| 29 | 5 | 1 | 1 | 3 | 4 |
| 30 | 5 | 1 | 1 | 1 | 2 |
| 31 | 3 | 1 | 3 | 2 | 3 |
| 32 | 2 | 1 | 1 | 1 | 3 |
| 33 | 4 | 1 | 1 | 1 | 5 |
| 38 | 4 | 1 | 1 | 1 | 2 |
| 39 | 2 | 1 | 1 | 1 | 1 |
| 40 | 2 | 1 | 1 | 1 | 1 |
| 41 | 2 | 1 | 1 | 1 | 1 |
| 43 | 1 | 1 | 1 | 1 | 1 |
| 44 | 2 | 1 | 1 | 1 | 2 |
| 45 | 1 | 1 | 1 | 1 | 3 |
| 47 | 4 | 1 | 1 | 1 | 3 |
| 48 | 1 | 1 | 1 | 1 | 2 |
| 49 | 2 | 1 | 1 | 1 | 2 |
| 50 | 3 | 1 | 1 | 1 | 2 |
| 51 | 6 | 2 | 1 | 4 | 4 |
| 53 | 4 | 1 | 1 | 3 | 2 |
| 54 | 1 | 1 | 1 | 1 | 1 |

Beispiel 18

Post-emergente Herbizid-Wirkung (Kontaktherbizid)

Unter den im Beispiel 17 angegebenen Bedingungen wird ein Test mit einer grossen Anzahl von Kulturpflanzen und Unkräutern durchgeführt, wobei eine Konzentration von 0,5 kg Wirkstoff/ha angewendet wird. Die Resultate werden nach der im Beispiel 15 angegebenen Notenskala bonitiert.

Die Verbindungen der Formel I zeigen in diesem Test eine gute Wirkung, wie die nachfolgenden Ergebnisse zeigen :

Tabelle 6

| Verbindung Nr. / Testpflanzen | 3 | 9 | 11 | 12 | 13 | 27 | 31 | 32 | 33 | 38 | 39 | 44 | 45 | 47 | 49 |
|---|---|---|---|---|---|---|---|---|---|---|---|---|---|---|---|
| Weizen | 8 | 7 | 9 | 7 | 9 | 9 | 9 | 8 | 9 | 9 | 9 | 7 | 8 | 9 | 8 |
| Mais | - | 5 | 9 | 6 | 7 | 9 | 9 | 6 | 8 | 6 | 8 | 6 | 7 | 6 | 5 |
| Reis trocken | 9 | 8 | 9 | 6 | 8 | 9 | 9 | 8 | 9 | 9 | 9 | 9 | 9 | 9 | 9 |
| Soja | 6 | 6 | 8 | 6 | 6 | 7 | 8 | 4 | 8 | 6 | 9 | 4 | 6 | 5 | 7 |
| Abutilon | 1 | 1 | 1 | 1 | 1 | 1 | 2 | 1 | 1 | 2 | 7 | 1 | 1 | 2 | 1 |
| Xanthium | 4 | 4 | 1 | 1 | 1 | 6 | 4 | 1 | 4 | 1 | 4 | 1 | 1 | 1 | 1 |
| Chenopodium res. | 3 | 2 | 3 | 1 | 2 | 3 | 3 | 1 | 2 | 3 | 2 | 1 | 1 | 1 | 2 |
| Ipomoea | 4 | 3 | 1 | 1 | 1 | 5 | 6 | 1 | 3 | 2 | 2 | 1 | 1 | 2 | 1 |
| Sinapis | 1 | 2 | 2 | 1 | 1 | 4 | 5 | 1 | 3 | 1 | 3 | 1 | 1 | 1 | 1 |
| Galium aparine | 4 | 3 | 3 | 1 | 2 | 8 | 4 | 2 | 9 | 2 | 3 | 2 | 1 | 3 | 1 |
| Viola tricolor | 1 | 1 | 1 | 1 | 1 | 1 | 1 | 1 | 2 | 1 | 2 | 1 | 1 | 1 | 1 |

Verbindungen der Formel I zeigen auch bei geringerer Wirkstoffkonzentration gute selektiv-herbizide Wirkung, wie der nachstehende Vergleich von a) 0.5 kg AS/ha gemäss Tabelle 6 mit b) 0.25 kg AS/ha und c) 0.12 kg AS/ha für einige Substanzen zeigt :

Tabelle 7

| Verbindung Nr. | 12 | | | 32 | | | 44 | | | 45 | | | 49 | | |
|---|---|---|---|---|---|---|---|---|---|---|---|---|---|---|---|
| Konzentration Variante | a | b | c | a | b | c | a | b | c | a | b | c | a | b | c |
| Testpflanze: | | | | | | | | | | | | | | | |
| Weizen | 7 | 8 | 9 | 8 | 8 | 9 | 7 | 8 | 8 | 8 | 9 | 9 | 8 | 9 | 9 |
| Mais | 6 | 7 | 8 | 6 | 7 | 8 | 6 | 7 | 9 | 7 | 7 | 8 | 5 | 7 | 9 |
| Reis trocken | 6 | 7 | 8 | 8 | 9 | 9 | 9 | 9 | 9 | 9 | 9 | 9 | 9 | 9 | 9 |
| Soja | 6 | 6 | 8 | 4 | 7 | 7 | 4 | 7 | 9 | 6 | 6 | 7 | 7 | 7 | 8 |
| Abutilon | 1 | 1 | 2 | 1 | 1 | 1 | 1 | 1 | 1 | 1 | 2 | 2 | 1 | 1 | 1 |
| Xanthium | 1 | 1 | 1 | 1 | 2 | 2 | 1 | 1 | 1 | 1 | 1 | 2 | 1 | 1 | 1 |
| Chenopodium res. | 1 | 1 | 1 | 1 | 1 | 1 | 1 | 2 | 2 | 1 | 2 | 2 | 2 | 2 | 2 |
| Ipomoea | 1 | 1 | 1 | 1 | 1 | 2 | 1 | 1 | 3 | 1 | 1 | 2 | 1 | 1 | 1 |
| Sinapis | 1 | 1 | 1 | 1 | 1 | 1 | 1 | 1 | 1 | 1 | 1 | 2 | 1 | 1 | 1 |
| Galium aparine | 1 | 1 | 2 | 2 | 2 | 2 | 2 | 2 | 4 | 1 | 1 | 1 | 1 | 1 | 2 |
| Viola tricolor | 1 | 1 | 1 | 1 | 1 | 1 | 1 | 1 | 1 | 1 | 1 | 1 | 1 | 1 | 1 |

Beispiel 19

Desiccations- und Defoliationswirkung

In einem Gewächshaus werden Baumwollpflanzen der Sorte Delta-Pine in Tontöpfen mit einem Erde/Torf-Gemisch angezogen. Nach 12 Wochen (Blühbeginn) werden sie mit wässrigen Zubereitungen von Wirkstoffen der Formel I in einer Aufwandmenge, welche 150, 300 und 600 g/ha Wirkstoff entspricht, gespritzt (500 l/ha). Als Kontrolle dienen unbehandelte Pflanzen. Die Auswertung des Versuchs erfolgt 14 Tage nach Applikation der Wirksubstanz durch Bestimmen des Ausmasses der Defoliation (% abgefallene Blätter) und der Desiccation (% Austrocknung der an der Pflanze verbleibenden Blätter), wobei jeweils der %-Wert der Defoliation im %-Wert der Desiccation enthalten ist. Die Verbindungen der Formel I zeigen in diesem Test eine gute Wirkung, insbesondere die Verbindungen Nr. 2, 8 und 48, wie die nachfolgende Tabelle zeigt :

Tabelle 8

| | | Verbindung Nr. | | |
|---|---|---|---|---|
| | Aufwandmenge g/ha | 2 | 8 | 48 |
| Defoliation | 150 | 60 % | 60 % | 80 % |
| | 300 | 30 % | 70 % | 100 % |
| | 600 | 70 % | 50 % | 100 % |
| Desiccation | 150 | 80 % | 80 % | 100 % |
| | 300 | 90 % | 90 % | 100 % |
| | 600 | 90 % | 90 % | 100 % |

Die vorgenannten Verbindungen erweisen sich auch bei einem entsprechenden Versuch mit Kartoffelpflanzen als besonders aktiv.

**Patentansprüche**

1. Verbindungen der Formel I

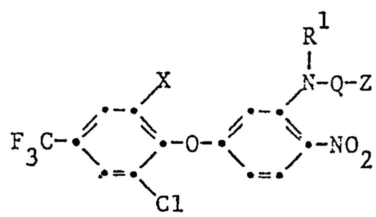

(I)

worin

X Wasserstoff, Chlor, Fluor oder Brom,

$R^1$ Wasserstoff, Nitroso oder $C_1$-$C_4$-Alkyl, $C_2$-$C_4$-Alkenyl, $C_1$-$C_4$-Alkylcarbonyl oder $C_2$-$C_4$-Alkenylcarbonyl, welche unsubstituiert oder durch eine oder mehrere Halogenatome substituiert sind,

Q einen geradkettigen oder verzweigten Alkylen- oder Alkenylenrest mit bis zu 5 Kohlenstoffatomen, in welchem neben der Hauptkette 0 bis 3 Seitenketten vorkiegen, wobei die Hauptkette gesättigt oder ungesättigt und unsubstituiert oder ein- oder zweifach durch Substituenten der Gruppe —$OR^2$, —$SR^3$ oder —$COOR^4$ substituiert ist, wobei keines der Kohlenstoffatome disubstituiert ist, und die Seitenketten ungesättigt und unsubstituiert oder eine oder zwei der Seitenketten durch —$OR^5$, —$SR^6$ oder —$COOR^7$ substituiert sind, wobei gleichzeitig mit der Hauptkette nur eine der Seitenketten substituiert sein kann, und $R^2$, $R^3$, $R^4$, $R^5$, $R^6$ und $R^7$ für Wasserstoff oder $C_1$-$C_4$-Alkyl stehen, und

Z—$COO^{\ominus}Me^{\oplus}$, —$COOR^8$, —$COSR^8$, —$CONR^9R^{10}$, —$COR^{11}$ oder —CN wobei $Me^{\oplus}$ für ein Kation, $R^8$ für Wasserstoff, $C_1$-$C_8$-Alkyl, welches unsubstituiert oder durch Halogen, Cyan oder $C_1$-$C_4$-Alkoxy substituiert ist, $C_2$-$C_8$-Alkenyl oder $C_3$-$C_8$-Alkinyl, $R^9$ für Wasserstoff, $C_1$-$C_4$-Alkyl oder $C_3$-$C_6$-Cycloalkyl,

$R^{10}$ für Wasserstoff, Amino, Hydroxy, $C_1$-$C_4$-Alkyl, welches unsubstituiert oder durch $C_1$-$C_4$-Alkoxy substituiert ist, $C_2$-$C_4$-Alkinyl, $C_1$-$C_4$-Alkoxy oder $C_3$-$C_6$-Cycloalkylaminocarbonyl und $R^{11}$ für Wasserstoff oder $C_1$-$C_4$-Alkyl, welches gegebenenfalls durch $C_1$-$C_4$-Alkoxy substituiert ist, stehen, oder

das Strukturelement —N($R^1$)—Q—Z einen Pyrrolidinoring, welcher in 2-Position durch Z und G' substituiert ist, worin G' für Wasserstoff oder $C_1$-$C_4$-Alkyl steht, bedeutet, mit der Massgabe, dass, wenn —N($R^1$)— und —Z unmittelbar nur über ein einziges Kohlenstoffatom miteinander verbunden sind und $R^1$ Wasserstoff bedeutet, Q für —CH($OR^2$), —CH($COOR^4$)—, —CH($C_1$—$C_4$—Alkyl, welches durch —$OR^5$, —$SR^6$ oder —$COOR^7$ substituiert ist)—, —C($C_1$—$C_4$—Alkyl)($OR^2$)—, —C($C_1$—$C_4$—Alkyl)($COOR^4$)—, —C($CH_3$)($C_1$—$C_3$-Alkyl, welches durch —$OR^5$, —$SR^6$ oder —$COOR^7$ substituiert ist)—, —C($C_2H_5$)($C_1$—$C_2$—Alkyl, welches durch —$OR^5$, —$SR^6$ oder —$COOR^7$ substituiert ist)—, —C($C_3H_7$)($CH_2OR^5$)—, —C($C_3H_7$)($CH_2SR^6$)—, —C($C_3H_7$)($CH_2COOR^7$)—, —C($CH_3$)($C_3H_7$)— oder —C($C_2H_5$)$_2$— steht.

2. Verbindungen nach Anspruch 1, dadurch gekennzeichnet, dass X und Q die im Anspruch 1 angegebenen Bedeutungen haben, $R^1$ Wasserstoff, Nitroso, $C_1$-$C_4$-Alkyl oder $C_1$-$C_4$-Alkylcarbonyl, welches unsubstituiert oder durch ein oder mehrere Halogenatome substituiert ist, und Z—$COO^{\ominus}Me^{\oplus}$, —$COOR^8$, —$CONR^9R^{10}$, oder —CN, wobei $Me^{\oplus}$ für ein Kation, $R^8$ für Wasserstoff, $C_1$-$C_4$-Alkyl, welches unsubstituiert oder durch Halogen oder $C_1$-$C_4$-Alkoxy substituiert ist, oder $C_2$-$C_4$-Alkenyl, $R^9$ für Wasserstoff, $C_1$-$C_4$-Alkyl oder $C_3$-$C_6$-Cycloalkyl und $R^{10}$ für Wasserstoff, Amino, Hydroxy, $C_1$-$C_4$-Alkyl, welches unsubstituiert oder durch $C_1$-$C_4$-Alkoxy substituiert ist, $C_2$-$C_4$-Alkinyl, $C_1$-$C_4$-Alkoxy oder $C_3$-$C_6$-Cycloalkylaminocarbonyl stehen, oder das Strukturelement —N($R^1$)—Q—Z einen Pyrrolidinoring, welcher in 2-Position durch Z und G' substituiert ist, worin G' für Wasserstoff oder $C_1$-$C_4$-Alkyl steht, bedeutet, mit der Massgabe, dass, wenn —N($R^1$)— und —Z unmittelbar nur über ein einziges Kohlenstoffatom miteinander verbunden sind und $R^1$ Wasserstoff bedeutet, Q für —CH($COOR^4$)—, —CH($C_1$—$C_4$—Alkyl, welches durch —$OR^5$, —$SR^6$ oder —$COOR^7$ substituiert ist)—, —C($C_1$—$C_4$—Alkyl)($COOR^4$)—, —C($CH_3$)($C_1$—$C_3$—Alkyl, welches durch —$OR^5$, —$SR^6$ oder —$COOR^7$ substituiert ist)—, —C($C_2H_5$)($C_1$—$C_2$—Alkyl, welches durch —$OR^5$, —$SR^6$ oder —$COOR^7$ substituiert ist)—, —C($C_3H_7$)($CH_2OR^5$)—, —C($C_3H_7$)($CH_2SR^6$)—, —C($C_3H_7$)($CH_2COOR^7$)—, —C($CH_3$)($C_3H_7$)— oder —C($C_2H_5$)$_2$ steht.

3. Verbindungen nach Anspruch 1, dadurch gekennzeichnet, dass X die im Anspruch 1 angegebene Bedeutung hat, $R^1$ Wasserstoff oder $C_1$-$C_4$-Alkyl, Q einen geradkettigen oder verzweigten Alkylenrest mit bis zu 5 Kohlenstoffatomen, in welchem neben der Hauptkette, welche die Strukturelemente —N($R^1$)— und —Z unmittelbar miteinander verbindet, 0 bis 2 Seitenketten vorliegen, wobei die Hauptkette unsubstituiert oder ein- oder zweifach durch —$OR^2$, —$SR^3$ oder —$COOR^4$ substituiert ist, wobei keines der Kohlenstoffatome disubstituiert ist und jedes Kohlenstoffatom mindestens ein Wasserstoffatom trägt, und die Seitenketten unsubstituiert oder durch —$OR^5$, —$SR^6$ oder —$COOR^7$ substituiert sind, wobei gleichzeitg mit der Hauptkette nur eine der Seitenketten substituiert sein kann und $R^2$, $R^3$, $R^4$, $R^5$, $R^6$ und $R^7$ für Wasserstoff oder $C_1$-$C_4$-Alkyl stehen, und Z—$COO^{\ominus}Me^{\oplus}$, —$COOR^8$, —$CONR^9R^{10}$, —$COR^{11}$ oder —CN, wobei $Me^{\oplus}$ für ein Metallkation, $R^8$ für Wasserstoff oder $C_1$-$C_4$-Alkyl, $R^9$ und $R^{10}$ für Wasserstoff oder $C_1$-$C_4$-Alkyl und $R^{11}$ für durch $C_1$-$C_4$-Alkoxy substituiertes $C_1$-$C_4$-Alkyl stehen, oder das Strukturelement —N($R^1$)—Q—Z einen Pyrrolidinoring, in welchem Z in 2-Position steht, bedeutet, mit der Massgabe, dass, wenn —N($R^1$)— und —Z unmittelbar nur über ein einziges Kohlenstoffatom miteinander verbunden sind und $R^1$ Wasserstoff bedeutet, Q für —CH($OR^2$)—, —CH($COOR^4$)— oder —CH($C_1$—$C_4$—Alkyl, welches durch —$OR^5$, —$SR^6$ oder —$COOR^7$ substituiert ist)— steht.

4. Verbindungen nach Anspruch 1, dadurch gekennzeichnet, dass X die im Anspruch 1 angegebene Bedeutung hat, $R^1$ Wasserstoff oder $C_1$-$C_4$-Alkyl, Q einen Alkylenrest mit bis zu 5 Kohlenstoffatomen, welcher

a) nur aus der Hauptkette, welche die Strukturelemente —$NR^1$— und —Z unmittelbar miteinander verbindet und aus 1 bis 3 Kohlenstoffatomen besteht und unsubstituiert oder durch —$CR^2$, —$SR^3$ oder —$COOR^4$ monosubstituiert ist, oder

b) aus der Hauptkette und einer Seitenkette besteht, wobei die Hauptkette aus 1 bis 3 Kohlenstoffatomen besteht und unsubstituiert ist, und die Seitenkette unsubstituiert oder durch —$CR^5$, —$SR^6$ oder —$COOR^7$ substituiert ist, und $R^2$, $R^3$, $R^4$, $R^5$, $R^6$ und $R^7$ für Wasserstoff oder $C_1$-$C_4$-Alkyl stehen, und Z —$COO^{\ominus}Me^{\oplus}$, —$COOR^8$, —$COR^{11}$ oder —CN, wobei $Me^{\oplus}$ für ein Metallkation, $R^8$ für Wasserstoff oder $C_1$-$C_4$-Alkyl und $R^{11}$ für durch $C_1$-$C_4$-Alkoxy substituiertes $C_1$-$C_4$-Alkyl steht, oder das Strukturelement —N($R^1$)—Q—Z einen Pyrrolidinoring bedeutet, in welchem Z in 2-Position steht, bedeutet, mit der Massgabe, dass, wenn —N($R^1$)— und —Z unmittelbar nur über ein einziges Kohlenstoffatom miteinander verbunden sind und $R^1$ Wasserstoff bedeutet, Q für —CH($OR^2$)—, —CH($COOR^4$)— oder —CH($C_1$—$C_4$—Alkyl, welches durch —$OR^5$, —$SR^6$ oder —$COOR^7$ substituiert ist)— steht.

5. Verbindungen nach Anspruch 1, dadurch gekennzeichnet, dass X Wasserstoff oder Chlor, $R^1$ Wasserstoff, Nitroso, Methyl, Methylcarbonyl, Aethylcarbonyl, Vinylcarbonyl, Chlormethylcarbonyl, Trichlormethylcarbonyl oder Trifluormethylcarbonyl, Q einen geradkettigen oder verzweigten Alkylen- oder Alkenylenrest mit bis zu 5 Kohlenstoffatomen, in welchem neben der Hauptkette, welche die Strukturelemente —N($R^1$)— und —Z unmittelbar miteinander verbindet, 0 bis 2 Seitenketten vorliegen, wobei die Hauptkette gesättigt oder ungesättigt und unsubstituiert oder durch —$OR^2$, —$SR^3$ oder —$COOR^4$ monobusbtituiert ist, und die Seitenketten gesättigt und unsubstituiert oder eine der Seitenketten durch

—$OR^5$, —$SR^6$ oder —$COOR^7$ substituiert ist, wobei nicht gleichzeitig die Hauptkette und die Seitenketten substituiert sind, und $R^2$, $R^3$, $R^4$, $R^5$, $R^6$ und $R^7$ für Wasserstoff oder $C_1$-$C_4$-Alkyl stehen, und Z —$COO^{\ominus}Me^{\oplus}$, —$COOR^8$, —$CONR^9R^{10}$ oder —CN, wobei $Me^{\oplus}$ für ein Kation, $R^8$ für Wasserstoff, $C_1$—$C_8$-Alkyl, welches unsubstituiert oder durch Halogen oder $C_1$-$C_4$-Alkoxy substituiert ist, $C_2$-$C_8$-Alkenyl oder $C_3$-$C_8$-Alkinyl, $R^9$ für Wasserstoff, $C_1$-$C_4$-Alkyl oder $C_3$-$C_6$-Cycloalkyl und $R^{10}$ für Wasserstoff, Amino, Hydroxy, $C_1$-$C_4$-Alkyl, welches unsubstituiert oder durch $C_1$-$C_4$-Alkoxy substituiert ist, $C_2$-$C_4$-Alkinyl, $C_1$-$C_4$-Alkoxy oder $C_3$-$C_6$-Cycloalkylaminocarbonyl stehen, oder das Strukturelement —$N(R^1)$—Q—Z einen Pyrrolidinoring, welcher in 2-Position durch Z und G' substituiert ist, worin G' für Wasserstoff oder $C_1$-$C_4$-Alkyl steht, bedeutet, mit der Massgabe, dass, wenn —$N(R^1)$— und —Z unmittelbar nur über ein einziges Kohlenstoffatom miteinander verbunden sind und $R^1$ Wasserstoff bedeutet, Q für —$CH(COOR^4)$—, —$CH(C_1$—$C_4$—Alkyl, welches durch —$OR^5$, —$SR^6$ oder —$COOR^7$ substituiert ist)—, —$C(C_1$—$C_4$—Alkyl)($COOR^4$)—, —$C(CH_3)(C_1$—$C_3$—Alkyl, welches durch —$OR^5$, —$SR^6$ oder —$COOR^7$ substituiert ist)—, —$C(C_2H_5)(C_1$—$C_2$—Alkyl, welches durch —$OR^5$, —$SR^6$ oder —$COOR^7$ substituiert ist)—, —$C(C_3H_7)(CH_2OR^5)$—, —$C(C_3H_7)(CH_2SR^6)$—, —$C(C_3H_7)(CH_2COOR^7)$—, —$C(CH_3)(C_3H_7)$— oder —$C(C_2H_5)_2$— steht.

6. Verbindungen nach Anspruch 1, dadurch gekennzeichnet, dass X Wasserstoff oder Chlor, $R^1$ Wasserstoff oder Methyl, Q einen Alkylenrest mit bis zu 5 Kohlenstoffatomen, welcher

a) nur aus der Hauptkette, welche die Strukturelemente —$N(R^1)$— und —Z unmittelbar miteinander verbindet und aus 1 bis 3 Kohlenstoffatomen besteht und unsubstituiert oder durch —$OR^2$, —$SR^3$ oder —$COOR^4$ monosubstituiert ist, oder

b) aus der Hauptkette und einer Seitenkette besteht, wobei die Hauptkette 1 bis 3 Kohlenstoffatome enthält und unsubstituiert ist, und die Seitenkette unsubstituiert oder durch —$OR^5$, —$SR^6$ oder —$COOR^7$ substituiert ist, und $R^2$, $R^3$, $R^4$, $R^5$, $R^6$ und $R^7$ für Wasserstoff oder $C_1$—$C_4$—Alkyl stehen, und Z —$COO^{\ominus}Me^{\oplus}$, —$COOR^8$, —$COR^{11}$ oder —CN, wobei $Me^{\oplus}$ für ein Metallkation, $R^8$ für Wasserstoff oder $C_1$—$C_4$—Alkyl und $R^{11}$ für durch $C_1$—$C_4$—Alkoxy substituiertes $C_1$—$C_4$—Alkyl steht, oder das Strukturelement —$N(R^1)$—Q—Z einen Pyrrolidinoring, in welchem Z in 2-Position steht, bedeutet, mit der Massgabe, dass, wenn —$N(R^1)$— und —Z unmittelbar nur über ein einziges Kohlenstoffatom miteinander verbunden sind und $R^1$ Wasserstoff bedeutet, Q für —$CH(OR^2)$—, —$CH(COOR^4)$— oder —$CH(C_1$—$C_4$—Alkyl, welches durch —$OR^5$, —$SR^6$ oder —$COOR^7$ substituiert ist)- steht.

7. Verbindungen nach Anspruch 1, dadurch gekennzeichnet, dass X Wasserstoff oder Chlor, $R^1$ Wasserstoff, Nitroso oder $C_1$—$C_4$—Alkyl, $C_2$—$C_4$—Alkenyl, $C_1$—$C_4$—Alkylcarbonyl oder $C_2$—$C_4$—Alkenylcarbonyl, welche unsubstituiert oder durch 1 bis 3 Chlor- oder Fluoratome substituiert sind, Q 1,5-Pentamethylen oder einen Alkylen- oder Alkenylenrest mit insgesamt bis zu 3 Kohlenstoffatomen, welcher

a) nur aus der Hauptkette, welche die Strukturelemente —$N(R^1)$— und —Z unmittelbar miteinander verbindet und gesättigt oder ungesättigt und unsubstituiert oder durch Carboxyl oder $C_1$—$C_4$—Alkoxycarbonyl monosubstituiert ist, besteht, oder

b) aus einer Hauptkette, welche aus 2 Kohlenstoffatomen besteht und gesättigt oder ungesättigt ist, und einer Seitenkette mit einem Kohlenstoffatom besteht, wobei i) die Hauptkette unsubstituiert oder durch Carboxyl oder $C_1$—$C_4$—Alkoxycarbonyl monosubstituiert ist und die Seitenkette aus einer unsubstituierten Methylgruppe besteht, oder ii) die Hauptkette unsubstituiert ist und die Seitenkette aus einer Methylgruppe, welche durch Hydroxy oder $C_1$—$C_4$—Alkylthio substituiert ist, besteht, oder

c) —CH(COOH)—, —$CH(COOC_1$—$C_4$—Alkyl)—, —$C(C_1$—$C_2$—Alkyl)(COOH)—, —$C(C_1$—$C_2$—Alkyl)($COOC_1$—$C_4$—Alkyl)—, —$CH(CH_2OH)$—, —$CH(CH_2$—S—$C_1$—$C_4$—Alkyl)—, —$CH(C_2H_5OH)$—, —$CH(C_2H_5$—S—$C_1$—$C_4$—Alkyl)—, —$C(CH_3)(CH_2OH)$—, —$C(CH_3)(CH_2$—S—$C_1$—$C_4$—Alkyl)—, —$C(CH_3)_2$ oder —$CH(C_1$—$C_2$—Alkyl) und Z —$COO^{\ominus}Me^{\oplus}$, —$COOR^8$, —$CONR^9R^{10}$ oder —CN, wobei $Me^{\oplus}$ für ein Alkalimetallkation, $R^8$ für Wasserstoff, $C_1$—$C_8$—Alkyl, welches unsubstituiert oder durch Chlor oder $C_1$—$C_4$—Alkoxy monosubstituiert ist, $C_2$—$C_8$—Alkenyl oder $C_3$—$C_8$—Alkinyl, $R^9$ für Wasserstoff, $C_1$—$C_4$—Alkyl oder $C_3$—$C_6$—Cycloalkyl und $R^{10}$ für Wasserstoff, Amino, Hydroxy, $C_1$—$C_4$—Alkyl, welches unsubstituiert oder durch $C_1$—$C_4$—Alkoxy monosubstituiert ist, $C_2$—$C_4$—Alkenyl, $C_1$—$C_4$—Alkoxy oder $C_3$—$C_6$—Cycloalkylaminocarbonyl stehen, oder das Strukturelement —$N(R^1)$—Q—Z einen Pyrrolidinoring, welcher in 2-Position durch Z und G' substituiert ist, worin G' für Wasserstoff oder $C_1$—$C_4$—Alkyl steht, bedeuten, mit der Massgabe, dass, wenn —$N(R^1)$ und —Z unmittelbar nur über ein einziges Kohlenstoffatom miteinander verbunden sind und $R^1$ Wasserstoff bedeutet, Q für —CH(COOH)—, —$CH(COOC_1$—$C_4$—Alkyl)—, —$C(C_1$—$C_2$—Alkyl)(COOH)—, —$C(C_1$—$C_2$—Alkyl)($COOC_1$—$C_4$—Alkyl)—, —$CH(CH_2OH)$—, —$CH(CH_2$—S—$C_1$—$C_4$—Alkyl)—, —$CH(C_2H_5OH)$—, —$CH(C_2H_5$—S—$C_1$—$C_4$—Alkyl)—, —$C(CH_3)(CH_2OH)$—, —$C(CH_3)(CH_2$—S—$C_1$—$C_4$—Alkyl)— oder —$C(CH_3)_2$ steht.

8. Verbindungen nach Anspruch 1, in denen X Wasserstoff, $R^1$ Wasserstoff, Q eine 1,2-Aethylenbrücke und Z —$COO^{\ominus}Me^{\oplus}$, —$COOR^8$, —$COSR^8$, —$CONR^9R^{10}$, —$COR^{11}$ oder —CN, wobei $Me^{\oplus}$ für ein Kation, R für Wasserstoff, $C_1$—$C_8$—Alkyl, welches unsubstituiert oder durch Halogen, Cyan oder $C_1$—$C_4$—Alkoxy substituiert ist, $C_2$—$C_8$—Alkenyl oder $C_3$—$C_8$—Alkinyl, $R^9$ für Wasserstoff, $C_1$—$C_4$—Alkyl oder $C_3$—$C_6$—Cycloalkyl, $R^{10}$ für Wasserstoff, Amino, Hydroxy, $C_1$—$C_4$—Alkyl, welches unsubstituiert oder durch $C_1$—$C_4$—Alkoxy substituiert ist, $C_2$—$C_4$—Alkinyl, $C_1$—$C_4$—Alkoxy oder $C_3$—$C_6$—Cycloalkylaminocarbonyl und $R^{11}$ für Wasserstoff oder $C_1$—$C_4$—Alkyl, welches gegebenenfalls durch $C_1$—$C_4$—Alkoxy substituiert ist, stehen, bedeuten.

9. Verbindungen nach Anspruch 1, dadurch gekennzeichnet, dass X und $R^1$ die im Anspruch 1 angegebenen Bedeutungen haben, Q 1,5-Pentamethylen oder einen Alkylen- oder Alkenylenrest mit insgesamt bis zu 3 Kohlenstoffatomen, welcher

a) nur aus der Hauptkette, welche die Strukturelemente $-N(R^1)-$ und $-Z$ unmittelbar miteinander verbindet und gesättigt oder ungesättigt und unsubstituiert oder durch Carboxyl, Methoxycarbonyl oder Aethoxycarbonyl monosubstituiert ist, besteht, oder

b) aus einer Hauptkette, welche aus 2 Kohlenstoffatomen besteht und gesättigt oder ungesättigt ist, und einer Seitenkette mit einem Kohlenstoffatom besteht, wobei i) die Hauptkette unsubstituiert oder durch Carboxyl, Methoxycarbonyl oder Aethoxycarbonyl monosubstituiert ist und die Seitenkette aus einer unsubstituierten methylgruppe besteht, oder ii) die Hauptkette unsubstituiert ist und die Seitenkette Hydroxymethyl ist, besteht, oder

c) aus einem Mitglied der Gruppe $-CH(COOH)-$, $-CH(COOCH_3)-$, $-CH(COOC_2H_5)-$, $-CH(CH_2OH)-$, $-CH(CHOH-CH_3)-$, $-CH(CH_2CH_2OH)-$, $-CH(CH_2CH_2-S-CH_3)-$, $-C(CH_3)(COOH)-$, $-C(CH_3)(COOCH_3)-$, $-C(CH_3)(COOC_2H_5)-$, $-C(CH_3)(CH_2OH)-$, $-CH(C_1-C_2-Alkyl)-$ oder $-C(CH_3)_2$ besteht, und Z $-COO^{\ominus}Me^{\oplus}$, $-COOR^8$, $-CONR^9R^{10}$ oder $-CN$, wobei $Me^{\oplus}$ für ein Natriumkation, $R^8$ für Wasserstoff, $C_1-C_4$-Alkyl, 2-Chloräthyl, 2-Methoxyäthyl, Allyl oder 2-Propinyl, $R^9$ für Wasserstoff, Methyl oder Cyclohexyl und $R^{10}$ für Wasserstoff, Amino, Hydroxy, $C_1-C_3$-Alkyl, 2-Methoxyäthyl, 3-Butin-2-yl, Methoxy oder Cyclohexylaminocarbonyl stehen, oder das Strukturelement $-N(R^1)-Q-Z$ einen Pyrrolidinoring, welcher in 2-Position durch Z und G' substituiert ist, worin G' für Wasserstoff oder methyl steht, bedeutet, mit der Massgabe, dass, wenn $-N(R^1)-$ und $-Z$ unmittelbar nur über ein einziges Kohlenstoffatom miteinander verbunden sind und $R^1$ Wasserstoff bedeutet, Q für $-C(CH_3)_2-$, $-CH(COOH)-$, $-CH(COOCH_3)-$, $-CH(COOC_2H_5)-$, $-CH(CH_2OH)-$, $-CH(CHOH-CH_3)-$, $-CH(CH_2CH_2OH)-$, $-CH(CH_2CH_2-S-CH_3)-$, $-C(CH_3)(COOH)-$, $-C(CH_3)(COOCH_3)-$, $-C(CH_3)(COOC_2H_5)-$ oder $-C(CH_3)(CH_2OH)-$ steht.

10. Verbindungen nach Anspruch 1, dadurch gekennzeichnet, dass X Wasserstoff oder chlor, $R^1$ Wasserstoff, Nitroso, $C_1-C_4$-Alkyl, $C_1-C_4$-Alkylcarbonyl, welches unsubstituiert oder durch 1 bis 3 Chlor- oder Fluoratome substituiert ist, oder $C_2-C_4$-Alkenylcarbonyl, Q 1,5-Pentamethylen oder einen Alkylen- oder Alkenylenrest mit insgesamt bis zu 3 Kohlenstoffatomen, welcher

a) nur aus der Hauptkette, welche die Strukturelemente $-N(R^1)-$ und $-Z$ unmittelbar miteinander verbindet und gesättigt oder ungesättigt und unsubstituiert oder durch Carboxyl oder $C_1-C_4$-Alkoxycarbonyl monosubstituiert ist, besteht, oder

b) aus einer Hauptkette, welche aus 2 Kohlenstoffatomen besteht und gesättigt oder ungesättigt ist, und einer Seitenkette mit einem Kohlenstoffatom besteht, wobei i) die Hauptkette unsubstituiert oder durch Carboxyl oder $C_1-C_4$-Alkoxycarbonyl monosubstituiert ist und die Seitenkette aus einer unsubstituierten Methylgruppe besteht, oder ii) die Hauptkette unsubstituiert ist und die Seitenkette aus einer Methylgruppe, welche durch Hydroxy oder $C_1-C_4$-Alkylthio substituiert ist, besteht, oder

c) $-CH(COOH)-$, $-CH(COOC_1-C_4-Alkyl)-$, $-C(C_1-C_2-Alkyl)(COOH)-$, $-C(C_1-C_2-Alkyl)(COOC_1-C_4-Alkyl)-$, $-CH(CH_2OH)-$, $-CH(CH_2-S-C_1-C_4-Alkyl)-$, $-CH(C_2H_5OH)-$, $-CH(C_2H_5S-C_1-C_4-Alkyl)-$, $-C(CH_3)(CH_2OH)-$, $-C(CH_3)(CH_2-S-C_1-C_4-Alkyl)-$, $-C(CH_3)_2$ oder $-CH(C_1-C_2-Alkyl)$ und Z $-COO^{\ominus}Me^{\oplus}$, $-COOR^8$, $-CONR^9R^{10}$ oder $-CN$, wobei $Me^{\oplus}$ für ein Alkalimetallkation, $R^8$ für Wasserstoff, $C_1-C_4$-Alkyl, welches unsubstituiert oder durch Chlor oder $C_1-C_4$-Alkoxy monosubstituiert ist, oder $C_2-C_4$-Alkenyl, $R^9$ für Wasserstoff, $C_1-C_4$-Alkyl oder $C_3-C_6$-Cycloalkyl und $R^{10}$ für Wasserstoff, Amino, Hydroxy, $C_1-C_4$-Alkyl, welches unsubstituiert oder durch $C_1-C_4$-Alkoxy monosubstituiert ist, $C_2-C_4$-Alkinyl, $C_1-C_4$-Alkoxy oder $C_3-C_6$-Cycloalkylaminocarbonyl stehen, oder das Strukturelement $-N(R^1)-Q-Z$ einen Pyrrolidinoring, welcher in 2-Position durch Z und G' substituiert ist, worin G' für Wasserstoff oder $C_1-C_4$-Alkyl steht, bedeutet, mit der Massgabe, dass, wenn $-N(R^1)-$ und $-Z$ unmittelbar nur über ein einziges Kohlenstoffatom miteinander verbunden sind und $R^1$ Wasserstoff bedeutet, Q für $-CH(COOH)-$, $-CH(COOC_1-C_4-Alkyl)-$, $-C(C_1-C_2-Alkyl)(COOH)-$, $-C(C_1-C_2-Alkyl)(COOC_1-C_4-Alkyl)-$, $-CH(CH_2OH)-$, $-CH(CH_2-S-C_1-C_4-Alkyl)-$, $-CH(C_2H_5OH)-$, $-CH(C_2H_5-S-C_1-C_4-Alkyl)-$, $-C(CH_3)(CH_2OH)-$, $-C(CH_3)(CH_2-S-C_1-C_4-Alkyl)-$ oder $-C(CH_3)_2$ steht.

11. Verbindungen nach Anspruch 1, dadurch gekennzeichnet, dass X Wasserstoff oder Chlor, $R^1$ Wasserstoff, Nitroso, Methyl, Methylcarbonyl, Aethylcarbonyl, Vinylcarbonyl, Chlormethylcarbonyl, Trichlormethylcarbonyl oder Trifluormethylcarbonyl, Q 1,5-Pentamethylen oder einen Alkylen- oder Alkenylenrest mit insgesamt bis zu 3 Kohlenstoffatomen, welcher

a) nur aus der hauptkette, welche die Strukturelemente $-N(R^1)-$ und $-Z$ unmittelbar miteinander verbindet und gesättigt oder ungesättigt und unsubstituiert oder durch Carboxyl, Methoxycarbonyl oder Aethoxycarbonyl monosubstituiert ist, besteht, oder

b) aus einer Hauptkette, welche aus 2 Kohlenstoffatomen besteht und gesättigt oder ungesättigt ist, und einer Seitenkette mit einem Kohlenstoffatom besteht, wobei i) die Hauptkette unsubstituiert oder durch Carboxyl, Methoxycarbonyl oder Aethoxycarbonyl monosubstituiert ist und die Seitenkette aus einer unsubstituierten Methylgruppe besteht, oder ii) die Hauptkette unsubstituiert ist und die Seitenkette Hydroxymethyl ist, oder

c) aus einem Mitglied der Gruppe $-CH(COOH)-$, $-CH(COOCH_3)-$, $-CH(COOC_2H_5)-$,

$-CH(CH_2OH)-$, $-CH(CHOH-CH_3)-$, $-CH(CH_2CH_2OH)-$, $-CH(CH_2CH_2-S-CH_3)-$, $-C(CH_3)(COOH)-$, $-C(CH_3)(COOCH_3)$, $-C(CH_3)(COOC_2H_5)-$, $-C(CH_3)(CH_2OH)-$ oder $-C(CH_3)_2-$ besteht, und Z $-COO^{\ominus}Me^{\oplus}$, $-COOR^8$, $-CONR^9R^{10}$ oder $-CN$, wobei $Me^{\oplus}$ für ein Natriumkation, $R^8$ für Wasserstoff, $C_1-C_4$-Alkyl, 2-Chloräthyl, 2-Methoxyäthyl, Allyl oder 2-Propinyl, $R^9$ für Wasserstoff, Methyl oder Cyclohexyl und $R^{10}$ für Wasserstoff, Amino, Hydroxy, $C_1-C_3$-Alkyl, 2-Methoxyäthyl, 3-Butin-2-yl, Methoxy oder Cyclohexylaminocarbonyl stehen, oder das Strukturelement $-N(R^1)-Q-Z$ einen Pyrrolidinoring, welcher in 2-Position durch Z und G' substituiert ist, worin G' für Wasserstoff oder Methyl steht, bedeutet, mit der Massgabe, dass, wenn $-N(R^1)-$ und $-Z$ unmittelbar nur über ein einziges Kohlenstoffatom miteinander verbunden sind und $R^1$ Wasserstoff bedeutet, Q für $-C(CH_3)_2-$, $-CH(COOH)-$, $-CH(COOCH_3)-$, $-CH(COOC_2H_5)-$, $-CH(CH_2OH)-$, $-CH(CHOH-CH_3)-$, $-CH(CH_2CH_2OH)-$, $-CH(CH_2CH_2-S-CH_3)-$, $-C(CH_3)(COOH)-$, $-C(CH_3)(COOCH_3)-$, $-C(CH_3)(COOC_2H_5)-$ oder $-C(CH_3)(CH_2OH)-$ steht.

12. Verbindungen nach Anspruch 1, dadurch gekennzeichnet, dass X Wasserstoff oder Chlor, $R^1$ Wasserstoff, Nitroso, Methyl, Methylcarbonyl, Aethylcarbonyl, Chlormethylcarbonyl, Trichlormethylcarbonyl oder Trifluormethylcarbonyl, Q 1,5-Pentamethylen oder einen Alkylen- oder Alkenylenrest mit bis zu 3 Kohlenstoffatomen, welcher

a) nur aus der Hauptkette, welche die Strukturelemente $-N(R^1)-$ und $-Z$ unmittelbar miteinander verbindet und gesättigt oder ungesättigt und unsubstituiert oder durch Carboxyl oder Aethoxycarbonyl monosubstituiert ist, besteht, oder

b) aus einer Hauptkette, welche aus 2 Kohlenstoffatomen besteht und gesättigt oder ungesättigt ist, und einer Seitenkette mit einem Kohlenstoffatom besteht, wobei i) die Hauptkette unsubstituiert oder durch Carboxyl oder Aethoxycarbonyl monosubstituiert ist und die Seitenkette aus einer unsubstituierten Methylgruppe besteht, oder ii) die Hauptkette unsubstituiert und die Seitenkette Hydroxymethyl ist, oder

c) aus einem Mitglied der Gruppe $-CH(COOH)-$, $-CH(COOC_2H_5)-$, $-CH(CH_2OH)-$, $-CH(CHOH-CH_3)-$, $-CH(CH_2CH_2-S-CH_3)-$, $-C(CH_3)(COOH)-$, $-CH(CH_3)(COOC_2H_5)-$, $-C(CH_3)(CH_2OH)-$ oder $-C(CH_3)_2$ besteht, und Z $-COOR^8$, $-CONR^9R^{10}$ oder $-CN$, wobei $R^8$ für Wasserstoff, $C_1-C_4$-Alkyl, 2-Chloräthyl, 2-Methoxyäthyl oder Allyl, $R^9$ für Wasserstoff, Methyl oder Cyclohexyl und $R^{10}$ für Wasserstoff, Amino, Hydroxy, $C_1-C_3$-Alkyl, 2-Methoxyäthyl, 3-Butin-2-yl, Methoxy oder Cyclohexylaminocarbonyl stehen, oder das Strukturelement $-N(R^1)-Q-Z$ einen Pyrrolidinoring, welcher in 2-Position durch Z und G' substituiert ist, worin G' für Wasserstoff oder Methyl steht, bedeutet, mit der Massgabe, dass, wenn $-N(R^1)-$ und $-Z$ unmittelbar nur über ein einziges Kohlenstoffatom miteinander verbunden sind und $R^1$ Wasserstoff bedeutet, Q für $-CH(COOH)-$, $-CH(COOC_2H_5)-$, $-CH(CH_2OH)-$, $-CH(CHOH-CH_3)-$, $-CH(CH_2CH_2-S-CH_3)-$, $-C(CH_3)(COOH)-$, $-C(CH_3)(COOC_2H_5)-$, $-C(CH_3)(CH_2OH)-$ oder $-C(CH_3)_2-$ steht.

13. Verbindungen nach Anspruch 1, dadurch gekennzeichnet, dass X Wasserstoff, $R^1$ Wasserstoff oder Methyl, Q ein Mitglied der Gruppe $-CH_2-$, $-(CH_2)_2-$, $-(CH_2)_3-$, $-CH(CH_3)-CH_2-$, $-CH_2-CH(CH_3)-$, $-CH(CH_2OH)-CH_2-$, $-CH_2-CH(CH_2OH)-$, $-CH(CH_3)-$, $-CH(CH_2OH)-$, $-CH(CHOH-CH_3)-$ oder $-CH(CH_2CH_2-S-CH_3)-$ und Z $-COOH$, $-COOC_1-C_4$-Alkyl, $-COO-CH_2-CH_2-O-CH_3$ oder $-CN$, oder das Strukturelement $-N(R^1)-Q-Z$ einen Pyrrolidinoring, in welchem Z in 2-Position steht, bedeutet, mit der Massgabe, dass, wenn $-N(R^1)-$ und $-Z$ unmittelbar nur über ein einziges Kohlenstoffatom miteinander verbunden sind und $R^1$ Wasserstoff bedeutet, Q für $-CH(CH_2OH)-$, $-CH(CHOH-CH_3)-$ oder $-C(CH_2CH_2-S-CH_3)-$ steht.

14. Verbindungen nach Anspruch 1, dadurch gekennzeichnet, dass X Wasserstoff, $R^1$ Wasserstoff, Q eine Aethylen- oder Trimethylenbrücke und Z $-COOR^8$ oder $-CONR^9R^{10}$, wobei $R^8$ für Wasserstoff, $C_1-C_3$-Alkyl oder Allyl und $R^9$ und $R^{10}$ für Wasserstoff stehen, bedeuten.

15. N-[2-nitro-5-(2-chlor-4-trifluormethyl-phenoxy)-phenyl]-3-amino-propionsäure-methylester.

16. N-[2-nitro-5-(2-chlor-4-trifluormethyl-phenoxy)-phenyl]-4-amino-n-buttersäure-methylester.

17. N-[2-nitro-5-(2-chlor-4-trifluormethyl-phenoxy)-phenyl]-3-amino-propionsäure-isopropylester.

18. N-[2-nitro-5-(2-chlor-4-trifluormethyl-phenoxy)-phenyl]-3-amino-propionsäure-äthylester.

19. N-[2-nitro-5-(2-chlor-4-trifluormethyl-phenoxy)-phenyl]-3-amino-propionsäure.

20. N-[2-nitro-5-(2-chlor-4-trifluormethyl-phenoxy)-phenyl]-4-amino-n-buttersäure.

21. 3-[N-{2-nitro-5-(2-chlor-4-trifluormethyl-phenoxy)-phenyl}-amino]-propionsäure-amid.

22. N-[2-nitro-5-(2-chlor-4-trifluormethyl-phenoxy)-phenyl]-3-amino-propionsäure-allylester.

23. Verfahren zur Herstellung von Verbindungen der Formel I nach Anspruch 1, dadurch gekennzeichnet, dass man

a) eine Verbindung der Formel II

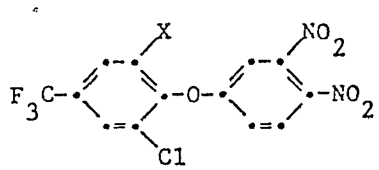

(II)

worin X die für Formel I angegebene Bedeutung hat, mit einer Verbindung der Formel III

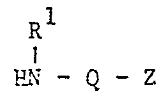

(III)

worin $R^1$, Q und Z die für Formel I angegebenen Bedeutungen haben, in Gegenwart einer Base umsetzt, oder

b) eine Verbindung der Formel IV

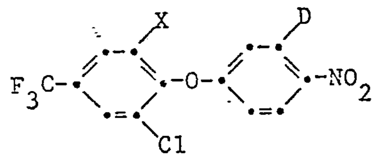

(IV)

worin X die für Formel I angegebene Bedeutung hat und D einen abspaltbaren Rest bedeutet, mit einer Verbindung der Formel III

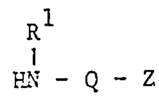

(III)

worin $R^1$, Q und Z die für Formel I angegebenen Bedeutungen haben, umsetzt, oder

c) zur Herstellung von Verbindungen der Formel I, in denen $R^1$ für $C_1$—$C_4$—Alkylcarbonyl steht, eine Verbindung der Formel V

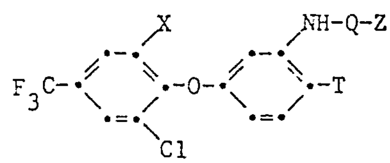

(V)

worin X, Q und Z die für Formel I angegebenen Bedeutungen haben und T Wasserstoff oder Nitro bedeutet, mit einem Säureanhydrid oder Säurechlorid umsetzt und, wenn T Wasserstoff bedeutet, das erhaltene Reaktionsprodukt nitriert, oder

d) eine Verbindung der Formel VII

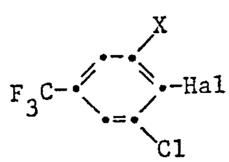

(VII)

worin X die für Formel I angegebene Bedeutung hat und Hal ein halogenatom bedeutet, mit einer Verbindung der Formel VIII

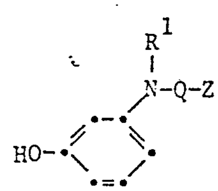

(VIII)

worin $R^1$, Q und Z die für Formel I angegebenen Bedeutungen haben, in Gegenwart einer Base bei erhöhter Temperatur umsetzt und das erhaltene Reaktionsprodukt der Formel IX

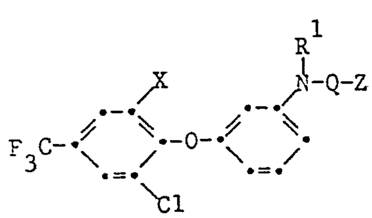

(IX)

worin X, $R^1$, Q und Z die für Formel I angegebenen Bedeutungen haben, nitriert, oder

e) zur Herstellung von Verbindungen der Formel I, in denen $R^1$ für $C_1$—$C_4$—Alkyl steht, eine Verbindung der Formel V, worin X, Q, Z und T die angegebenen Bedeutungen haben, mit einem entsprechenden Alkylierungsmittel umsetzt, und

f) gegebenenfalls die erhaltenen Verbindungen der Formel I, in denen Z für eine Carboxylgruppe steht, in die entsprechenden Carboxylsäure-Derivate überführt.

24. Herbizides Mittel, dadurch gekennzeichnet, dass es als aktive Komponente mindestens eine Verbindung der Formel I nach Anspruch 1 enthält.

25. Mittel nach Anspruch 24, dadurch gekennzeichnet, dass es 0,1 bis 99 Gewichtsprozent einer Verbindung der Formel I nach Anspruch 1, 1 bis 99,9 Gewichtsprozent an Zusatzstoffen und 0 bis 25 Gewichtsprozent eines Tensides enthält.

26. Mittel nach Anspruch 25, dadurch gekennzeichnet, dass es 0,1 bis 95 Gewichtsprozent einer Verbindung der Formel I nach Anspruch 1, 5 bis 99,8 Gewichtsprozent an Zusatzstoffen und 0,1 bis 25 Gewichtsprozent eines Tensides enthält.

27. Mittel nach Anspruch 24, dadurch gekennzeichnet, dass es als aktive Komponente eine Verbindung der Formel I enthält, in welcher X und $R^1$ Wasserstoff, Q eine Aethylen- oder Trimethylenbrücke und Z —$COOR^8$ oder —$CONR^9R^{10}$, wobei $R^8$ für Wasserstoff oder $C_1$—$C_3$—Alkyl und $R^9$ und $R^{10}$ für Wasserstoff stehen, bedeuten.

28. Mittel nach Anspruch 27, dadurch gekennzeichnet, dass es als aktive Komponente N-[2-nitro-5-(2-chlor-4-trifluormethyl-phenoxy)-phenyl]-3-amino-propionsäure-methylester enthält.

29. Mittel nach Anspruch 27, dadurch gekennzeichnet, dass es als aktive Komponente N-[2-nitro-5-(2-chlor-4-trifluormethyl-phenoxy)-phenyl]-4-amino-n-buttersäure-methylester enthält.

30. Mittel nach Anspruch 27, dadurch gekennzeichnet, dass es als aktive Komponente N-[2-nitro-5-(2-chlor-4-trifluormethyl-phenoxy)-phenyl]-3-amino-propionsäure-isopropylester enthält.

31. Mittel nach Anspruch 27, dadurch gekennzeichnet, dass es als aktive Komponente N-[-2-nitro-5-(2-chlor-4-trifluormethyl-phenoxy)-phenyl]-3-amino-propionsäure-äthylester enthält.

32. Mittel nach Anspruch 27, dadurch gekennzeichnet, dass es als aktive Komponente N-[2-nitro-5-(2-chlor-4-trifluormethylphenoxy)-phenyl]-3-amino-propionsäure enthält.

33. Mittel nach Anspruch 27, dadurch gekennzeichnet, dass es als aktive Komponente N-[2-nitro-5-(2-chlor-4-trifluormethyl-phenoxy)-phenyl]-4-amino-n-buttersäure enthält.

34. Mittel nach Anspruch 27, dadurch gekennzeichnet, dass es als aktive Komponente 3-[N-{2-nitro-5-(2-chlor-4-trifluormethyl-phenoxy)-phenyl}-amino]-propionsäure-amid enthält.

35. Mittel zur desiccation oder Defoliation von Baumwoll- oder Kartoffelpflanzen, dadurch gekennzeichnet, dass es als aktive Komponente eine Verbindung der Formel I nach Anspruch 1 enthält.

36. Mittel nach Anspruch 35, dadurch gekennzeichnet, dass es 0,1 bis 99 Gewichtsprozent einer Verbindung der Formel I nach Anspruch 1, 1 bis 99,9 Gewichtsprozent an Zusatzstoffen und 0 bis 25 Gewichtsprozent eines Tensides enthält.

37. Mittel nach Anspruch 36, dadurch gekennzeichnet, dass es 0,1 bis 95 Gewichtsprozent einer Verbindung der Formel I nach Anspruch 1, 5 bis 99,8 Gewichtsprozent an Zusatzstoffen und 0,1 bis 25 Gewichtsprozent eines Tensides enthält.

38. Mittel nach Anspruch 35, dadurch gekennzeichnet, dass es als aktive Komponente eine Verbindung der Formel I enthält, in welcher X und $R^1$ Wasserstoff, Q eine Aethylen- oder Trimethylenbrücke und Z Methoxycarbonyl oder Allyloxycarbonyl bedeuten.

39. Mittel nach Anspruch 38, dadurch gekennzeichnet, dass es als aktive Komponente N-[2-nitro-5-(2-chlor-4-trifluormethyl-phenoxy)-phenyl]-4-amino-n-buttersäure-methylester enthält.

40. Mittel nach Anspruch 38, dadurch gekennzeichnet, dass es als aktive Komponente N-[2-nitro-5-(2-chlor-4-trifluormethyl-phenoxy)-phenyl]-3-amino-propionsäure-methylester enthält.

41. Mittel nach Anspruch 38, dadurch gekennzeichnet, dass es als aktive Komponente N-[2-nitro-5-(2-chlor-4-trifluormethyl-phenoxy)-phenyl]-3-amino-propionsäure-allylester enthält.

42. Verfahren zur Herstellung eines Mittels nach Ansprüchen 24 und 35, dadurch gekennzeichnet, dass man eine oder mehrere aktive Komponenten mit festen oder flüssigen Trägerstoffen und gegebenenfalls Tensiden innig vermischt.

43. Verwendung von Verbindungen der Formel I nach Anspruch 1 oder einem Mittel, welches eine Verbindung der Formel I enthält, zur Bekämpfung von unerwünschtem Pflanzenwuchs.

44. Verwendung nach Anspruch 43 zur Bekämpfung von Unkräutern in Kulturpflanzenbeständen.

45. Verwendung nach Anspruch 44 zur Bekämpfung von Unkräutern in Getreidekulturen.

46. Verwendung nach Anspruch 45 zur pre-emergenten Bekämpfung von Unkräutern in Getreidekulturen.

47. Verwendung nach Anspruch 46 zur pre-emergenten Bekämpfung dikotyler Unkräuter in Getreidekulturen.

48. Verwendung nach Anspruch 47 zur pre-emergenten Bekämpfung dikotyler Unkräuter in Weizen, Gerste und Reis.

49. Verwendung nach Anspruch 45 zur post-emergenten Bekämpfung dikotyler Unkräuter in Getreidekulturen.

50. Verfahren zur Bekämpfung von unerwünschtem Pflanzenwuchs, dadurch gekennzeichnet, dass

man eine Verbindung der Formel I nach Anspruch 1 oder ein Mittel, welches eine Verbindung der Formel I enthält, auf Pflanzen, deren Standorte oder Pflanzenteile appliziert.

51. Verwendung von Verbindungen der Formel I oder eines Mittels, welches eine Verbindung der Formel I enthält, zur Desiccation oder Defoliation von Baumwoll- oder Kartoffelpflanzen.

52. Verfahren zur Desiccation oder Defoliation von Baumwoll- oder Kartoffelpflanzen, dadurch gekennzeichnet, dass man eine Verbindung der Formel I oder ein Mittel, welches eine Verbindung der Formel I enthält, vor der Ernte auf die Pflanzen appliziert.

## Claims

1. A compound of the formula I

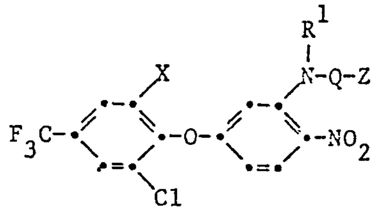

(I)

wherein

X is hydrogen, chlorine, fluorine or bromine,

$R^1$ is hydrogen, nitroso, or $C_1$-$C_4$ alkyl, $C_2$-$C_4$ alkenyl, $C_1$-$C_4$ alkylcarbonyl or $C_2$-$C_4$ alkenylcarbonyl, each of which is unsubstituted or substituted by one or more halogen atoms,

Q is a straight chain or branched alkylene or alkenylene radical of up to 5 carbon atoms which contains 0 to 3 side chains in addition to the main chain, which main chain is saturated or unsaturated and unsubstituted or substituted by one or two members selected from the group consisting of —$OR^2$, —$SR_3$ or —$COOR^4$, with none of the carbon atoms being disubstituted, and which side chains are unsaturated and unsubstituted or one or two of said side chains are substituted by —$OR^5$, —$SR_6$ or —$COOOR_7$, with the proviso that only one of the side chains may be substituted simultaneously with the main chain, and $R^2$, $R^3$, $R^4$, $R^5$, $R^6$ and $R^7$ are hydrogen or $C_1$-$C_4$ alkyl, and

Z is —$COO^{\ominus}Me^{\oplus}$, —$COOR^8$, —$COSR^8$, —$CONR^9R^{10}$, —$COR^{11}$ or —CN, where $Me^{\oplus}$ is a cation, $R^8$ is hydrogen, $C_1$-$C_8$ alkyl which is unsubstituted or substituted by halogen, cyano or $C_1$-$C_4$ alkoxy, or is $C_2$-$C_8$ alkenyl or $C_3$-$C_8$ alkynyl, $R^9$ is hydrogen, $C_1$-$C_4$ alkyl or $C_3$-$C_6$ cycloalkyl, $R^{10}$ is hydrogen, amino, hydroxyl, $C_1$-$C_4$ alkyl which is unsubstituted or substituted by $C_1$-$C_4$ alkoxy, or is $C_2$—$C_4$ alkynyl, $C_1$-$C_4$ alkoxy or $C_3$-$C_6$-cycloalkylaminocarbonyl and $R^{11}$ is hydrogen or $C_1$-$C_4$ alkyl which is unsubstituted or substituted by $C_1$-$C_4$ alkoxy,

or the structural unit —$N(R^1)$—Q—Z is a pyrrolidino ring which is substituted in the 2-position by Z and G', wherein G' is hydrogen or $C_1$-$C_4$ alkyl,

with the proviso that, if —$N(R^1)$— and —Z are linked to each other direct only through a single carbon atom and $R^1$ is hydrogen, Q is —$CH(OR^2)$—, —$CH(COOR^4)$—, —$CH(C_1$—$C_4$—alkyl substituted by —$OR^5$, —$SR^6$ or —$COOR^7$), —$C(C_1$—$C_4$ alkyl)$(OR^2)$—, —$C(C_1$—$C_4$ alkyl)$(COOR^4)$—, —$C(CH_3)(C_1$—$C_3$ alkyl substituted by —$OR^5$, —$SR^6$ or —$COOR^7$)—, —$C(C_2H_5)(C_1$—$C_2$ alkyl substituted by —$OR^5$, —$SR^6$ or —$COOR^7$)—, $C(C_3H_7)(CH_2OR^5)$—, —$C(C_3H_7)(CH_2SR^6)$—, —$C(C_3H_7)(CH_2COOR^7)$—, —$C(CH_3)$—$(C_3H_7)$— or —$C(C_2H_5)_2$—.

2. A compound according to claim 1, wherein X and Q are as defined in claim 1, $R^1$ is hydrogen, nitroso, $C_1$-$C_4$ alkyl or $C_1$-$C_4$ alkylcarbonyl which is unsubstituted or substituted by one or more halogen atoms, and Z is —$COO^{\ominus}Me^{\oplus}$, —$COOR^8$, $CONR^9R^{10}$ or —CN, where $Me^{\oplus}$ is a cation, $R^8$ is hydrogen, $C_1$-$C_4$ alkyl which is unsubstituted or substituted by halogen or $C_1$-$C_4$ alkoxy, or is $C_2$-$C_4$ alkenyl ; $R^9$ is hydrogen, $C_1$-$C_4$-alkyl or $C_3$-$C_6$ cycloalkyl and $R^{10}$ is hydrogen, amino, hydroxyl, $C_1$-$C_4$ alkyl which is unsubstituted or substituted by $C_1$-$C_4$ alkoxy, or is $C_2$-$C_4$ alkynyl, $C_1$-$C_4$ alkoxy or $C_3$-$C_6$ cycloalkylaminocarbonyl, or the structural unit —$N(R^1)$—Q—Z is a pyrrolidino ring which is substituted in the 2-position by Z and G', wherein G' is hydrogen or $C_1$-$C_4$ alkyl, with the proviso that, if —$N(R^1)$— and —Z are linked to each other direct only through a single carbon atom and $R^1$ is hydrogen, Q is —$CH(COOR^4)$—, —$CH(C_1$—$C_4$ alkyl substituted by —$OR^5$, —$SR^6$ or —$COOR^7$), —$C(C_1$—$C_4$ alkyl)$(COOR^4)$—, —$C(CH_3)(C_1$—$C_3$ alkyl substituted by —$OR^5$, —$SR^6$ or —$COOR^7$)—, —$C(C_2H_5)(C_1$—$C_2$ alkyl substituted by —$OR^5$, —$SR_6$ or —$COOR^7$)—, —$C(C_3H_7)(CH_2OR^5)$—, —$C(C_3H_7)(CH_2SR^6)$—, —$C(C_3H_7)(CH_2COOR^7)$—, —$C(CH_3)(C_3H_7)$— or —$C(C_2H_5)_2$—.

3. A compound according to claim 1, wherein X is as defined in claim 1, $R^1$ is hydrogen or $C_1$-$C_4$ alkyl, Q is a straight chain or branched alkylene radical of up to 5 carbon atoms which contains 0 to 2 side chains in addition to the main chain which directly links the structural units —$N(R^1)$— and —Z, which main chain is unsubstituted or mono- or disubstituted by —$OR^2$, —$SR^3$ and —$COOR^4$, and none of the carbon atoms is disubstituted and each carbon atom carries at least one hydrogen atom, and the side chains are unsubstituted or substituted by —$OR^5$, —$SR^6$ or —$COOR^7$, with the proviso that only one of

said side chains may be substituted simultaneously with the main chain, and $R^2$, $R^3$, $R^4$, $R^5$, $R^6$ and $R^7$ are hydrogen or $C_1$-$C_4$ alkyl, and Z is $—COO^{\ominus}Me^{\oplus}$, $—COOR^8$, $—CONR^9R^{10}$, $—COR^{11}$ or —CN, where $Me^{\oplus}$ is a metal cation, $R^8$ is hydrogen or $C_1$-$C_4$ alkyl, $R^9$ and $R^{10}$ are hydrogen or $C_1$-$C_4$ alkyl and $R^{11}$ is $C_1$-$C_4$ alkyl substituted by $C_1$-$C_4$ alkoxy, or the structural unit $—N(R^1)—Q—Z$ is a pyrrolidino ring in which Z is in the 2-position, with the proviso that, if $—N(R^1)—$ and —Z are linked to each other direct only through a single carbon atom and $R^1$ is hydrogen, Q is $CH(OR^2)—$, $—CH(COOR^4)—$, or $—CH(C_1—C_4$ alkyl substituted by $—OR^5$, $—SR^6$ or $—COOR^7)$.

4. A compound according to claim 1, wherein X is as defined in claim 1, $R^1$ is hydrogen or $C_1$-$C_4$ alkyl, Q is an alkylene radical of up to 5 carbon atoms which consists

a) only of the main chain which directly links the structural units $—NR^1—$ and —Z and comprises 1 to 3 carbon atoms and is unsusbstituted or monosubstituted by $—OR^2$, $—SR^3$ or $—COOR^4$, or

b) of the main chain and a side chain, which main chain comprises 1 to 3 carbon atoms and is unsubstituted, and which side chain is unsubstituted or substituted by $—OR^5$, $—SR^6$ or $—COOR^7$, and $R^2$, $R^3$, $R^4$, $R^5$, $R^6$ and $R^7$ are hydrogen or $C_1$-$C_4$ alkyl, and Z is $—COO^{\ominus}Me^{\oplus}$, $—COOR^8$, $—COR^{11}$ or —CN, where $Me^{\oplus}$ is a metal cation, $R^8$ is hydrogen or $C_1$-$C_4$ alkyl, and $R^{11}$ is $C_1$-$C_4$ alkyl substituted by $C_1$-$C_4$ alkoxy, or the structural unit $—N(R^1)—Q—Z$ is a pyrrolidino ring in which Z is in the 2-position, with the proviso that, if $—N(R^1)—$ and —Z are linked to each other direct only through a single carbon atom and $R^1$ is hydrogen, Q is $CH(OR^2)—$, $—CH(COOR^4)—$, or $—CH(C_1—C_4$ alkyl substituted by $—OR^5$, $—SR^6$ or $—COOR^7)$.

5. A compound according to claim 1, wherein X is hydrogen or chlorine, $R^1$ is hydrogen, nitroso, methyl, methylcarbonyl, ethylcarbonyl, vinylcarbonyl, chloromethylcarbonyl, trichloromethylcarbonyl or trifluoromethylcarbonyl, Q is a straight chain or branched alkylene or alkenylene radical of up to 5 carbon atoms which contains 0 to 2 side chains in addition to the main chain which directly links the structural units $N(R^1)—$ and —Z, which main chain is unsaturated or saturated and unsubstituted or monosubstituted by $—OR^2$, $—SR^3$ or $—COOR^4$, and which side chains are unsaturated and unsubstituted or one of said side chains is substituted by $—OR^5$, $—SR^6$ or $—COOR^7$, with the proviso that the main chain and the side chains are simultaneously not substituted, and $R^2$, $R^3$, $R^4$, $R^5$, $R^6$ and $R^7$ are hydrogen or $C_1$-$C_4$ alkyl, and Z is $—COO^{\ominus}Me^{\oplus}$, $—COOR^8$, $—CONR^9R^{10}$ or —CN, where $Me^{\oplus}$ is a cation, $R^8$ is hydrogen, $C_1$-$C_8$ alkyl which is unsubstituted or substituted by halogen or $C_1$-$C_4$ alkoxy, or is $C_2$-$C_8$-alkenyl or $C_3$-$C_8$ alkynyl, $R^9$ is hydrogen, $C_1$-$C_4$ alkyl or $C_3$-$C_6$ cycloalkyl, $R^{10}$ is hydrogen, amino, hydroxyl, $C_1$-$C_4$ alkyl which is unsubstituted or substituted by $C_1$-$C_4$ alkoxy, or is $C_2$-$C_4$ alkynyl, $C_1$-$C_4$ alkoxy or $C_3$-$C_6$-cycloalkylaminocarbonyl, or the structural unit $—N(R^1)—Q—Z$ is a pyrrolidino ring which is substituted in the 2-position by Z and G', wherein G' is hydrogen or $C_1$-$C_4$ alkyl, with the proviso that, if $—N(R^1)—$ and —Z are linked to each other direct only through a single carbon atom and $R^1$ is hydrogen, Q is $—CH(COOR^4)—$, $—CH(C_1—C_4$ alkyl substituted by $—OR^5$, $—SR^6$ or $COOR^7)$, $—C(C_1—C_4$ alkyl$)(COOR^4)—$, $—C(CH_3)(C_1—C_3$ alkyl substituted by $—OR^5$, $—SR^6$ or $—COOR^7)—$, $—C(C_2H_5)(C_1—C_2$ alkyl substituted by $—OR^5$, $SR_6$ or $—COOR^7)—$, $—C(C_3H_7)(CH_2OR^5)—$, $—C(C_3H_7)(CH_2SR^6)—$, $—C(C_3H_7)(CH_2COOR^7)—$, $—C(CH_3)(C_3H_7)—$ or $—C(C_2H_5)_2—$.

6. A compound according to claim 1, wherein X is hydrogen or chlorine, $R^1$ is hydrogen or methyl, Q is an alkylene radical of up to 5 carbon atoms which consists

a) only of the main chain which directly links the structural units $—N(R^1)—$ and —Z and comprises 1 to 3 carbon atoms and is unsubstituted or monosubstituted by $—OR^2$, $—SR^3$ or $—COOR^4$, or

b) of the main chain and a side chain, which main chain comprises 1 to 3 carbon atoms and is unsubstituted, and which side chain is unsubstituted or substituted by $—OR^5$, $—SR^6$ or $—COOR^7$, and $R^2$, $R^3$, $R^4$, $R^5$, $R^6$ and $R^7$ are hydrogen or $C_1$-$C_4$ alkyl, and Z is $—COO^{\ominus}Me^{\oplus}$, $—COOR^8$, $—COR^{11}$ or —CN, where $Me^{\oplus}$ is a metal cation, $R^8$ is hydrogen or $C_1$-$C_4$ alkyl and $R^{11}$ is $C_1$-$C_4$ alkyl substituted by $C_1$-$C_4$ alkoxy, or the structural unit $—N(R^1)—Q—Z$ is a pyrrolidino ring in which Z is in the 2-position, with the proviso that, if $—N(R^1)—$ and —Z are linked to each other direct only through a single carbon atom and $R^1$ is hydrogen, Q is $CH(OR^2)—$, $—CH(COOR^4)—$, or $—CH(C_1—C_4$ alkyl substituted by $—OR^5$, $—SR^6$ or $—COOR^7)$.

7. A compound according to claim 1, wherein X is hydrogen or chlorine, $R^1$ is hydrogen, nitroso or $C_1$-$C_4$ alkyl, $C_2$-$C_4$ alkenyl, $C_1$-$C_4$ alkylcarbonyl or $C_1$-$C_4$ alkenylcarbonyl, which radicals are unsubstituted or substituted by 1 to 3 chlorine or fluorine atoms, Q is 1,5-pentamethylene or an alkylene or alkenylene radical containing a total of up to 3 carbon atoms and which consists

a) only of the main chain which directly links the structural units $—N(R^1)—$ and —Z and which is saturated or unsaturated and unsubstituted or is monosubstituted by carboxy or $C_1$-$C_4$ alkoxycarbonyl, or

b) of a main chain of 2 carbon atoms which is saturated or unsaturated and a side chain of one carbon atom, i) which main chain is unsubstituted or monosubstituted by carboxyl or $C_1$-$C_4$ alkoxycarbonyl and which side chain consists of an unsubstituted methyl group, or ii) which main chain is unsubstituted and which side chain consists of a methyl group which is substituted by hydroxy or $C_1$-$C_4$ alkylthio, or

c) of a member selected from the group consisting of $—CH(COOH)—$, $—CH(COOC_1—C_4$ alkyl$)—$, $—C(C_1—C_2$ alkyl$)(COOH)—$, $—C(C_1—C_2$ alkyl$)(COOC_1—C_4—$alkyl, $—CH(CH_2OH)—$, $—CH(CH_2—S—C_1—C_4$ alkyl$)—$, $—CH(C_2H_5OH)—$, $—CH(C_2H_5—S—C_1—C_4$ alkyl$)—$,

$—C(CH_3)(CH_2OH)—$, $—C(CH_3)(CH_2—S—C_1—C_4$ alkyl)—, $—C(CH_3)_2$ or $—CH(C_1—C_2$ alkyl) and Z is $—COO^{\ominus}Me^{\oplus}$, $—COOR^8$, $—CONR^9R^{10}$ or —CN, where $Me^{\oplus}$ is an alkali metal cation, $R^8$ is hydrogen, $C_1$-$C_8$ alkyl which is unsubstituted or monosubstituted by chlorine or $C_1$-$C_4$ alkoxy, or is $C_2$-$C_8$ alkenyl or $C_3$-$C_8$ alkynyl, $R^9$ is hydrogen, $C_1$-$C_4$ alkyl or $C_3$-$C_6$ cycloalkyl and $R^{10}$ is hydrogen, amino, hydroxyl, $C_1$-$C_4$ alkyl which is unsubstituted or monosubstituted by $C_1$-$C_4$ alkoxy, or is $C_2$-$C_4$ alkenyl, $C_1$-$C_4$ alkoxy or $C_3$-$C_6$ cycloalkylaminocarbonyl, or the structural unit $—N(R^1)—Q—Z$ is a pyrrolidino ring which is substituted in the 2-position by Z and G', with the proviso that, if $—N(R^1)—$ and —Z are linked to each other direct only through a single carbon atom and $R^1$ is hydrogen, O is —CH(COOH)—, $—CH(COOC_1—C_4$ alkyl)—, $—C(C_1—C_2$ alkyl)(COOH)—, $—C(C_1—C_2$ alkyl)— $(COOC_1—C_4$ alkyl)—, $—CH(CH_2OH)—$, $—CH(CH_2—S—C_1—C_4$ alkyl)—, $—CH(C_2H_5OH)—$, $—CH(C_2H_5—S—C_1—C_4$ alkyl)—, $—C(CH_3)(CH_2OH)—$, $—C(CH_3)(CH_2—S—C_1—C_4$ alkyl)— or $—C(CH_3)_2$.

8. A compound according to claim 1, wherein X is hydrogen, $R^1$ is hydrogen, Q is 1,2-ethylene bridge and Z is $—COO^{\ominus}Me^{\oplus}$, $—COOR^8$, $COSR^8$, $—CONR^9R^{10}$, $—COR^{11}$ or —CN, where $Me^{\oplus}$ is a cation, $R^8$ is hydrogen, $C_1$-$C_8$ alkyl which is unsubstituted or substituted by halogen, cyano or $C_1$-$C_4$ alkoxy, or is $C_2$-$C_8$ alkenyl or $C_3$-$C_8$ alkynyl, $R^9$ is hydrogen, $C_1$-$C_4$ alkyl or $C_3$-$C_6$ cycloalkyl, $R^{10}$ is hydrogen, amino, hydroxyl, $C_1$-$C_4$ alkyl which is unsubstituted or substituted by $C_1$-$C_4$ alkoxy, or is $C_2$-$C_4$ alkynyl, $C_1$-$C_4$ alkoxy or $C_3$-$C_6$ cycloalkylaminocarbonyl, and $R^{11}$ is hydrogen or $C_1$-$C_4$ alkyl which is unsubstituted or substituted by $C_1$-$C_4$ alkoxy.

9. A compound according to claim 1, wherein X and $R^1$ are as defined in claim 1, Q is 1,5-pentamethylene or an alkylene or alkenylene radical containing a total of up to 3 carbon atoms and which consists

a) only of the main chain which directly links the structural units $—N(R^1)—$ and —Z and is saturated or unsaturated and unsubstituted or substituted by carboxyl, methoxycarbonyl or ethoxycarbonyl, or

b) of a main chain of 2 carbon atoms which is saturated or unsaturated and a side chain of one carbon atom, i) which main chain is unsubstituted or monosubstituted by carboxyl, methoxycarbonyl or ethoxycarbonyl and which side chain consists of an unsubstituted methyl group, or ii) which main chain is unsubstituted and which side chain consists of hydroxymethyl, or

c) of a member selected from the group consisting of —CH(COOH)—, $—CH(COOCH_3)—$, $—CH(COOC_2H_5)—$, $—CH(CH_2OH)—$, $—CH(CHOH—CH_3)—$, $—CH(CH_2CH_2OH)—$, $—CH(CH_2CH_2—S—CH_3)—$, $—C(CH_3)(COOH)—$, $—C(CH_3)(COOCH_3)—$, $—C(CH_3)(COOC_2H_5)—$, $—C(CH_3)(CH_2OH)—$, $—CH(C_1—C_2$ alkyl)— or $—C(CH_3)_2$ and Z is $—COO^{\ominus}Me^{\oplus}$, $—COOR^8$, $—CONR^9R^{10}$ or —CN, where $Me^{\oplus}$ is a sodium cation, $R^8$ is hydrogen, $C_1$-$C_4$ alkyl, 2-chloroethyl, 2-methoxyethyl, allyl, or prop-2-ynyl, $R^9$ is hydrogen, methyl or cyclohexyl, and $R^{10}$ is hydrogen, amino, hydroxyl, $C_1$-$C_3$ alkyl, 2-methoxyethyl, but-3-yn-2-yl, methoxy or cyclohexylaminocarbonyl, with the proviso that, if $—N(R^1)—$ and —Z are linked to each other direct only through a single carbon atom and $R^1$ is hydrogen, Q is $—C(CH_3)_2—$, —CH(COOH)—, $—CH(COOCH_3)—$, $—CH(COOC_2H_5)—$, $—CH(CH_2OH)—$, $—CH(CHOH—CH_3)—$, $—CH(CH_2CH_2OH)—$, $—CH(CH_2CH_2—S—CH_3)—$, $—C(CH_3)(COOH)—$, $—C(CH_3)(COOCH_3)—$, $—C(CH_3)(COOC_2H_5)—$ or $C(CH_3)(CH_2OH)—$.

10. A compound according to claim 1, wherein X is hydrogen or chlorine, $R^1$ is hydrogen, nitroso, $C_1$-$C_4$ alkyl, $C_1$-$C_4$ alkylcarbonyl which is unsubstituted or substituted by 1 to 3 chlorine or fluorine atoms, or is $C_2$-$C_4$ alkenylcarbonyl ; Q is 1,5-pentamethylene or is an alkylene or alkenylene radical containing a total of up to 3 carbon atoms and which consists

a) only of the main chain which directly links the structural units $—N(R^1)—$ and —Z and is saturated or unsaturated and unsubstituted or monosubstituted by carboxy or $C_1$-$C_4$ alkoxycarbonyl, or

b) of a main chain of 2 carbon atoms which is saturated or unsaturated and a side chain of one carbon atom, i) which main chain is unsubstituted or monosubstituted by carboxy or $C_1$-$C_4$ alkoxycarbonyl and which side chain consists of an unsubstituted methyl group, or ii) which main chain is unsubstituted and which side chain consists of a methyl group which is substituted by hydroxy or $C_1$-$C_4$ alkylthio, or

c) of a member selected from the group consisting of —CH(COOH)—, $—CH(COOC_1—C_4$ alkyl)—, $—C(C_1—C_2$ alkyl)(COOH)—, $—C(C_1—C_2$ alkyl)$(COOC_1—C_4$ alkyl)—, $—CH(CH_2OH)—$, $—CH(CH_2—S—C_1—C_4$ alkyl)—, $—CH(C_2H_5OH)—$, $—CH(C_2H_5—S—C_1—C_4$ alkyl)—, $—C(CH_3)(CH_2OH)—$, $—C(CH_3)(CH_2—S—C_1—C_4$ alkyl)—, $—C(CH_3)_2$ or $—CH(C_1—C_2$ alkyl), and Z is $—COO^{\ominus}Me^{\oplus}$, $—COOR^8$, $—CONR^9R^{10}$ or —CN, where $Me^{\oplus}$ is an alkali metal cation, $R^8$ is hydrogen, $C_1$-$C_4$ alkyl which is unsubstituted or monosubstituted by chlorine or $C_1$-$C_4$ alkoxy, or is $C_2$-$C_4$ alkenyl, $R^9$ is hydrogen, $C_1$-$C_4$ alkyl or $C_3$-$C_6$ cycloalkyl and $R^{10}$ is hydrogen, amino, hydroxyl, $C_1$-$C_4$ alkyl, which is unsubstituted or monosubstituted by $C_1$-$C_4$ alkoxy, or is $C_2$-$C_4$ alkynyl, $C_1$-$C_4$ alkoxy or $C_3$-$C_6$ cycloalkylaminocarbonyl, or the structural unit $—N(R^1)—Q—Z$ is a pyrrolidino ring which is substituted in the 2-position by Z and G', wherein G' is hydrogen or $C_1$-$C_4$ alkyl, with the proviso that, if $—N(R^1)—$ and —Z are linked to each other direct only through a single carbon atom and $R^1$ is hydrogen, Q is —CH(COOH)—, $—CH(COOC_1—C_4$ alkyl)—, $—C(C_1—C_2$ alkyl)(COOH)—, $—C(C_1—C_2$ alkyl)$(COOC_1—C_4$ alkyl)—, $—CH(CH_2OH)—$, $—CH(CH_2—S—C_1—C_4$ alkyl)—, $—CH(C_2H_5OH)—$, $—CH(C_2H_5—S—C_1—C_4$ alkyl)—, $—C(CH_3)(CH_2OH)—$, $—C(CH_3)(CH_2—S—C_1—C_4$ alkyl)— or $—C(CH_3)_2$.

11. A compound according to claim 1, wherein X is hydrogen or chlorine, $R^1$ is hydrogen, nitroso,

methyl, methylcarbonyl, ethylcarbonyl, vinylcarbonyl, chloromethylcarbonyl, trichloromethylcarbonyl or trifluoromethylcarbonyl, Q is 1,5-pentamethylene or an alkylene or alkenylene radical containing a total of up to 3 carbon atoms, which radical consists

a) only of the main chain which directly links the structural units $—N(R^1)—$ and —Z and is saturated or unsaturated and unsubstituted or monosubstituted by carboxy, methoxycarbonyl or ethoxycarbonyl, or

b) of a main chain of 2 carbon atoms which is saturated or unsaturated and a side chain of one carbon atom, i) which main chain is unsubstituted or monosubstituted by carboxy, methoxycarbonyl or ethoxycarbonyl, and which side chain consists of an unsubstituted methyl group, or ii) which main chain is unsubstituted and which side chain consists of hydroxymethyl, or

c) of a member selected from the group consisting of $—CH(COOH)—$, $—CH(COOCH_3)—$, $—CH(COOC_2H_5)—$, $—CH(CH_2OH)—$, $—CH(CHOH—CH_3)—$, $—CH(CH_2CH_2OH)—$, $—CH(CH_2CH_2—S—CH_3)—$, $—C(CH_3)(COOH)—$, $—C(CH_3)(COOCH_3)—$, $—C(CH_3)(COOC_2H_5)—$, $—C(CH_3)(CH_2OH)—$ or $—C(CH_3)_2—$ and Z is $—COO^{\ominus}Me^{\oplus}$, $—COOR^8$, $—CONR^9R^{10}$ or —CN, where $Me^{\oplus}$ is a sodium cation, $R^8$ is hydrogen, $C_1$-$C_4$ alkyl, 2-chloroethyl, 2-methoxyethyl, allyl or prop-2-ynyl, $R^9$ is hydrogen, methyl or cyclohexyl, and $R^{10}$ is hydrogen, amino, hydroxyl, $C_1$-$C_3$ alkyl, 2-methoxyethyl, but-3-yn-2-yl, methoxy or cyclohexylaminocarbonyl, or the structural unit $—N(R^1)—Q—Z$ is a pyrrolidino ring which is substituted in the 2-position by Z and G', wherein G' is hydrogen or methyl, with the proviso that, if $—N(R^1)—$ and —Z are linked to each other direct only through a single carbon atom and $R^1$ is hydrogen, Q is $—C(CH_3)_2—$, $—CH(COOH)—$, $—CH(COOCH_3)—$, $—CH(COOC_2H_5)—$, $—CH(CH_2OH)—$, $—CH(CHOH—CH_3)—$, $—CH(CH_2CH_2OH)—$, $—CH(CH_2CH_2—S—CH_3)—$, $—C(CH_3)(COOH)—$, $—C(CH_3)(COOCH_3)—$, $—C(CH_3)(COOC_2H_5)—$ or $—C(CH_3)(CH_2OH)—$.

12. A compound according to claim 1, wherein X is hydrogen or chlorine, $R^1$ is hydrogen, nitroso, methyl, methylcarbonyl, ethylcarbonyl, chloromethylcarbonyl, trichloromethylcarbonyl or trifluoromethylcarbonyl, Q is 1,5-pentamethylene or an alkylene or alkenylene radical of up to 5 carbon atoms, which radical consists

a) only of the main chain which directly links the structural units $—N(R^1)—$ and —Z and is saturated or unsaturated and unsubstituted or monosubstituted by carboxy or ethoxycarbonyl, or

b) of a main chain of 2 carbon atoms which is saturated or unsaturated and a side chain of one carbon atom, i) which main chain is unsubstituted or monosubstituted by carboxy or ethoxycarbonyl and which side chain consists of an unsubstituted methyl group, or ii) which main chain is unsubstituted and which side chain consists of hydroxymethyl, or

c) of a member selected from the group consisting of $—CH(COOH)—$, $—CH(COOC_2H_5)—$, $—CH(CH_2OH)—$, $—CH(CHOH—CH_3)—$, $—CH(CH_2CH_2—S—CH_3)—$, $—C(CH_3)(COOH)—$, $—CH(CH_3)(COOC_2H_5)—$, $—C(CH_3)(CH_2OH)—$ or $—C(CH_3)_2$ and Z is $—COOR^8$, $—CONR^9R^{10}$ or —CN, where $R^8$ is hydrogen, $C_1$-$C_4$ alkyl, 2-chloroethyl, 2-methoxyethyl or allyl, $R^9$ is hydrogen, methyl or cyclohexyl, and $R^{10}$ is hydrogen, amino, hydroxyl, $C_1$-$C_3$ alkyl, 2-methoxyethyl, but-3-yn-2-yl, methoxy or cyclohexylaminocarbonyl, or the structural unit $—N(R^1)—Q—Z$ is a pyrrolidino ring which is substituted in the 2-position by Z and G', wherein G' is hydrogen or methyl, with the proviso that, if $—N(R^1)—$ and —Z are attached to each other direct only through a single carbon atom and $R^1$ is hydrogen, Q is $—CH(COOH)—$, $—CH(COOC_2H_5)—$, $—CH(CH_2OH)—$, $CH(CHOH—CH_3)—$, $—CH(CH_2CH_2—S—CH_3)—$, $—C(CH_3)(COOH)—$, $—C(CH_3)(COOC_2H_5)—$, $—C(CH_3)(CH_2OH)—$ or $—C(CH_3)_2—$.

13. A compound according to claim 1, wherein X is hydrogen, $R^1$ is hydrogen or methyl, Q is a member selected from the group consisting of $—CH_2—$, $—(CH_2)_2—$, $—(CH_2)_3—$, $—CH(CH_3)—CH_2—$, $—CH_2—CH(CH_3)—$, $—CH(CH_2OH)—CH_2—$, $—CH_2—CH(CH_2OH)—$, $—CH(CH_3)—$, $—CH(CH_2OH)—$, $—CH(CHOH—CH_3)—$ or $—CH(CH_2CH_2—S—CH_3)—$ and Z is —COOH, $—COO—C_1—C_4$ alkyl, $—COO—CH_2—CH_2—O—CH_3$ or —CN, or the structural unit $—N(R^1)—Q—Z$ is a pyrrolidino ring which Z is in the 2-position, with the proviso that, if $—N(R^1)—$ and —Z are linked to each other direct only through a single carbon atom and $R^1$ is hydrogen, Q is $—CH(CH_2OH)—$, $—CH(CHOH—CH_3)—$ or $—C(CH_2CH_2—S—CH_3)—$.

14. A compound according to claim 1, wherein X is hydrogen, $R^1$ is hydrogen, Q is an ethylene or trimethylene bridge and Z is $—COOR^8$ or $—CONR^9R^{10}$, where $R^8$ is hydrogen, $C_1$-$C_3$ alkyl or allyl, and $R^9$ and $R^{10}$ are hydrogen.

15. Methyl N-[2-nitro-5-(2-chloro-4-trifluoromethylphenoxy)-phenyl]-3-aminopropionate.

16. Methyl N-[2-nitro-5-(2-chloro-4-trifluoromethylphenoxy)-phenyl]-4-amino-n-butyrate.

17. Isopropyl N-[2-nitro-5-(2-chloro-4-trifluoromethylphenoxy)-phenyl]-3-aminopropionate.

18. Ethyl N-[2-nitro-5-(2-chloro-4-trifluoromethylphenoxy)-phenyl]-3-aminopropionate.

19. N-[2-Nitro-5-(2-chloro-4-trifluoromethylphenoxy)-phenyl]-3-aminopropionic acid.

20. N-[2-Nitro-5-(2-chloro-4-trifluoromethylphenoxy)-phenyl]-4-amino-n-butyric acid.

21. 3-[N-{2-Nitro-5-(2-chloro-4-trifluoromethylphenoxy)-phenyl}-amino]-propionamide.

22. Allyl N-[2-nitro-5-(2-chloro-4-trifluoromethylphenoxy)-phenyl]-3-aminopropionate.

23. A process for the preparation of a compound of the formula I according to claim 1, which process comprises

a) reacting a compound of formula II

(See formula page 34)

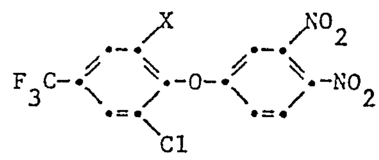

(II)

wherein X is as defined for formula I, with a compound of formula III

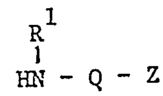

(III)

wherein $R^1$, Q and Z are as defined for formula I, in the presence of a base, or

b) reacting a compound of formula IV

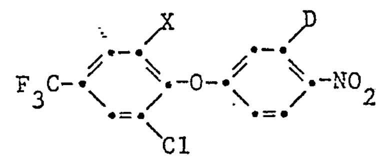

(IV)

wherein X is as defined for formula I and D is a removable radical, with a compound of formula III

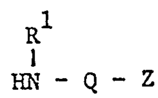

(III)

wherein $R^1$, Q and Z are as defined for formula I, or

c) reacting a compound of formula V

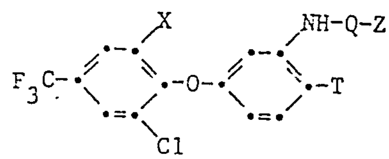

(V)

wherein X, Q and Z are as defined for formula I and T is hydrogen or nitro, with an acid hydride or acid chloride and, if T is hydrogen, nitrating the reaction product obtained, to give compounds of formula I, wherein $R^1$ is $C_1$-$C_4$-alkylcarbonyl, or

d) reacting a compound of formula VII

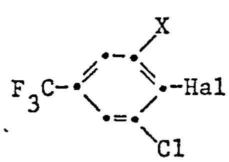

(VII)

wherein X is as defined for formula I and Hal is a halogen atom, with a compound of the formula VIII

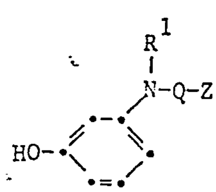

(VIII)

wherein $R^1$, Q and Z are as defined for formula I, in the presence of a base at elevated temperature, and nitrating the reaction product of the formula IX

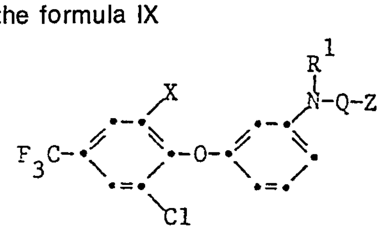

(IX)

wherein X, $R^1$, Q and Z are as defined for formula I, or

e) reacting a compound of formula V, wherein X, Q, Z and T are as defined reacting a compound of the formula V, wherein X, Q, Z and T are as defined, with a corresponding alkylating agent, to give compounds of formula I, wherein $R^1$ is $C_1$-$C_4$ alkyl, and

f) if desired, converting the compounds of formula I, wherein Z is a carboxyl group, in a manner known per se, into the corresponding carboxylic acid derivatives.

24. A herbicidal composition which contains as active component at least one compound of the formula I according to claim 1.

25. A composition according to claim 24, which contains 0.1 to 99 % by weight of a compound of formula I according to claim 1, 1 to 99.9 % by weight of adjuvants and 0 to 25 % by weight of a surfactant.

26. A composition according to claim 25, which contains 0.1 to 95 % by weight of a compound of formula I according to claim 1, 5 to 99.8 % by weight of adjuvants and 0.1 to 25 % by weight of a surfactant.

27. A composition according to claim 24, which contains as active component a compound of formula I, wherein X is hydrogen, $R^1$ is hydrogen, Q is an ethylene or trimethylene bridge and Z is —$COOR^8$ or —$CONR^9R^{10}$, where $R^8$ is hydrogen or $C_1$-$C_3$ alkyl and $R^9$ and $R^{10}$ are hydrogen.

28. A composition according to claim 27, which contains methyl N-[2-nitro-5-(2-chloro-4-trifluoromethylphenoxy)-phenyl]-3-aminopropionate as active component.

29. A composition according to claim 27, which contains methyl N-[2-nitro-5-(2-chloro-4-trifluoromethylphenoxy)-phenyl]-4-amino-n-butyrate as active component.

30. A composition according to claim 27, which contains isopropyl N-[2-nitro-5-(2-chloro-4-trifluoromethylphenoxy)-phenyl]-3-aminopropionate as active component.

31. A composition according to claim 27, which contains ethyl N-[2-nitro-5-(2-chloro-4-trifluoromethylphenoxy)-phenyl]-3-aminopropionate as active component.

32. A composition according to claim 27, which contains N-[2-nitro-5-(2-chloro-4-trifluoromethylphenoxy)-phenyl]-3-aminopropionic acid as active component.

33. A composition according to claim 27, which contains N-[2-nitro-5-(2-chloro-4-trifluoromethylphenoxy)-phenyl]-4-amino-n-butyric acid as active component.

34. A composition according to claim 27, which contains 3-[N-{2-nitro-5-(2-chloro-4-trifluoromethylphenoxy)-phenyl}-amino]-propionamide as active component.

35. A composition for desiccating or defoliating cotton or potato plants, which contains as active component a compound of the formula I according to claim 1.

36. A composition according to claim 35, which contains 0.1 to 99 % by weight of a compound of formula I according to claim 1, 1 to 99.9 % by weight of adjuvants and 0 to 25 % by weight of a surfactant.

37. A composition according to claim 36, which contains 0.1 to 95 % by weight of a compound of formula I according to claim 1, 5 to 99.8 % by weight of adjuvants and 0.1 to 25 % by weight of a surfactant.

38. A composition according to claim 35 which contains as active component a compound of formula I, wherein X and $R^1$ are hydrogen, Q is an ethylene or trimethylene bridge and Z is methoxycarbonyl or allyloxycarbonyl.

39. A composition according to claim 38, which contains methyl N-[2-nitro-5-(2-chloro-4-trifluoromethylphenoxy)-phenyl]-4-amino-n-butyrate as active component.

40. A composition according to claim 38, which contains methyl N-[2-nitro-5-(2-chloro-4-trifluoromethylphenoxy)-phenyl]-3-aminopropionate as active component.

41. A composition according to claim 38, which contains allyl N-[2-nitro-5-(2-chloro-4-trifluoromethylphenoxy)-phenyl]-3-aminopropionate as active component.

42. A process for the preparation of a composition according to either of claims 24 or 35, which process comprises homogeneously mixing one or more active components with solid or liquid carriers and optionally surfactants.

43. Use of a compound of formula I according to claim 1 or of a composition containing such a compound for controlling undesirable plant growth.

44. Use according to claim 43 for controlling weeds in crops of useful plants.

45. Use according to claim 44 for controlling weeds in crops of cereals.

46. Use according to claim 45 for controlling weeds preemergence in crops of cereals.

47. Use according to claim 46 for controlling dicotyledonous weeds in crops of cereals.

48. Use according to claim 47 for controlling dicotyledonous weeds postemergence in wheat, barley and rice.

49. Use according to claim 45 for controlling dicotyledonous weeds postemergence in crops of cereals.

50. A method of controlling undesirable plant growth, which comprises applying to plants, to the locus thereof or to parts of plants a herbicidally effective amount of a compound of the formula I according to claim 1 or of a composition containing such a compound.

51. A method of desiccating or defoliating cotton or potato plants, which comprises applying thereto a herbicidally effective amount of a compound of the formula I according to claim 1 or of a composition containing such a compound.

52. A method of desiccating or defoliating cotton or potato plants, which comprises applying thereto before harvesting a herbicidally effective amount of a compound of formula I according to claim 1 or of a composition containing such a compound.

**Revendications**

1. Composés de formule I

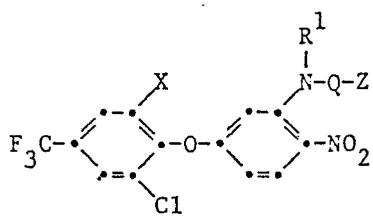

(I)

où

X représente un hydrogène, un chlore, un fluor ou un brome,

$R^1$ représente un hydrogène, un nitroso ou un alcoyle en C1 à C4, alcényle en C2 à C4, alcoyle en C1 à C4-carbonyle ou alcényle en C2 à C4-carbonyle, qui sont non substitués ou substitués par un ou plusieurs atomes d'halogène,

Q représente un radical alcoylène ou alcénylène à chaîne droite ou ramifiée ayant jusqu'à 5 atomes de carbone, où l'on trouve outre la chaîne principale de 0 à 3 chaînes latérales, où la chaîne principale est saturée ou non saturée ou mono- ou di-substituée par des substituants du groupe $—OR^2$, $—SR^3$ ou $—COOR^4$, où aucun des atomes de carbone n'est disubstitué, et les chaînes latérales sont non saturées et non substituées ou bien où une ou deux des chaînes latérales sont substituées par $—OR^5$, $—SR^6$ ou $—COOR^7$, où en même temps que la chaîne principale, seule, une des chaînes latérales peut être substituée, et $R^2$, $R^3$, $R^4$, $R^5$, $R^6$ et $R^7$ représentent un hydrogène ou un alcoyle en C1 à C4, et

Z représente $—COO^{\ominus}Me^{\oplus}$, $—COOR^8$, $—COSR^8$, $—CONR^9R^{10}$, $—COR^{11}$ ou —CN, où $Me^{\oplus}$ représente un cation, $R^8$ un hydrogène, un alcoyle en C1 à C8, qui est non substitué ou substitué par un halogène, un cyano ou un alcoxy en C1 à C4, un alcényle en C2 à C8 ou un alcynyle en C3 à C8, $R^9$ représente un hydrogène, un alcoyle en C1 à C4 ou un cycloalcoyle en C3 à C6, $R^{10}$ représente un hydrogène, un amino, un hydroxy, un alcoyle en C1 à C4, qui est non substitué ou substitué par un alcoxy en C1 à C4, un alcynyle en C2 à C4, un alcoxy en C1 à C4 ou un cycloalcoyle en C3 à C6-aminocarbonyle et $R^{11}$ représente un hydrogène ou un alcoyle en C1 à C4, qui est éventuellement substitué par un alcoxy en C1 à C4,

ou l'élément de structure $—N(R^1)—Q—Z$ est un noyau pyrrolidino, qui est substitué en position 2 par Z et G', où G' représente un hydrogène ou un alcoyle en C1 à C4,

avec la précision que lorsque $—N(R^1)—$ et —Z ne sont immédiatement liés l'un à l'autre que par un seul atome de carbone et $R^1$ représente un hydrogène, Q représente $—CH(OR^2)$, $—CH(COOR^4)—$, $—CH(C_1—C_4$—alcoyle, qui est substitué par $—OR^5$, $—SR^6$ ou $—COOR^7)—$, $—C(C_1—C_4$—alcoyle)$(OR^2)—$, $—C(C_1—C_4$—alcoyle)$(COOR^4)—$, $—C(CH_3)(C_1—C_3$—alcoyle, qui est substitué par $—OR^5$, $—SR^6$ ou $—COOR^7)—$, $—C(C_2H_5)(C_1—C_2$—alcoyle, qui est substitué par $—OR^5$, $—SR^6$ ou $—COOR^7)—$, $—C(C_3H_7)(CH_2OR^5)—$, $—C(C_3H_7)(CH_2SR^6)—$, $—C(C_3H_7)(CH_2COOR^7)—$, $—C(CH_3)(C_3H_7)—$ ou $—C(C_2H_5)_2—$

2. Composés selon la revendication 1, caractérisés en ce que X et Q ont les significations données dans la revendication 1, $R^1$ représente un hydrogène, un nitroso, un alcoyle en C1 à C4 ou un alcoyle en C1 à C4-carbonyle, qui est non substitué ou substitué par un ou plusieurs atomes d'halogène, et Z représente $—COO^{\ominus}Me^{\oplus}$, $—COOR^8$, $CONR^9R^{10}$, ou —CN, où $Me^{\oplus}$ un cation, $R^8$ un hydrogène, un alcoyle en C1 à C4, qui est non substitué ou substitué par un halogène ou un alcoxy en C1 à C4, ou un alcényle en C2 à C4, $R^9$ représente un hydrogène, un alcoyle en C1 à C4 ou un cycloalcoyle en C3 à C6 et R10 représente un hydrogène, un amino, un hydroxy, un alcoyle en C1 à C4, qui est non substitué ou substitué par un alcoxy en C1 à C4, un alcynyle en C2 à C4, un alcoxy en C1 à C4 ou un cycloalcoyle en C3 à C6-aminocarbonyle, ou l'élément de structure $—N(R^1)—Q—Z$ représente un noyau pyrrolidino, qui est substitué en position 2 par Z et G', où G' représente un hydrogène ou un alcoyle en C1 à C4, avec la précision que lorsque $—N(R^1)—$ et —Z ne sont liés immédiatement l'un à l'autre que par un seul atome de carbone et $R^1$ représente un hydrogène, Q représente $—CH(COOR^4)—$, $—CH(C_1—C_4$—Alcoyle, qui est substitué par $—OR^5$, $—SR^6$ ou $—COOR^7)—$, $—C(C_1—C_4$—Alcoyle)$(COOR^4)—$, $—C(CH_3)(C_1—C_3$—Alcoyle, qui est substitué par $—OR^5$, $—SR^6$ ou $—COOR^7)—$, $—C(C_2H_5)(C_1—C_2$—Alcoyle qui est substitué par $—OR^5$, $—SR^6$ ou $—COOR^7)—$, $—C(C_3H_7)(CH_2OR^5)—$, $—C(C_3H_7)(CH_2SR^6)—$, $—C(C_3H_7)(CH_2COOR^7)—$, $—C(CH_3)(C_3H_7)—$ ou $—C(C_2H_5)_2$.

3. Composés selon la revendication 1, caractérisés en ce que X a la signification donnée dans la revendication 1, $R^1$ représente un hydrogène ou un alcoyle en C1 à C4, Q représente un radical alcoylène à chaîne droite ou ramifiée ayant jusqu'à 5 atomes de carbone, où outre la chaîne principale qui relie immédiatement l'un à l'autre les éléments de structure $—N(R^1)—$ et —Z, se trouvent de 0 à 2 chaînes latérales, où la chaîne principale est non substituée ou mono- ou disubstituée par $—OR^2$, $—SR^3$ ou $—COOR^4$, où aucun des atomes de carbone n'est disubstitué et chaque atome de carbone porte au moins un atome d'hydrogène, et les chaînes latérales sont non substituées ou substituées par $—OR^5$, $—SR^6$ ou $—COOR^7$, où simultanément à la chaîne principale une seule des chaînes latérales peut être substituée et

$R^2$, $R^3$, $R^4$, $R^5$, $R^6$ et $R^7$ représentent un hydrogène ou un alcoyle en C1 à C4, et Z représente —$COO^{\ominus}Me^{\oplus}$, —$COOR^8$, —$CONR^9R^{10}$, —$COR^{11}$ ou —CN, où $Me^{\oplus}$ un cation métallique, $R^8$ un hydrogène ou un alcoyle en C1 à C4, $R^9$ et $R^{10}$ représentent un hydrogène ou un alcoyle en C1 à C4 et $R^{11}$ représente un alcoyle en C1 à C4 substitué par un alcoxy en C1 à C4, ou l'élément de structure —N($R^1$)—Q—Z représente un noyau pyrrolidino, où Z est en position 2, avec la précision que lorsque —N($R^1$)— et —Z ne sont immédiatement liés l'un à l'autre que par un seul atome de carbone et $R^1$ représente un hydrogène, Q représente —CH($OR^2$)—, —CH($COOR^4$)— ou —CH($C_1$—$C_4$—Alcoyle, qui est substitué par —$OR^5$, —$SR^6$ ou —$COOR^7$)—.

4. Composés selon la revendication 1, caractérisés en ce que X a la signification donnée dans la revendication 1, $R^1$ représente un hydrogène ou un alcoyle en C1 à C4, Q représente un radical alcoylène ayant jusqu'à 5 atomes de carbone, qui

a) ne se compose que de la chaîne principale, qui relie immédiatement l'un à l'autre les éléments de structure —$NR^1$— et —Z et se compose de 1 à 3 atomes de carbone et est non substituée ou substituée par —$OR^2$, —$SR^3$ ou —$COOR^4$, ou

b) se compose de la chaîne principale et d'une chaîne latérale, où la chaîne principale se compose de 1 à 3 atomes de carbone et est non substituée, et la chaîne latérale est non substituée ou substituée par —$OR^5$, —$SR^6$ ou —$COOR^7$, et $R^2$, $R^3$, $R^4$, $R^5$, $R^6$ et $R^7$ représentent un hydrogène ou un alcoyle en C1 à C4, et Z représentent —$COO^{\ominus}Me^{\oplus}$, —$COOR^8$, —$COR^{11}$ ou —CN, où $Me^{\oplus}$ un cation métallique, $R^8$ un hydrogène ou un alcoyle en C1 à C4 et $R^{11}$ représente un alcoyle en C1 à C4 substitué par un alcoxy en C1 à C4, ou l'élément de structure —N($R^1$)—Q—Z représente un noyau pyrrolidino où Z est en position 2, avec la précision que lorsque —N($R^1$)— et —Z ne sont immédiatement liés l'un à l'autre que par un seul atome de carbone et $R^1$ représente un hydrogène, Q représente —CH($OR^2$)—, —CH($COOR^4$)— ou —CH($C_1$—$C_4$—Alcoyle, qui est substitué par —$OR^5$, —$SR^6$ ou —$COOR^7$).

5. Composés selon la revendication 1, caractérisés en ce que X représente un hydrogène ou un chlore, $R^1$ représente un hydrogène, un nitroso, un méthyle, un méthylcarbonyle, éthylcarbonyle, vinylcarbonyle, chlorocarbonyle, trichlorométhylcarbonyle ou trifluorométhylcarbonyle, Q représente un radical alcoylène ou alcénylène à chaîne droite ou ramifiée ayant jusqu'à 5 atomes de carbone, où outre la chaîne principale qui relie immédiatement l'un à l'autre les éléments de structure —N($R^1$)— et —Z, on trouve de 0 à 2 chaînes latérales, où la chaîne principale est saturée ou non saturée ou monosubstituée par —$OR^2$, —$SR^3$ ou —$COOR^4$ et les chaînes latérales sont saturées et non substituées ou l'une des chaînes latérales est substituée par —$OR^5$, —$SR^6$ ou —$COOR^7$, où la chaîne principale et les chaînes latérales ne sont pas simultanément substituées, et $R^2$, $R^3$, $R^4$, $R^5$, $R^6$ et $R^7$ représentent un hydrogène ou un alcoyle en C1 à C4, et Z représente —$COO^{\ominus}Me^{\oplus}$, —$COOR^8$, —$CONR^9R^{10}$ ou —CN, où $Me^{\oplus}$ un cation, $R^8$ représente un hydrogène, un alcoyle en C1 à C8, qui est non substitué ou substitué par un halogène ou un alcoxy en C1 à C4, un alcényle en C2 à C8 ou un alcynyle en C3 à C8, $R^9$ représente un hydrogène, un alcoyle en C1 à C4 ou un cycloalcoyle en C3 à C6 et $R^{10}$ représente un hydrogène, un amino, un hydroxy, un alcoyle en C1 à C4, qui est non substitué ou substitué par un alcoxy en C1 à C4, un alcynyle en C2 à C4, un alcoxy en C1 à C4 ou un cycloalcoyle en C3 à C6-aminocarbonyle, ou l'élément de structure —N($R^1$)—Q—Z représente un noyau pyrrolidino, qui est substitué en position 2 par Z et G', où G' représente un hydrogène ou un alcoyle en C1 à C4, avec la précision que lorsque —N($R^1$)— et —Z ne sont liés immédiatement l'un à l'autre que par un seul atome de carbone et $R^1$ représente un hydrogène, Q représente —CH($COOR^4$)—, —CH($C_1$—$C_4$—Alcoyle, qui est substitué par —$OR^5$, —$SR^6$ ou —$COOR^7$)—, —C($C_1$—$C_4$—Alcoyle)($COOR^4$)—, —C($CH_3$)($C_1$—$C_3$—Alcoyle, qui est substitué par —$OR^5$, —$SR^6$ ou —$COOR^7$)—, —C($C_2H_5$)($C_1$—$C_2$—Alcoyle, qui est substitué par —$OR^5$, —$SR^6$ ou —$COOR^7$)—, —C($C_3H_7$)($CH_2OR^5$)—, —C($C_3H_7$)($CH_2SR^6$)—, —C($C_3H_7$)($CH_2COOR^7$)—, —C($CH_3$)($C_3H_7$)— ou —C($C_2H_5$)$_2$—.

6. Composés selon la revendication 1, caractérisés en ce que X représente un hydrogène ou un chlore, $R^1$ représente un hydrogène ou un méthyle, Q représente unr adical alcoylène ayant jusqu'à 5 atomes de carbone, qui

a) ne se compose que de la chaîne principale, qui relie immédiatement l'un à l'autre les éléments de structure —N($R^1$)— et —Z et se compose de 1 à 3 atomes de carbone et est non substitué ou substitué par —$OR^2$, —$SR^3$ ou —$COOR^4$, ou

b) se compose de la chaîne principale et d'une chaîne latérale, où la chaîne principale contient de 1 à 3 atomes de carbone et est non substituée, et la chaîne latérale est non substituée ou substituée par —$OR^5$, —$SR^6$ ou —$COOR^7$ et $R^2$, $R^3$, $R^4$, $R^5$, $R^6$ et $R^7$ représentent un hydrogène ou un alcoyle en C1 à C4, et Z représente —$COO^{\ominus}Me^{\oplus}$, —$COOR^8$, —$COR^{11}$ ou —CN, où $Me^{\oplus}$ représente un cation métallique, $R^8$ un hydrogène ou un alcoyle en C1 à C4 et $R^{11}$ un alcoyle en $C^1$ à $C^4$ substitué par un alcoxy en C1 à C4, ou l'élément de structure —N($R^1$)—Q—Z représente un noyau pyrrolidino, où Z est en position 2, avec la précision que lorsque —N($R^1$)— et —Z ne sont liés immédiatement l'un à l'autre que par un seul atome de carbone et $R^1$ représente un hydrogène, Q représente —CH($OR^2$)—, —CH($COOR^4$)— ou —CH($C_1$—$C_4$—Alcoyle, qui est substitué par —$OR^5$, —$SR^6$ ou —$COOR^7$).

7. Composés selon la revendication 1, caractérisés en ce que X représente un hydrogène ou un chlore $R^1$ représente un hydrogène, un nitroso ou un alcoyle en C1 à C4, un alcényle en C2 à C4, un alcoyle en C1 à C4-carbonyle ou un alcényle en C2 à C4-carbonyle, qui sont non substitués ou substitués par de 1 à 3 atomes de chlore ou de fluor, Q représente un 1,5-pentaméthylène ou un radical alcoylène ou

alcénylène avec au total jusqu'à 3 atomes de carbone, qui

a) ne se compose que de la chaîne principale, qui relie immédiatement l'un à l'autre les éléments de structure —N($R^1$)— et —Z et est saturé ou non saturé et non substitué ou substitué par un carboxyle ou un alcoxy en C1 à C4-carbonyle, ou

b) se compose d'une chaîne principale, qui se compose de 2 atomes de carbone et est saturée ou non saturée, et d'une chaîne latérale ayant 1 atome de carbone, où i) la chaîne principale est non substituée ou monosubstituée par un carboxyle ou un alcoxy en C1 à C4-carbonyle et la chaîne latérale se compose d'un groupe méthyle non substitué, ou ii) la chaîne principale est non substituée et la chaîne latérale se compose d'un groupe méthyle qui est substitué par un hydroxy ou un alcoylthio en C1 à C4, ou

c) —CH(COOH)—, —CH(COOC$_1$—C$_4$—Alcoyle)—, —C(C$_1$—C$_2$—Alcoyle)(COOH)—, —C(C$_1$—C$_2$—Alcoyle)(COOC$_1$—C$_4$—Alcoyle)—, —CH(CH$_2$OH)—, —CH(CH$_2$—S—C$_1$—C$_4$—Alcoyle)—, —CH(C$_2$H$_5$OH)—, —CH(C$_2$H$_5$—S—C$_1$—C$_4$—Alcoyle)—, —C(CH$_3$)(CH$_2$OH)—, —C(CH$_3$)(CH$_2$—S—C$_1$—C$_4$—Alcoyle)—, —C(CH$_3$)$_2$ ou —CH(C$_1$—C$_2$—Alcoyle) et Z représente —COO$^⊖$Me$^⊕$, —COOR$^8$, —CONR$^9$R$^{10}$ ou —CN, où Me$^⊕$ représente un cation de métal alcalin, R$^8$ un hydrogène, un alcoyle en C1 à C8, qui est non substitué ou monosubstitué par un chlore ou un alcoxy en C1 à C4, un alcényle en C2 à C8 ou un alcynyle en C3 à C8, R$^9$ représente un hydrogène, un alcoyle en C1 à C4 ou un cycloalcoyle en C3 à C6 et R10 représente un hydrogène, un amino, un hydroxy, un alcoyle en C1 à C4, qui est non substitué ou substitué par un alcoxy en C1 à C4, un alcényle en C2 à C4, un alcoxy en C1 à C4 ou un cycloalcoyle en C3 à C6-aminocarbonyle, ou l'élément de structure —N($R^1$)—Q—Z est un noyau pyrrolidino qui est substitué en position 2 par Z et G', où G' représente un hydrogène ou un alcoyle en C1 à C4, avec la précision que lorsque —N($R^1$) et —Z ne sont liés immédiatement l'un à l'autre que par un seul atome de carbone et $R^1$ représente un hydrogène, Q représente —CH(COOH—, —CH(COOC$_1$—C$_4$—Alcoyl)—, —C(C$_1$—C$_2$—Alcoyl)(COOH)—, —C(C$_1$—C$_2$—Alcoyl)(COOC$_1$—C$_4$—Alcoyl)—, —CH(CH$_2$OH)—, —CH(CH$_2$—S—C$_1$—C$_4$—Alcoyl)—, —CH(C$_2$H$_5$OH)—, —CH(C$_2$H$_5$—S—C$_1$—C$_4$—Alcoyl)—, —C(CH$_3$)(CH$_2$OH)—, —C(CH$_3$)(CH$_2$—S—C$_1$—C$_4$—Alcoyl)— ou —C(CH$_3$)$_2$.

8. Composés selon la revendication 1, où X représente un hydrogène, $R^1$ un hydrogène, Q un pont 1,2-étylène et Z —COO$^⊖$Me$^⊕$, —COOR$^8$, —COSR$^8$, —CONR$^9$R$^{10}$, —COR$^{11}$ ou —CN, où Me$^⊕$ un cation, R représente un hydrogène, un alcoyle en C1 à C8, qui est non substitué ou substitué par un halogène, un cyano ou un alcoxy en C1 à C4, un alcényle en C2 à C8 ou un alcynyle en C3 à C8, $R^9$ représente un hydrogène, un alcoyle en C1 à C4 ou un cycloalcoyle en C3 à C6, $R^{10}$ représente un hydrogène, un amino, un hydroxy, un alcoyle en C1 à C4, qui est non substitué ou substitué par un alcoxy en C1 à C4, un alcynyle en C2 à C4, un alcoxy en C2 à C4 ou un cycloalcoyle en C3 à C6-aminocarbonyle et $R^{11}$ représente un hydrogène ou un alcoyle en C1 à C4, qui est éventuellement substitué par un alcoxy en C1 à C4.

9. Composés selon la revendication 1, caractérisés en ce que X et $R^1$ ont les significations données dans la revendication 1, Q représente un 1,5-pentaméthylène ou un radical alcoylène ou alcénylène avec au total jusqu'à 3 atomes de carbone, qui

a) ne se compose que de la chaîne principale, qui relie immédiatement l'un à l'autre les éléments de structure —N($R^1$)— et —Z et est saturé ou non saturé ou monosubstitué par un carboxyle, un méthoxycarbonyle ou un éthoxycarbonyle, ou

b) se compose d'une chaîne principale qui se compose de 2 atomes de carbone et est saturée ou non saturée, et d'une chaîne latérale ayant un atome de carbone, où i) la chaîne principale est non substituée ou monosubstituée par un carboxyle, méthoxycarbonyle ou éthoxycarbonyle et la chaîne latérale se compose d'un groupe méthyle non substitué, ou ii) la chaîne principale est non substituée et la chaîne latérale est un hydroxyméthyle, ou

c) se compose d'un membre du groupe —CH(COOH)—, —CH(COOCH$_3$)—, —CH(COOC$_2$H$_5$)—, —CH(CH$_2$OH)—, —CH(CHOH—CH$_3$)—, —CH(CH$_2$CH$_2$OH)—, —CH(CH$_2$CH$_2$—S—CH$_3$)—, —C(CH$_3$)(COOH)—, —C(CH$_3$)(COOCH$_3$)—, —C(CH$_3$)(COOC$_2$H$_5$)—, —C(CH$_3$)(CH$_2$OH)—, —CH(C$_1$—C$_2$—Alkyl)— ou —C(CH$_2$)$_2$, et Z représente —COO$^⊖$Me$^⊕$, —COOR$^8$, —CONR$^9$R$^{10}$ ou —CN, où Me$^⊕$ représente un cation sodium, R$^8$ représente un hydrogène, un alcoyle en C1 à C4, un 2-chloroéthyle, un 2-méthoxyéthyle, un allyle ou un 2-propinyle, $R^9$ représente un hydrogène, un méthyle ou un cyclohexyle et $R^{10}$ représente un hydrogène, un amino, un hydroxy, un alcoyle en C1 à C3, un 2-méthoxyéthyle, un 3-butyn-2-yle, un méthoxy ou un cyclohexylaminocarbonyle, ou l'élément de structure —N($R^1$)—Q—Z représente un noyau pyrrolidino, qui est substitué en position 2 par Z et G', où G' représente un hydrogène ou un méthyle, avec la précision que lorsque —N($R^1$)— et —Z ne sont immédiatement liés l'un à l'autre que par un seul atome de carbone et $R^1$ représente un hydrogène, Q représente —C(CH$_3$)$_3$—, —CH(COOH)—, —CH(COOCH$_3$)—, —CH(COOC$_2$H$_5$)—, —CH(CH$_2$OH)—, —CH(CHOH—CH$_3$)—, —CH(CH$_2$CH$_2$OH)—, —CH(CH$_2$CH$_2$—S—CH$_3$)—, —C(CH$_3$)(COOH)—, —C(CH$_3$)(COOCH$_3$)—, —C(CH$_3$)(COOC$_2$H$_5$)— ou —C(CH$_3$)(CH$_2$OH)—.

10. Composés selon la revendication 1, caractérisés en ce que X représente un hydrogène ou un chlore, $R^1$ un hydrogène, un nitroso, un alcoyle en C1 à C4, un alcoyle en C1 à C4-carbonyle, qui est non substitué ou substitué par de 1 à 3 atomes de chlore ou de fluor, ou un alcényle en C2 à C4-carbonyle, Q représente un 1,5-pentaméthylène ou un radical alcoylène ou alcénylène avec au total jusqu'à 3 atomes de carbone, qui

a) ne se compose que de la chaîne principale, qui relie immédiatement l'un à l'autre les éléments de structure —N($R^1$)— et —Z et est saturée ou non saturée et non substituée ou monosubstituée par un carboxyle ou un alcoxy en C1 à C4-carbonyle, ou

b) se compose d'une chaîne principale qui se compose de 2 atomes de carbone et est saturée ou non saturée, et d'une chaîne latérale avec un atome de carbone, où i) la chaîne principale est non substituée ou monosubstituée par un carboxyle ou un alcoxy en C1 à C4-carbonyle et la chaîne latérale se compose d'un groupe méthyle non substitué, ou ii) la chaîne principale est non substituée et la chaîne latérale se compose d'un groupe méthyle qui est substitué par un hydroxy ou un alcoylthio en C1 à C4, ou

c) —CH(COOH)—, —CH(COO$C_1$—$C_4$—Alcoyl)—, —C($C_1$—$C_2$—Alcoyl)(COOH)—, —C($C_1$—$C_4$—Alcoyl)—, —CH($CH_2$OH)—, —CH($CH_2$—S—$C_1$—$C_4$—Alcoyl)—, —CH($C_2H_5$OH)—, —CH($C_2H_5$—S—$C_1$—$C_4$—Alcoyl)—, —C($CH_3$)($CH_2$OH)—, —C($CH_3$)($CH_2$—S—$C_1$—$C_4$—Alcoyl)—, —C$(CH_3)_2$ ou —CH($C_1$—$C_2$—Alcoyl) et Z représente —$COO^\ominus$ $Me^\oplus$, —COO$R^8$, —CON$R^9R^{10}$ ou —CN, où $Me^\oplus$ représente un cation de métal alcalin, $R^8$ un hydrogène, un alcoyle en C1 à C4, qui est non substitué ou monosubstitué par un chlore ou un alcoxy en C1 à C4, ou un alcényle en C2 à C4, $R^9$ représente un hydrogène, un alcoyle en C1 à C4 ou un cycloalcoyle en C3 à C6 et $R^{10}$ représente un hydrogène, un amino, un hydroxy, un alcoyle en C1 à C4, qui est non substitué ou monosubstitué par un alcoxy en C1 à C4, un alcynyle en C2 à C4, un alcoxy en C1 à C4 ou un cycloalcoyle en C3 à C6-aminocarbonyle, ou l'élément de structure —N($R^1$)—Q—Z représente un noyau pyrrolidino, qui est substitué en position 2 par Z et G' est substitué, où G' représente un hydrogène ou un alcoyle en C1 à C4, avec la précision que lorsque —N($R^1$)— et —Z ne sont immédiatement reliés l'un à l'autre que par un seul atome de carbone, Q représente —CH(COOH)—, —CH(COO$C_1$—$C_4$—Alcoyl)—, —C($C_1$—$C_2$—Alcoyl)(COOH)—, —C($C_1$—$C_2$—Alcoyl)(COO$C_1$—$C_4$—Alcoyl)—, —CH($CH_2$OH)—, —CH($CH_2$—S—$C_1$—$C_4$—Alcoyl)—, —CH($C_2H_5$OH)—, —CH($C_2H_5$—S—$C_1$—$C_4$—Alcoyl)—, —C($CH_3$)($CH_2$OH)—, —C($CH_3$)($CH_2$—S—$C_1$—$C_4$—Alcoyl)— ou —C$(CH_3)_2$.

11. Composés selon la revendication 1, caractérisés en ce que X représente un hydrogène ou un chlore, $R^1$ représente un hydrogène, un nitroso, un méthyle, un méthylcarbonyle, un éthylcarbonyle, un vinylcarbonyle, un chlorométhylcarbonyle, un trichlorométhylcarbonyle ou un trifluorométhylcarbonyle, Q représente un 1,5-pentaméthylène ou un radical alcoylène ou alcénylène avec au total jusqu'à 3 atomes de carbone, qui

a) ne se compose que de la chaîne principale, qui relie immédiatement l'un à l'autre les éléments de structure —N($R^1$)— et —Z et est saturée ou non saturée ou est monosubstituée par un carboxyle, méthoxycarbonyle ou éthoxycarbonyle, ou

b) se compose d'une chaîne principale qui se compose de 2 atomes de carbone et est saturée ou non saturée, et d'une chaîne latérale ayant un atome de carbone, où i) la chaîne principale est non substituée ou monosubstituée par un carboxyle, méthoxycarbonyle ou éthoxycarbonyle et la chaîne latérale se compose d'un groupe méthyle non substitué, ou ii) la chaîne principale est non substituée et la chaîne latérale est un hydroxyméthyle, ou

c) se compose d'un membre du groupe —CH(COOH)—, —CH(COO$CH_3$)—, —CH(COO$C_2H_5$)—, —CH($CH_2$OH)—, —CH(CHOH—$CH_3$)—, —CH($CH_2CH_2$OH)—, —CH($CH_2CH_2$—S—$CH_3$)—, —C($CH_3$)(COOH)—, —C($CH_3$)(COO$CH_3$)—, —C($CH_3$)(COO$C_2H_5$)—, —C($CH_3$)($CH_2$OH)—, ou —C$(CH_3)_2$— et Z représente —$COO^\ominus Me^\oplus$, —COO$R^8$, —CON$R^9R^{10}$ où $Me^\oplus$ représente un cation sodium, $R^8$ représente un hydrogène, un alcoyle en C1 à C4, un 2-chloroéthyle, un 2-méthoxyéthyle, un allyle ou un 2-propynyle, $R^9$ représente un hydrogène, un méthyle ou un cyclohexyle et $R^{10}$ représente un hydrogène, un amino, un hydroxy, un alcoyle en C1 à C3, un 2-méthoxyéthyle, un 3-butyn-2-yle, un méthoxy ou un cyclohexylaminocarbonyle, ou l'élément de structure —N($R^1$)—Q—Z représente un noyau pyrrolidino, qui est substitué en position 2 par Z et G', où G' représente un hydrogène ou un méthyle, avec la précision que lorsque —N($R^1$) et —Z ne sont immédiatement liés l'un à l'autre que par un seul atome de carbone et $R^1$ représente un hydrogène; Q représente —C$(CH_3)_2$—, —CH(COOH)—, —CH(COO$CH_3$)—, —CH(COO$C_2H_5$)—, —CH($CH_2$OH)—, —CH(CHOH—$CH_3$)—, —CH($CH_2CH_2$OH)—, —CH($CH_2CH_2$—S—$CH_3$)—, —C($CH_3$)(COOH)—, —C($CH_3$)(COO$CH_3$)—, —C($CH_3$)(COO$C_2H_5$)— ou —C($CH_3$)($CH_2$OH)—.

12. Composés selon la revendication 1, caractérisés en ce que X représente un hydrogène ou un chlore, $R^1$ représente un hydrogène, un nitroso, méthyle, méthylcarbonyle, éthylcarbonyle, chlorométhylcarbonyle, trichlorométhylcarbonyle ou trifluorométhylcarbonyle, Q représente un 1,5-pentaméthylène ou un radical alcoylène ou alcénylène ayant jusqu'à 3 atomes de carbone, qui

a) ne se compose que de la chaîne principale, qui relie immédiatement l'un et l'autre les éléments de structure —N($R^1$)— et —Z est saturée ou non saturée ou est monosubstituée par un carboxyle ou un éthoxycarbonyle, ou

b) se compose d'une chaîne principale qui se compose de 2 atomes de carbone et est saturée ou non saturée, et d'une chaîne latérale ayant un atome de carbone, où i) la chaîne principale est non substituée ou monosubstituée par un carboxyle ou un éthoxycarbonyle et la chaîne latérale se compose d'un groupe méthyle non substitué, ou ii) la chaîne principale est non substituée et la chaîne latérale est un hydroxyméthyle, ou

c) se compose d'un membre du groupe —CH(COOH)—, —CH(COO$C_2H_5$)—, —CH($CH_2$OH)—, —CH(CHOH—$CH_3$)—, —CH($CH_2CH_2$—S—$CH_3$)—, —C($CH_3$)(COOH)—, —CH($XH_3$)(COO$C_2H_5$)—,

$—C(CH_3)(CH_2OH)—$ ou $—C(CH_3)_2$ et Z représente $—COOR^8$, $—CONR^9R^{10}$ ou —CN, où $R^8$ représente un hydrogène, un alcoyle en C1 à C4, un 2-chloroéthyle, un 2-méthoxyéthyle ou un allyle, $R^9$ représente un hydrogène, un méthyle ou un cyclohexyle et $R^{10}$ représente un hydrogène, un amino, un hydroxy, un alcoyle en C1 à C3, un 2-méthoxyéthyle, un 3-butyn-2-yle, un méthoxy ou un cyclohexylaminocarbonyle, ou l'élément de structure $—N(R^1)—Q—Z$ représente un noyau pyrrolidino, qui est substitué en position 2 par Z et G', où G' représente un hydrogène ou un méthyle, avec la précision que lorsque $—N(R^1)—$ et —Z ne sont liés immédiatement l'un à l'autre que par un seul atome de carbone et $R^1$ représente un hydrogène, Q représente $—CH(COOH)—$, $—CH(COOC_2H_5)—$, $—CH(CH_2OH)—$, $—CH(CHOH—CH_3)—$, $—CH(CH_2CH_2—S—CH_3)—$, $—C(CH_3)(COOH)—$, $—C(CH_3)(COOC_2H_5)—$, $—C(CH_3)(CH_2OH)—$ ou $—C(CH_3)_2—$.

13. Composés selon la revendication 1, caractérisés en ce que X représente un hydrogène, $R^1$ un hydrogène ou un méthyle, Q un membre du groupe $—CH_2—$, $—(CH_2)_2—$, $—(CH_2)_3—$, $—CH(CH_3)—CH_2—$, $—CH_2—CH(CH_3)—$, $—CH(CH_2OH)—CH_2—$, $—CH_2—CH(CH_2OH)—$, $—CH(CH_3)—$, $—CH(CH_2OH)—$, $—CH(CHOH—CH_3)—$, ou $—CH(CH_2CH_2—S—CH_3)—$ et Z représente —COOH, $—COOC_1—C_4—$Alcoyle, $—COO—CH_2CH_2—O—CH_3$ ou —CN, ou l'élément de structure $—N(R^1)—Q—Z$ représente un noyau pyrrolidino, où Z est en position 2, avec la précision que lorsque $—N(R^1)—$ et —Z ne sont immédiatement liés l'un à l'autre que par un seul atome de carbone et $R^1$ représente un hydrogène, Q représente $—CH(CH_2OH)—$, $—CH(CHOH—CH_3)—$ ou $—C(CH_2CH_2—S—CH_3)—$.

14. Composés selon la revendication 1, caractérisés en ce que X représente un hydrogène, $R^1$ un hydrogène, Q un pont éthylène ou triméthylène et Z représente $—COOR^8$ ou $—CONR^9R^{10}$, où $R^8$ représente un hydrogène, un alcoyle en C1 à C3 ou un allyle et $R^9$ et $R^{10}$ représentent un hydrogène.

15. Méthylester de l'acide N-[2-nitro-5-(2-chloro-4-trifluorométhyl-phénoxy)-phényl]-3-amino-propionique.

16. Méthylester de l'acide N-[2-nitro-5-(2-chloro-4-trifluorométhyl-phénoxy)-phényl]-4-amino-n-butyrique.

17. Isopropylester de l'acide N-[2-nitro-5-(2-chloro-4-trifluorométhyl-phénoxy)-phényl]-3-amino-propionique.

18. Ethylester de l'acide N-[2-nitro-5-(2-chloro-4-trifluorométhyl-phénoxy)-phényl]-3-amino-propionique.

19. Acide N-[2-nitro-5-(2-chloro-4-trifluorométhyl-phénoxy)-phényl]-3-amino-propionique.

20. Acide N-[2-nitro-5-(2-chloro-4-trifluorométhyl-phénoxy)-phényl]-4-amino-n-butyrique.

21. Amide de l'acide 3-[N-{2-nitro-5-(2-chloro-4-trifluorométhyl-phénoxy)-phényl}-amino]-propionique.

22. Allylester de l'acide N-[2-nitro-5-(2-chloro-4-trifluorométhyl-phénoxy)-phényl]-3-amino-propionique.

23. Procédé de préparation de composés de formule I selon A revendication 1, caractérisé en ce que

a) on fait réagir un composé de formule II

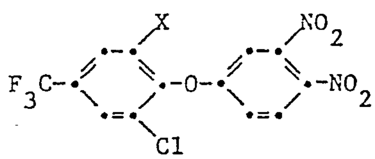

(II)

où X a la signification donnée pour la formule I, avec un composé de formule III

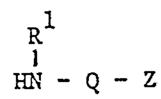

(III)

où $R^1$, Q et Z ont les significations données pour la formule I, en présence d'une base, ou

b) on fait réagir un composé de formule IV

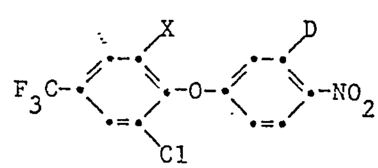

(IV)

où X a la signification donnée pour la formule I et D représente un radical séparable, avec un composé de formule III

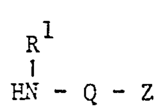

(III)

où $R^1$, Q et Z ont les significations données pour la formule I, ou

c) pour préparer des composés de formule I où $R^1$ représente un alcoyle en C1 à C4-carbonyle, on fait réagir un composé de formule V

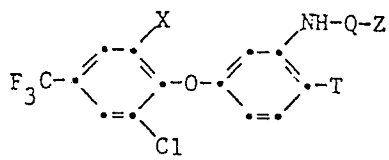

(V)

où X, Q et Z ont les significations données pour la formule I et T représente un hydrogène ou un nitro, avec un anhydride d'acide ou un chlorure d'acide et, lorsque T représente un hydrogène, on nitre le produit réactionnel obtenu, ou

d) on fait réagir un composé de formule VII

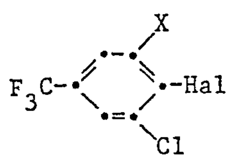

(VII)

où X a la signification donnée pour la formule I et Hal représente un atome d'halogène, avec un composé de formule VIII

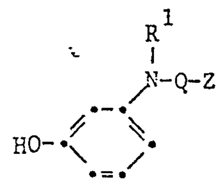

(VIII)

où $R^1$, Q et Z ont les significations données pour la formule I, en présence d'une base à une température augmentée et on nitre le produit réactionnel de formule IX obtenu

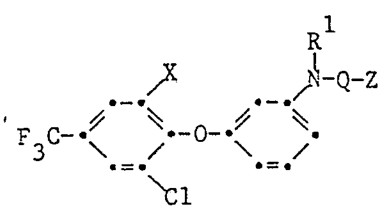

(IX)

où X, $R^1$, Q et Z ont les significations données pour la formule I, ou

e) pour préparer des composés de formule I où $R^1$ représente un alcoyle en C1 à C4, on fait réagir un composé de formule V où X, Q, Z et T ont les significations données ci-dessus, avec un agent alcoylant correspondant, et

f) le cas échéant on transforme les composés obtenus de formule I où Z représente un groupe carboxyle, en les dérivés d'acide carboxylique correspondants.

24. Agent herbicide caractérisé en ce qu'il contient comme composant actif au moins un composé de formule I selon la revendication 1.

25. Agent selon la revendication 24, caractérisé en ce qu'il contient de 0,1 à 99 % en poids d'un composé de formule I selon la revendication 1, de 1 à 99,9 % en poids d'additifs et de 0 à 25 % en poids d'un agent tensio-actif.

26. Agent selon la revendication 25, caractérisé en ce qu'il contient de 0,1 à 95 % en poids d'un composé de formule I selon la revendication 1, de 5 à 99,8 % en poids d'additifs et de 0,1 à 25 % en poids d'un agent tensio-actif.

27. Agent selon la revendication 24, caractérisé en ce qu'il contient comme composant actif un composé de formule I, où X et $R^1$ représentent un hydrogène, Q un pont éthylène ou triméthylène et Z $-COOR^8$ ou $-CONR^9R^{10}$, où $R^8$ représente un hydrogène ou un alcoyle en C1 à C3 et $R^9$ et $R^{10}$ représentent un hydrogène.

28. Agent selon la revendication 27, caractérisé en ce qu'il contient comme composant actif l'ester méthylique de l'acide N-[2-nitro-5-(2-chloro-4-trifluorométhyl-phénoxy)-phényl]-3-amino-propionique.

29. Agent selon la revendication 27, caractérisé en ce qu'il contient comme composant actif l'ester méthylique de l'acide N-[2-nitro-5-(2-chloro-4-trifluorométhyl-phénoxy)-phényl]-4-amino-n-butyrique.

30. Agent selon la revendication 27, caractérisé en ce qu'il contient comme composant actif l'isopropylester de l'acide N-[2-nitro-5-(2-chloro-4-trifluorométhyl-phénoxy)-phényl]-3-amino-propionique.

31. Agent selon la revendication 27, caractérisé en ce qu'il contient comme composant actif l'éthylester de l'acide N-[2-nitro-5-(2-chloro-4-trifluorométhyl-phénoxy)-phényl]-3-amino-propionique.

32. Agent selon la revendication 27, caractérisé en ce qu'il contient comme composant actif l'acide N-[2-nitro-5-(2-chloro-4-trifluorométhylphénoxy)-phényl]-3-amino-propionique.

33. Agent selon la revendication 27, caractérisé en ce qu'il contient comme composant actif l'acide N-[2-nitro-5-(2-chloro-4-trifluorométhyl-phénoxy)-phényl]-4-amino-n-butyrique.

34. Agent selon la revendication 27, caractérisé en ce qu'il contient comme composant actif l'amide de l'acide 3-[N-{2-nitro-5-(2-chloro-4-trifluorométhylphénoxy)-phényl}-amino]-propionique.

35. Agent de dessiccation ou de défoliation des plants de coton ou de pomme de terre caractérisé en ce qu'il contient comme composant actif un composé de formule I selon la revendication 1.

36. Agent selon la revendication 35, caractérisé en ce qu'il contient de 0,1 à 99 % en poids d'un composé de formule I selon la revendication 1, de 1 à 99,9 % en poids d'additifs et de 0 à 25 % en poids d'un agent tensio-actif.

37. Agent selon la revendication 36, caractérisé en ce qu'il contient de 0,1 à 95 % en poids d'un composé de formule I selon la revendication 1, de 5 à 99,8 % en poids d'additifs et de 0,1 à 25 % en poids d'un agent tensio-actif.

38. Agent selon la revendication 35, caractérisé en ce qu'il contient comme composant actif un composé de formule I où X et $R^1$ représentent un hydrogène, Q représente un pont éthylène ou triméthylène et Z représente un méthoxycarbonyle ou un allyloxycarbonyle.

39. Agent selon la revendication 38, caractérisé en ce qu'il contient comme composant actif le méthylester de l'acide N-[2-nitro-5-(2-chloro-4-trifluorométhyl-phénoxy)-phényl]-4-amino-n-butyrique.

40. Agent selon la revendication 38, caractérisé en ce qu'il contient comme composant actif le méthylester de l'acide N-[2-nitro-5-(2-chloro-4-trifluorométhyl-phénoxy)-phényl]-3-amino-propionique.

41. Agent selon la revendication 38, caractérisé en ce qu'il contient comme composant actif l'alkylester de l'acide N-[2-nitro-5-(2-chloro-4-trifluorométhyl-phénoxy)-phényl]-3-amino-propionique.

42. Procédé de préparation d'un agent selon les revendications 24 et 35, caractérisé en ce qu'on mélange intimement un ou plusieurs composants actifs avec des supports solides ou liquides et éventuellement des agents tensio-actifs.

43. Application de composés de formule I selon la revendication 1 ou d'un agent qui contient un composé de formule I pour combattre la croissance végétale indésirable.

44. Application selon la revendication 43 à la lutte contre les mauvaises herbes dans les surfaces de plantes cultivées.

45. Application selon la revendication 44 pour combattre les mauvaises herbes dans les cultures de céréales.

46. Application selon la revendication 45 à la lutte en pré-levée contre les mauvaises herbes dans les cultures de céréales.

47. Application selon la revendication 46 à la lutte en pré-levée contre les mauvaises herbes dicotylédones dans les cultures de céréales.

48. Application selon la revendication 47 à la lutte en pré-levée contre les mauvaises herbes dicotylédones dans le blé, l'orge et le riz.

49. Application selon la revendication 45 à la lutte en post-levée contre les mauvaises herbes dicotylédones dans les cultures de céréales.

50. Procédé de lutte contre la croissance végétale indésirable, caractérisé en ce qu'on applique un composé de formule I selon la revendication 1 ou un agent qui contient un composé de formule I aux plantes, à l'endroit où elles poussent ou à des parties de plantes.

51. Application de composés de formule I ou d'un agent qui contient un composé de formule I, à la dessiccation ou à la défoliation des plants de coton ou de pomme de terre.

52. Procédé de dessiccation ou de défoliation de plants de coton ou de pomme de terre, caractérisé en ce qu'on applique un composé de formule I ou un agent qui contient un composé de formule I, sur les plantes avant la récolte.